# EUROPEAN PATENT SPECIFICATION

(11) **EP 2 395 023 B1**
(45) Date of publication and mention of the grant of the patent: **03.04.2019**
(21) Application number: 11163023.2
(22) Date of filing: 25.08.2006
(51) Int. Cl.: A61K 39/00, C07K 16/28

(54) **MODIFIED ANTIGEN BINDING MOLECULES WITH ALTERED CELL SIGNALING ACTIVITY**
MODIFIZIERTE ANTIGENBINDEMOLEKÜLE MIT VERÄNDERTER ZELLSIGNALISIERUNGSAKTIVITÄT
MOLÉCULES DE LIAISON D'ANTIGÈNE MODIFIÉES AVEC ACTIVITÉ DE SIGNALISATION DES CELLULES ALTÉRÉE

(30) Priority: 26.08.2005 US 711454 P
(43) Date of publication of application: 14.12.2011
(62) Divisional of application: 06831581.1
(73) Proprietor: Roche Glycart AG, 8952 Schlieren (CH)
(72) Inventor: UMAÑA, Pablo, 8032 Wollerau (CH); MÖSSNER, Ekkehard, 8280 Kreuzlingen (CH)
(74) Representative: Vossius & Partner Patentanwälte Rechtsanwälte mbB

(56) References cited:
- WO-A-97/17446
- WO-A-03/024388
- WO-A-2005/044859
- WO-A-2005/062955
- US-A1- 2003 129 185
- US-A1- 2003 161 832
- US-A1- 2004 202 655
- US-A1- 2004 229 310
- US-A1- 2005 042 664
- US-A1- 2005 058 649
- NESHAT M N ET AL: "Mapping the B cell superantigen binding site for HIV-1 gp120 on a V(H)3 Ig.", INTERNATIONAL IMMUNOLOGY MAR 2000, vol. 12, no. 3, March 2000 (2000-03), pages 305-312, XP002455647, ISSN: 0953-8178
- POTTER KATHLEEN N ET AL: "Evidence for involvement of a hydrophobic patch in framework region 1 of human V4-34-encoded Igs in recognition of the red blood cell I antigen.", JOURNAL OF IMMUNOLOGY (BALTIMORE, MD. : 1950) 1 OCT 2002, vol. 169, no. 7, 1 October 2002 (2002-10-01), pages 3777-3782, XP002455648, ISSN: 0022-1767
- FAN Z C ET AL: "Comparison of the three-dimensional structures of a humanized and a chimeric Fab of an anti-gamma-interferon antibody.", JOURNAL OF MOLECULAR RECOGNITION : JMR 1999 JAN-FEB, vol. 12, no. 1, January 1999 (1999-01), pages 19-32, XP002455649, ISSN: 0952-3499
- CORTI A ET AL: "IDIOTOPE DETERMINING REGIONS OF A MOUSE MONOCLONAL ANTIBODY AND ITSHUMANIZED VERSIONS IDENTIFICATION OF FRAMEWORK RESIDUES THAT AFFECT IDIOTYPE EXPRESSION", JOURNAL OF MOLECULAR BIOLOGY, LONDON, GB, vol. 235, no. 1, January 1994 (1994-01), pages 53-60, XP000564648, ISSN: 0022-2836

### SEQUENCE LISTING

<110> GlycArt Biotechnology AG
<120> Modified Antigen Binding Molecules with Altered Cell Signaling Activity
<130> 1975.056PC01
<150> US 60/711,454
   <151> 2005-08-26
<160> 134
<170> PatentIn version 3.3
<210> 1
   <211> 357
   <212> DNA
   <213> Artificial Sequence
<220>
   <223> B-HH1 construct of a mouse/human modified antigen binding molecule
<400> 1
   caggtgcaat tggtgcagtc tggcgctgaa gttaagaagc ctgggagttc agtgaaggtc     60
   tcctgcaagg cttccggata caccttcagc tattcttgga tgagctgggt gcggcaggcc    120
   cctggacaag ggctcgagtg gatgggacgg atctttcccg gcgatgggga tactgactac    180
   gcacagaaat tccaaggaag agtcacaatt accgccgaca aatccactag cacagcctat    240
   atggagctga gcagcctgag atctgaggac acggccgtgt attactgtgc aagaaatgtc    300
   tttgatggtt actggcttgt ttactggggc cagggaaccc tggtcaccgt ctcctca       357
<210> 2
   <211> 119
   <212> PRT
   <213> Artificial Sequence
<220>
   <223> B-HH1 construct of a mouse/human modified antigen binding molecule
<400> 2
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Thr Phe Ser Tyr Ser
               20                  25                  30
   Trp Met Ser Trp Val Arg Gln Ala Pro Gly Gln Gly Leu Glu Trp Met
           35                  40                  45
   Gly Arg Ile Phe Pro Gly Asp Gly Asp Thr Asp Tyr Ala Gln Lys Phe
       50                  55                  60
   Gln Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 3
   <211> 357
   <212> DNA
   <213> Artificial Sequence
<220>
   <223> B-HH2 construct of a mouse/human modified antigen binding molecule
<400> 3
   caggtgcaat tggtgcagtc tggcgctgaa gttaagaagc ctgggagttc agtgaaggtc     60
   tcctgcaagg cttccggata cgccttcagc tattcttgga tgaactgggt gcggcaggcc    120
   cctggacaag ggctcgagtg gatgggacgg atctttcccg gcgatgggga tactgactac    180
   aatgggaaat tcaagggcag agtcacaatt accgccgaca aatccactag cacagcctat    240
   atggagctga gcagcctgag atctgaggac acggccgtgt attactgtgc aagaaatgtc    300
   tttgatggtt actggcttgt ttactggggc cagggaaccc tggtcaccgt ctcctca       357
<210> 4
   <211> 119
   <212> PRT
   <213> Artificial Sequence
<220>
   <223> B-HH2 construct of a mouse/human modified antigen binding molecule
<400> 4
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 5
   <211> 366
   <212> DNA
   <213> Artificial Sequence
<220>
   <223> B-HH3 construct of a mouse/human modified antigen binding molecule
<400> 5.
   caggtgcaat tggtgcagtc tggcgctgaa gttaagaagc ctgggagttc agtgaaggtc     60
   tcctgcaagg cttccggata cgccttcagc tattcttgga tgaactgggt gcggcaggcc    120
   cctggacaag ggctcgagtg gatgggacgg atctttcccg gcgatgggga tactgactac    180
   aatgggaaat tcaagggcag agtcacaatt accgccgaca aatccactag cacagcctat    240
   atggagctga gcagcctgag atctgaggac acggccgtgt atctgtgtgc aagaaatgtc    300
   tttgatggtt actggcttgt ttactggggc cagggaaccc tggtcaccgt ctcctcagct    360
   agcacc                                                                366
<210> 6
   <211> 119
   <212> PRT
   <213> Artificial Sequence
<220>
   <223> B-HH3 construct of a mouse/human modified antigen binding molecule
<400> 6
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Leu Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 7
   <211> 357
   <212> DNA
   <213> Artificial Sequence
<220>
   <223> B-HH4 construct of a mouse/human modified antigen binding molecule
<400> 7
   caggtgcaat tggtgcagtc tggcgctgaa gttaagaagc ctggagcttc agtgaaggtc     60
   tcctgcaagg tctccggata cgcgttcagc tattcttgga tgaactgggt gcggcaggcc    120
   cctggacaag ggctcgagtg gatgggacgg atctttcccg gcgatgggga tactgactac    180
   aatgggaaat tcaagggcag agtcacaatt accgccgaca aatccactag cacagcctat    240
   atggagctga gcagcctgag atctgaggac acggccgtgt attactgtgc aagaaatgtc    300
   tttgatggtt actggcttgt ttactggggc cagggaaccc tggtcaccgt ctcctca       357
<210> 8
   <211> 119
   <212> PRT
   <213> Artificial Sequence
<220>
   <223> B-HH4 construct of a mouse/human modified antigen binding molecule
<400> 8
   Gln Val Gln Leu Val Gln Ser Gly Ala Glu Val Lys Lys Pro Gly Ala
   1               5                   10                  15
   Ser Val Lys Val Ser Cys Lys Val Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 9
   <211> 357
   <212> DNA
   <213> Artificial Sequence
<220>
   <223> B-HH5 construct of a mouse/human modified antigen binding molecule
<400> 9
   caggtgcaat tggtgcagtc tggcgctgaa gttaagaagc ctgggagttc agtgaaggtc    60
   tcctgcaagg cttccggata cgcgttcagc tattcttgga tgagctgggt gcggcaggcg   120
   cctggacaag ggctcgagtg gatgggacgg atctttcccg gcgatgggga tactgactac   180
   aatgggaaat tcaagggcag agtcacaatt accgccgaca aatccactag cacagcctat   240
   atggagctga gcagcctgag atctgaggac acggccgtgt attactgtgc aagaaatgtc   300
   tttgatggtt actggcttgt ttactggggc cagggaaccc tggtcaccgt ctcctca      357
<210> 10
   <211> 119
   <212> PRT
   <213> Artificial Sequence
<220>
   <223> B-HH5 construct of a mouse/human modified antigen binding molecule
<400> 10
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Ser Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 11
   <211> 357
   <212> DNA
   <213> Artificial Sequence
<220>
   <223> B-HH6 construct of a mouse/human modified antigen binding molecule
<400> 11
   caggtgcaat tggtgcagtc tggcgctgaa gttaagaagc ctgggagttc agtgaaggtc     60
   tcctgcaagg cttccggata cgccttcagc tattcttgga tcaattgggt gcggcaggcg    120
   cctggacaag ggctcgagtg gatgggacgg atctttcccg gcgatgggga tactgactac    180
   aatgggaaat tcaagggcag agtcacaatt accgccgaca aatccactag cacagcctat    240
   atggagctga gcagcctgag atctgaggac acggccgtgt attactgtgc aagaaatgtc    300
   tttgatggtt actggcttgt ttactggggc cagggaaccc tggtcaccgt ctcctca       357
<210> 12
   <211> 119
   <212> PRT
   <213> Artificial Sequence
<220>
   <223> B-HH6 construct of a mouse/human modified antigen binding molecule
<400> 12
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Ile Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 13
   <211> 357
   <212> DNA
   <213> Artificial Sequence
<220>
   <223> B-HH7 construct of a mouse/human modified antigen binding molecule
<400> 13
   caggtgcaat tggtgcagtc tggcgctgaa gttaagaagc ctgggagttc agtgaaggtc      60
   tcctgcaagg cttccggata cgccttcagc tattcttgga tctcgtgggt gcggcaggcg 120
   cctggacaag ggctcgagtg gatgggacgg atctttcccg gcgatgggga tactgactac 180
   aatgggaaat tcaagggcag agtcacaatt accgccgaca aatccactag cacagcctat 240
   atggagctga gcagcctgag atctgaggac acggccgtgt attactgtgc aagaaatgtc 300
   tttgatggtt actggcttgt ttactggggc cagggaaccc tggtcaccgt ctcctca 357
<210> 14
   <211> 357
   <212> PRT
   <213> Artificial Sequence
<220>
   <223> B-HH7 construct of a mouse/human modified antigen binding molecule
<400> 14
   Cys Ala Gly Gly Thr Gly Cys Ala Ala Thr Thr Gly Gly Thr Gly Cys
   1               5                   10                  15
   Ala Gly Thr Cys Thr Gly Gly Cys Gly Cys Thr Gly Ala Ala Gly Thr
               20                  25                  30
   Thr Ala Ala Gly Ala Ala Gly Cys Cys Thr Gly Gly Gly Ala Gly Thr
           35                  40                  45
   Thr Cys Ala Gly Thr Gly Ala Ala Gly Gly Thr Cys Thr Cys Cys Thr
       50                  55                  60
   Gly Cys Ala Ala Gly Gly Cys Thr Thr Cys Cys Gly Gly Ala Thr Ala
   65                  70                  75                  80
   Cys Gly Cys Cys Thr Thr Cys Ala Gly Cys Thr Ala Thr Thr Cys Thr
                   85                  90                  95
   Thr Gly Gly Ala Thr Cys Thr Cys Gly Thr Gly Gly Gly Thr Gly Cys
               100                 105                 110
   Gly Gly Cys Ala Gly Gly Cys Gly Cys Cys Thr Gly Gly Ala Cys Ala
           115                 120                 125
   Ala Gly Gly Gly Cys Thr Cys Gly Ala Gly Thr Gly Gly Ala Thr Gly
       130                 135                 140
   Gly Gly Ala Cys Gly Gly Ala Thr Cys Thr Thr Thr Cys Cys Cys Gly
   145                 150                 155                 160
   Gly Cys Gly Ala Thr Gly Gly Gly Gly Ala Thr Ala Cys Thr Gly Ala
                   165                 170                 175
   Cys Thr Ala Cys Ala Ala Thr Gly Gly Gly Ala Ala Ala Thr Thr Cys
               180                 185                 190
   Ala Ala Gly Gly Gly Cys Ala Gly Ala Gly Thr Cys Ala Cys Ala Ala
           195                 200                 205
   Thr Thr Ala Cys Cys Gly Cys Cys Gly Ala Cys Ala Ala Ala Thr Cys
       210                 215                 220
   Cys Ala Cys Thr Ala Gly Cys Ala Cys Ala Gly Cys Cys Thr Ala Thr
   225                 230                 235                 240
   Ala Thr Gly Gly Ala Gly Cys Thr Gly Ala Gly Cys Ala Gly Cys Cys
                   245                 250                 255
   Thr Gly Ala Gly Ala Thr Cys Thr Gly Ala Gly Gly Ala Cys Ala Cys
               260                 265                 270
   Gly Gly Cys Cys Gly Thr Gly Thr Ala Thr Thr Ala Cys Thr Gly Thr
           275                 280                 285
   Gly Cys Ala Ala Gly Ala Ala Ala Thr Gly Thr Cys Thr Thr Thr Gly
       290                 295                 300
   Ala Thr Gly Gly Thr Thr Ala Cys Thr Gly Gly Cys Thr Thr Gly Thr
   305                 310                 315                 320
   Thr Thr Ala Cys Thr Gly Gly Gly Gly Cys Cys Ala Gly Gly Gly Ala
                   325                 330                 335
   Ala Cys Cys Cys Thr Gly Gly Thr Cys Ala Cys Cys Gly Thr Cys Thr
               340                 345                 350
   Cys Cys Thr Cys Ala
           355
<210> 15
   <211> 357
   <212> DNA
   <213> Artificial Sequence
<220>
   <223> B-HH8 construct of a mouse/human modified antigen binding molecule
<400> 15
   caggtgcaat tggtgcagtc tggcgctgaa gttaagaagc ctggcgcctc agtgaaggtc 60
   tcctgcaagg cttccggata caccttcaca tacagctgga tgaactgggt gcggcaggcc 120
   cctggacaag ggctcgagtg gatgggacgg atctttcccg gcgatgggga tactgactac 180
   aatgggaaat tcaagggcag agtcacaatt accgccgaca aatccactag cacagcctat 240
   atggagctga gcagcctgag atctgaggac acggccgtgt attactgtgc aagaaatgtc 300
   tttgatggtt actggcttgt ttactggggc cagggaaccc tggtcaccgt ctcctca 357
<210> 16
   <211> 119
   <212> PRT
   <213> Artificial Sequence
<220>
   <223> B-HH8 construct of a mouse/human modified antigen binding molecule
<400> 16
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Tyr Ser
   20 25 30
   Trp Met Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 17
   <211> 357
   <212> DNA
   <213> Artificial Sequence
<220>
   <223> B-HH9 construct of a mouse/human modified antigen binding molecule
<400> 17
   caggtgcaat tggtgcagtc tggcgctgaa gttaagaagc ctggcgcctc agtgaaggtc     60
   tcctgcaagg cttccggata caccttcagc tattcttgga tgaactgggt gcggcaggcc    120
   cctggacaag ggctcgagtg gatgggacgg atctttcccg gcgatgggga tactgactac    180
   aatgggaaat tcaagggcag agtcacaatt accgccgaca aatccactag cacagcctat    240
   atggagctga gcagcctgag atctgaggac acggccgtgt attactgtgc aagaaatgtc    300
   tttgatggtt actggcttgt ttactggggc cagggaaccc tggtcaccgt ctcctca       357
<210> 18
   <211> 119
   <212> PRT
   <213> Artificial Sequence
<220>
   <223> B-HH9 construct of a mouse/human modified antigen binding molecule
<400> 18
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 19
   <211> 357
   <212> DNA
   <213> Artificial Sequence
<220>
   <223> B-HL1 construct of a mouse/human modified antigen binding molecule
<400> 19
   caggtgcaat tggtgcagtc tggcgctgaa gttaagaagc ctggggcctc agtgaaggtc     60
   tcctgcaagg cttccggata caccttcacc tattcttgga tgcactgggt gcggcaggcc    120
   cctggacaag ggctcgagtg gatgggacgg atctttcccg gcgatgggga tactgactac    180
   gcacagaaat tccaaggaag agtcacaatg acacgggaca cgtccacttc caccgtctat    240
   atggagctga gcagcctgag atctgaggac acggccgtgt attactgtgc aagaaatgtc    300
   tttgatggtt actggcttgt ttactggggc cagggaaccc tggtcaccgt ctcctca       357
<210> 20
   <211> 119
   <212> PRT
   <213> Artificial Sequence
<220>
   <223> B-HL1 construct of a mouse/human modified antigen binding molecule
<400> 20
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Tyr Ser
               20                  25                  30
   Trp Met His Trp Val Arg Gln Ala Pro Gly Gln Gly Leu Glu Trp Met
           35                  40                  45
   Gly Arg Ile Phe Pro Gly Asp Gly Asp Thr Asp Tyr Ala Gln Lys Phe
       50                  55                  60
   Gln Gly Arg Val Thr Met Thr Arg Asp Thr Ser Thr Ser Thr Val Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 21
   <211> 357
   <212> DNA
   <213> Artificial Sequence
<220>
   <223> B-HL2 construct of a mouse/human modified antigen binding molecule
<400> 21
   gaggtgcaat tggtgcagtc tggcgctgaa gttaagaagc ctggggccac cgtgaagatc     60
   tcctgcaagg tgtccggata caccttcacc tattcttgga tgcactgggt gcagcaggcc    120
   cctggaaagg ggctcgagtg gatgggacgg atctttcccg gcgatgggga tactgactac    180
   gcagagaaat tccaaggaag agtcacaatc acagccgaca cgtccactga caccgcctat    240
   atggagctga gcagcctgag atctgaggac acggccgtgt attactgtgc aaccaatgtc    300
   tttgatggtt actggcttgt ttactggggc cagggaaccc tggtcaccgt ctcctca       357
<210> 22
   <211> 119
   <212> PRT
   <213> Artificial Sequence
<220>
   <223> B-HL2 construct of a mouse/human modified antigen binding molecule
<400> 22
   Glu Val Gln Leu Val Gln Ser Gly Ala Glu Val Lys Lys Pro Gly Ala
   1               5                   10                  15
   Thr Val Lys Ile Ser Cys Lys Val Ser Gly Tyr Thr Phe Thr Tyr Ser
               20                  25                  30
   Trp Met His Trp Val Gln Gln Ala Pro Gly Lys Gly Leu Glu Trp Met
           35                  40                  45
   Gly Arg Ile Phe Pro Gly Asp Gly Asp Thr Asp Tyr Ala Glu Lys Phe
       50                  55                  60
   Gln Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
   85 90 95
   Ala Thr Asn Val Phe Asp Gly Tyr Trp Leu Val Tyr Trp Gly Gln Gly
   100 105 110
   Thr Leu Val Thr Val Ser Ser
   115
<210> 23
   <211> 357
   <212> DNA
   <213> Artificial Sequence
<220>
   <223> B-HL3 construct of a mouse/human modified antigen binding molecule
<400> 23
   gaggtgcaat tggtgcagtc tggcgctgaa gttaagaagc ctggggccac cgtgaagatc     60
   tcctgcaagg tgtccggata caccttcacc tattcttgga tgaactgggt gcagcaggcc    120
   cctggaaagg ggctcgagtg gatgggacgg atctttcccg gcgatgggga tactgactac    180
   aatgggaaat tcaagggaag agtcacaatc acagccgaca cgtccactga caccgcctat    240
   atggagctga gcagcctgag atctgaggac acggccgtgt attactgtgc aaccaatgtc    300
   tttgatggtt actggcttgt ttactggggc cagggaaccc tggtcaccgt ctcctca       357
<210> 24
   <211> 119
   <212> PRT
   <213> Artificial Sequence
<220>
   <223> B-HL3 construct of a mouse/human modified antigen binding molecule
<400> 24
   Glu Val Gln Leu Val Gln Ser Gly Ala Glu Val Lys Lys Pro Gly Ala
   1 5 10 15
   Thr Val Lys Ile Ser Cys Lys Val Ser Gly Tyr Thr Phe Thr Tyr Ser
   20 25 30
   Trp Met Asn Trp Val Gln Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Thr Ser Thr Asp Thr Ala Tyr
   65                  70                  75                  80
   Met Glu Leu Ser Ser Leu Arg Ser Glu Asp Thr Ala Val Tyr Tyr Cys
                   85                  90                  95
   Ala Thr Asn Val Phe Asp Gly Tyr Trp Leu Val Tyr Trp Gly Gln Gly
               100                 105                 110
   Thr Leu Val Thr Val Ser Ser
           115
<210> 25
   <211> 366
   <212> DNA
   <213> Artificial Sequence
<220>
   <223> B-HL4 construct of a mouse/human modified antigen binding molecule
<400> 25
   cagatgcaat tggtgcagtc tggcgctgaa gttaagaaga ccgggagttc agtgaaggtc      60
   tcctgcaagg cttccggata caccttcacc tattcttgga tgagctgggt gcggcaggcc     120
   cctggacaag ggctcgagtg gatgggacgg atctttcccg gcgatgggga tactgactac     180
   gcacagaaat tccaaggaag agtcacaatt accgccgaca aatccactag cacagcctat     240
   atggagctga gcagcctgag atctgaggac acggccgtgt attactgtgc aagaaatgtc     300
   tttgatggtt actggcttgt ttactggggc cagggaaccc tggtcaccgt ctcctcagct     360
   agcacc                                                                366
<210> 26
   <211> 119
   <212> PRT
   <213> Artificial Sequence
<220>
   <223> B-HL4 construct of a mouse/human modified antigen binding molecule
<400> 26
   Gln Met Gln Leu Val Gln Ser Gly Ala Glu Val Lys Lys Thr Gly Ser
   1               5                   10                  15
   Ser Val Lys Val Ser Cys Lys Ala Ser Gly Tyr Thr Phe Thr Tyr Ser
               20                  25                  30
   Trp Met Ser Trp Val Arg Gln Ala Pro Gly Gln Gly Leu Glu Trp Met
   35 40 45
   Gly Arg Ile Phe Pro Gly Asp Gly Asp Thr Asp Tyr Ala Gln Lys Phe
   50 55 60
   Gln Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 27
   <211> 357
   <212> DNA
   <213> Artificial Sequence
<220>
   <223> B-HL8 construct of a mouse/human modified antigen binding molecule
<400> 27
   gaagtgcagc tggtggagtc tggaggaggc ttggtcaagc ctggcgggtc cctgcggctc 60
   tcctgtgcag cctctggatt cacatttagc tattcttgga tgaactgggt gcggcaggct 120
   cctggaaagg gcctcgagtg ggtgggacgg atctttcccg gcgatgggga tactgactac 180
   aatgggaaat tcaagggcag agtcacaatt accgccgaca aatccactag cacagcctat 240
   atggagctga gcagcctgag atctgaggac acggccgtgt attactgtgc aagaaatgtc 300
   tttgatggtt actggcttgt ttactggggc cagggaaccc tggtcaccgt ctcctca 357
<210> 28
   <211> 119
   <212> PRT
   <213> Artificial Sequence
<220>
   <223> B-HL8 construct of a mouse/human modified antigen binding molecule
<400> 28
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 29
   <211> 456
   <212> DNA
   <213> Artificial Sequence
<220>
   <223> B-HL10 construct of a mouse/human modified antigen binding molecule
<400> 29
   cggaattcgg cccaccggtg gccaccatgg actggacctg gaggatcctc ttcttggtgg     60
   cagcagccac aggagcccac tccgaagtgc agctggtgga gtctggagga ggcttggtca    120
   agcctggcgg gtccctgcgg ctctcctgtg cagcctctgg attcgcattc agctattctt    180
   ggatgaactg ggtgcggcag gctcctggaa agggcctcga gtgggtggga cggatctttc    240
   ccggcgatgg ggatactgac tacaatggga aattcaaggg cagagtcaca attaccgccg    300
   acaaatccac tagcacagcc tatatggagc tgagcagcct gagatctgag gacacggccg    360
   tgtattactg tgcaagaaat gtctttgatg gttactggct tgtttactgg ggccagggaa    420
   ccctggtcac cgtctcctca gctagcgaat tctcga                              456
<210> 30
   <211> 119
   <212> PRT
   <213> Artificial Sequence
<220>
   <223> B-HL10 construct of a mouse/human modified antigen binding molecule
<400> 30
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Ala Phe Ser Tyr Ser
   20 25 30
   Trp Met Asn Trp Val Arg Gln Ala Pro Gly Lys Gly Leu Glu Trp Val
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 31
   <211> 357
   <212> DNA
   <213> Artificial Sequence
<220>
   <223> B-HL11 construct of a mouse/human modified antigen binding molecule
<400> 31
   caggtgcagc tggtggagtc tggaggaggc ttggtcaagc ctggcgggtc cctgcggctc 60
   tcctgtgcag cctctggatt cacatttagc tattcttgga tgaactgggt gcggcaggct 120
   cctggaaagg gcctcgagtg ggtgggacgg atctttcccg gcgatgggga tactgactac 180
   aatgggaaat tcaagggcag agtcacaatt accgccgaca aatccactag cacagcctat 240
   atggagctga gcagcctgag atctgaggac acggccgtgt attactgtgc aagaaatgtc 300
   tttgatggtt actggcttgt ttactggggc cagggaaccc tggtcaccgt ctcctca 357
<210> 32
   <211> 119
   <212> PRT
   <213> Artificial Sequence
<220>
   <223> B-HL11 construct of a mouse/human modified antigen binding molecule
<400> 32
   Gln Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Val
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 33
   <211> 456
   <212> DNA
   <213> Artificial Sequence
<220>
   <223> B-HL12 construct of a mouse/human modified antigen binding molecule
<400> 33
   cggaattcgg cccaccggtg gccaccatgg actggacctg gaggatcctc ttcttggtgg     60
   cagcagccac aggagctcac tccgaagtgc agctcgtgga gtctggagca ggcttggtca    120
   agcctggcgg gtccctgcgg ctctcctgcg cagcctctgg attcacattt agctattctt    180
   ggatgaactg ggtgcggcag gctcctggaa agggcctcga gtgggtggga cggatctttc    240
   ccggcgatgg ggatactgac tacaatggga aattcaaggg cagagtcaca attaccgccg    300
   acaaatccac tagcacagcc tatatggagc tgagcagcct gagatctgag gacacggccg    360
   tgtattactg tgcaagaaat gtctttgatg gttactggct tgtttactgg ggccagggaa    420
   ccctggtcac cgtctcctca gctagcgaat tctcga                              456
<210> 34
   <211> 119
   <212> PRT
   <213> Artificial Sequence
<220>
   <223> B-HL12 construct of a mouse/human modified antigen binding molecule
<400> 34
   Glu Val Gln Leu Val Glu Ser Gly Ala Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 35
   <211> 456
   <212> DNA
   <213> Artificial Sequence
<220>
   <223> B-HL13 construct of a mouse/human modified antigen binding molecule
<400> 35
   cggaattcgg cccaccggtg gccaccatgg actggacctg gaggatcctc ttcttggtgg 60
   cagcagccac aggagctcac tccgaagtgc agctcgtcga gtctggagga ggcgtggtca 120
   agcctggcgg gtccctgcgg ctctcctgcg cagcctctgg attcacattt agctattctt 180
   ggatgaactg ggtgcggcag gctcctggaa agggcctcga gtgggtggga cggatctttc 240
   ccggcgatgg ggatactgac tacaatggga aattcaaggg cagagtcaca attaccgccg 300
   acaaatccac tagcacagcc tatatggagc tgagcagcct gagatctgag gacacggccg 360
   tgtattactg tgcaagaaat gtctttgatg gttactggct tgtttactgg ggccagggaa 420
   ccctggtcac cgtctcctca gctagcgaat tctcga 456
<210> 36
   <211> 119
   <212> PRT
   <213> Artificial Sequence
<220>
   <223> B-HL13 construct of a mouse/human modified antigen binding molecule
<400> 36
   Glu Val Gln Leu Val Glu Ser Gly Gly Gly Val Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 37
   <211> 456
   <212> DNA
   <213> Artificial Sequence
<220>
   <223> B-HL14 construct of a mouse/human modified antigen binding molecule
<400> 37
   cggaattcgg cccaccggtg gccaccatgg actggacctg gaggatcctc ttcttggtgg     60
   cagcagccac aggagctcac tccgaagtgc agctggtcga gtccggagga ggcttgaaga    120
   agcctggcgg gtccctgcgg ctctcctgcg cagcctctgg attcacattt agctattctt    180
   ggatgaactg ggtgcggcag gctcctggaa agggcctcga gtgggtggga cggatctttc    240
   ccggcgatgg ggatactgac tacaatggga aattcaaggg cagagtcaca attaccgccg    300
   acaaatccac tagcacagcc tatatggagc tgagcagcct gagatctgag gacacggccg    360
   tgtattactg tgcaagaaat gtctttgatg gttactggct tgtttactgg ggccagggaa    420
   ccctggtcac cgtctcctca gctagcgaat tctcga                              456
<210> 38
   <211> 119
   <212> PRT
   <213> Artificial Sequence
<220>
   <223> B-HL14 construct of a mouse/human modified antigen binding molecule
<400> 38
   Glu Val Gln Leu Val Glu Ser Gly Gly Gly Leu Lys Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 39
   <211> 456
   <212> DNA
   <213> Artificial Sequence
<220>
   <223> B-HL15 construct of a mouse/human modified antigen binding molecule
<400> 39
   cggaattcgg cccaccggtg gccaccatgg actggacctg gaggatcctc ttcttggtgg     60
   cagcagccac aggagcccac tccgaagtgc agctggtgga gtctggagga ggcttggtca    120
   agcctggctc ttccctgcgg ctctcctgcg cagcctctgg attcacattt agctattctt    180
   ggatgaactg ggtgcggcag gctcctggaa agggcctcga gtgggtggga cggatctttc    240
   ccggcgatgg ggatactgac tacaatggga aattcaaggg cagagtcaca attaccgccg    300
   acaaatccac tagcacagcc tatatggagc tgagcagcct gagatctgag gacacggccg    360
   tgtattactg tgcaagaaat gtctttgatg gttactggct tgtttactgg ggccagggaa    420
   ccctggtcac cgtctcctca gctagcgaat tctcga                              456
<210> 40
   <211> 119
   <212> PRT
   <213> Artificial Sequence
<220>
   <223> B-HL15 construct of a mouse/human modified antigen binding molecule
<400> 40
   Glu Val Gln Leu Val Glu Ser Gly Gly Gly Leu Val Lys Pro Gly Ser
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 41
   <211> 456
   <212> DNA
   <213> Artificial Sequence
<220>
   <223> B-HL16 construct of a mouse/human modified antigen binding molecule
<400> 41
   cggaattcgg cccaccggtg gccaccatgg actggacctg gaggatcctc ttcttggtgg 60
   cagcagccac aggagcccac tccgaagtgc agctggtgga gtctggagga ggcttggtca 120
   agcctggcgg gtccctgcgg gtcagctgcg cagcctctgg attcacattt agctattctt 180
   ggatgaactg ggtgcggcag gctcctggaa agggcctcga gtgggtggga cggatctttc 240
   ccggcgatgg ggatactgac tacaatggga aattcaaggg cagagtcaca attaccgccg 300
   acaaatccac tagcacagcc tatatggagc tgagcagcct gagatctgag gacacggccg 360
   tgtattactg tgcaagaaat gtctttgatg gttactggct tgtttactgg ggccagggaa 420
   ccctggtcac cgtctcctca gctagcgaat tctcga 456
<210> 42
   <211> 119
   <212> PRT
   <213> Artificial Sequence
<220>
   <223> B-HL16 construct of a mouse/human modified antigen binding molecule
<400> 42
   Glu Val Gln Leu Val Glu Ser Gly Gly Gly Leu Val Lys Pro Gly Gly
   1 5 10 15
   Ser Leu Arg Val Ser Cys Ala Ala Ser Gly Phe Thr Phe Ser Tyr Ser
   20 25 30
   Trp Met Asn Trp Val Arg Gln Ala Pro Gly Lys Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 43
   <211> 456
   <212> DNA
   <213> Artificial Sequence
<220>
   <223> B-HL17 construct of a mouse/human modified antigen binding molecule
<400> 43
   cggaattcgg cccaccggtg gccaccatgg actggacctg gaggatcctc ttcttggtgg 60
   cagcagccac aggagcccac tccgaagtgc agctggtgga gtctggagga ggcttggtca 120
   agcctggcgg gtccctgcgg ctctcctgcg cagcctctgg attcacattt agctattctt 180
   ggatgaactg ggtgcggcag gctcctggaa agggcctcga gtgggtggga cggatctttc 240
   ccggcgatgg ggatactgac tacaatggga aattcaaggg cagagtcaca attaccgccg 300
   acaaatccac tagcacagcc tatatggagc tgagcagcct gagatctgag gacacggccg 360
   tgtattactg tgcaagaaat gtctttgatg gttactggct tgtttactgg ggccagggaa 420
   ccctggtcac cgtctcctca gctagcgaat tctcga 456
<210> 44
   <211> 119
   <212> PRT
   <213> Artificial Sequence
<220>
   <223> B-HL17 construct of a mouse/human modified antigen binding molecule
<400> 44
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Tyr Ser
               20                  25                  30
   Trp Met Asn Trp Val Arg Gln Ala Pro Gly Lys Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 45
   <211> 57
   <212> DNA
   <213> Homo sapiens
<400> 45
   atggactgga cctggaggat cctcttcttg gtggcagcag ccacaggagc ccactcc 57
<210> 46
   <211> 19
   <212> PRT
   <213> Homo sapiens
<400> 46
   Met Asp Trp Thr Trp Arg Ile Leu Phe Leu Val Ala Ala Ala Thr Gly
   1               5                   10                  15
   Ala His Ser
<210> 47
   <211> 345
   <212> DNA
   <213> Artificial Sequence
<220>
   <223> B-KV1 of a mouse/human modified antigen binding molecule
<400> 47
   gatatcgtga tgacccagac tccactctcc ctgcccgtca cccctggaga gcccgccagc      60
   attagctgca ggtctagcaa gagcctcttg cacagcaatg gcatcactta tttgtattgg     120
   tacctgcaaa agccagggca gtctccacag ctcctgattt atcaaatgtc caaccttgtc     180
   tctggcgtcc ctgaccggtt ctccggatcc gggtcaggca ctgatttcac actgaaaatc     240
   agcagggtgg aggctgagga tgttggagtt tattactgcg ctcagaatct agaacttcct     300
   tacaccttcg gcggagggac caaggtggag atcaaacgta cggtg                     345
<210> 48
   <211> 114
   <212> PRT
   <213> Artificial Sequence
<220>
   <223> B-KV1 of a mouse/human modified antigen binding molecule
<400> 48
   Asp Ile Val Met Thr Gln Thr Pro Leu Ser Leu Pro Val Thr Pro Gly
   1               5                   10                  15
   Glu Pro Ala Ser Ile Ser Cys Arg Ser Ser Lys Ser Leu Leu His Ser
               20                  25                  30
   Asn Gly Ile Thr Tyr Leu Tyr Trp Tyr Leu Gln Lys Pro Gly Gln Ser
           35                  40                  45
   Pro Gln Leu Leu Ile Tyr Gln Met Ser Asn Leu Val Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ala Gln Asn
                   85                  90                  95
   Leu Glu Leu Pro Tyr Thr Phe Gly Gly Gly Thr Lys Val Glu Ile Lys
               100                 105                 110
   Arg Thr
<210> 49
   <211> 66
   <212> DNA
   <213> Homo sapiens
<400> 49
   atggacatga gggtccccgc tcagctcctg ggcctcctgc tgctctggtt cccaggtgcc     60
   aggtgt                                                                66
<210> 50
   <211> 22
   <212> PRT
   <213> Homo sapiens
<400> 50
   Met Asp Met Arg Val Pro Ala Gln Leu Leu Gly Leu Leu Leu Leu Trp
   1               5                   10                  15
   Phe Pro Gly Ala Arg Cys
               20
<210> 51
   <211> 360
   <212> DNA
   <213> Artificial Sequence
<220>
   <223> I-HHD construct of a mouse/human modified antigen binding molecule
<400> 51
   caggtgcagc tggtgcagtc tggggctgag gtgaagaagc ctgggtcctc ggtgaaggtc     60
   tcctgcaagg cctctggttt cacattcact gactacaaga tacactgggt gcgacaggcc    120
   cctggacaag ggctcgagtg gatgggatat ttcaacccta acagcggtta tagtacctac    180
   gcacagaagt tccagggcag ggtcaccatt accgcggaca aatccacgag cacagcctac    240
   atggagctga gcagcctgag atctgaggac acggccgtgt attactgtgc gagactatcc    300
   ccaggcggtt actatgttat ggatgcctgg ggccaaggga ccaccgtgac cgtctcctca    360
<210> 52
   <211> 120
   <212> PRT
   <213> Artificial Sequence
<220>
   <223> I-HHD construct of a mouse/human modified antigen binding molecule
<400> 52
<210> 53
   <211> 366
   <212> DNA
   <213> Artificial Sequence
<220>
   <223> M-HHA construct of a mouse/human modified antigen binding molecule
<400> 53
   gaagtgcagc tggtggagtc tggaggaggc ttggtcaagc ctggcgggtc cctgcggctc     60
   tcctgtgcag cctccggatt cacatttagc aactattgga tgaactgggt gcggcaggct    120
   cctggaaagg gcctcgagtg ggtgggagag atcagattga aatccaataa cttcggaaga    180
   tattacgctg caagcgtgaa gggccggttc accatcagca gagatgattc caagaacacg    240
   ctgtacctgc agatgaacag cctgaagacc gaggatacgg ccgtgtatta ctgtaccaca    300
   tacggcaact acgttgggca ctacttcgac cactggggcc aagggaccac cgtcaccgtc    360
   tccagt                                                               366
<210> 54
   <211> 122
   <212> PRT
   <213> Artificial Sequence
<220>
   <223> M-HHA construct of a mouse/human modified antigen binding molecule
<400> 54
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Asn Tyr
               20                  25                  30
   Trp Met Asn Trp Val Arg Gln Ala Pro Gly Lys Gly Leu Glu Trp Val
           35                  40                  45
   Gly Glu Ile Arg Leu Lys Ser Asn Asn Phe Gly Arg Tyr Tyr Ala Ala
       50                  55                  60
   Ser Val Lys Gly Arg Phe Thr Ile Ser Arg Asp Asp Ser Lys Asn Thr
   65                  70                  75                  80
   Leu Tyr Leu Gln Met Asn Ser Leu Lys Thr Glu Asp Thr Ala Val Tyr
                   85                  90                  95
   Tyr Cys Thr Thr Tyr Gly Asn Tyr Val Gly His Tyr Phe Asp His Trp
               100                 105                 110
   Gly Gln Gly Thr Thr Val Thr Val Ser Ser
           115                 120
<210> 55
   <211> 122
   <212> PRT
   <213> Artificial Sequence
<220>
   <223> IF5-VH - mouse/human chimeric polypeptide
<400> 55'
<210> 56
   <211> 120
   <212> PRT
   <213> Artificial Sequence
<220>
   <223> B9E9-VH - mouse/human chimeric polypeptide
<400> 56
   Gln Val Gln Leu Val Gln Ser Gly Ala Glu Leu Val Lys Pro Gly Ala
   1               5                   10                  15
   Ser Val Lys Met Ser Cys Lys Ala Ser Gly Tyr Thr Phe Thr Ser Tyr
               20                  25                  30
   Asn Met His Trp Val Lys Gln Thr Pro Gly Gln Gly Leu Glu Trp Ile
           35                  40                  45
   Gly Ala Ile Tyr Pro Gly Asn Gly Asp Thr Ser Tyr Asn Gln Lys Phe
       50                  55                  60
   Lys Gly Lys Ala Thr Leu Thr Ala Asp Lys Ser Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Ala Gln Leu Arg Pro Asn Tyr Trp Tyr Phe Asp Val Trp Gly
               100                 105                 110
   Ala Gly Thr Thr Val Thr Val Ser
           115                 120
<210> 57
   <211> 121
   <212> PRT
   <213> Artificial Sequence
<220>
   <223> C2B8-VH - mouse/human chimeric polypeptide
<400> 57
<210> 58
   <211> 121
   <212> PRT
   <213> Artificial Sequence
<220>
   <223> 2H7-VH - mouse/human chimeric polypeptide
<400> 58
   Gln Ala Tyr Leu Gln Gln Ser Gly Ala Glu Leu Val Arg Pro Gly Ala
   1               5                   10                  15
   Ser Val Lys Met Ser Cys Lys Ala Ser Gly Tyr Thr Phe Thr Ser Tyr
               20                  25                  30
   Asn Met His Trp Val Lys Gln Thr Pro Arg Gln Gly Leu Glu Trp Ile
   35 40 45
   Gly Ala Ile Tyr Pro Gly Asn Gly Asp Thr Ser Tyr Asn Gln Lys Phe
   50 55 60
   Lys Gly Lys Ala Thr Leu Thr Val Asp Lys Ser Ser Ser Thr Ala Tyr
   65 70 75 80
   Met Gln Leu Ser Ser Leu Thr Ser Glu Asp Ser Ala Val Tyr Phe Cys
   85 90 95
   Ala Arg Val Val Tyr Tyr Ser Asn Ser Tyr Trp Tyr Phe Asp Val Trp
   100 105 110
   Gly Thr Gly Thr Thr Val Thr Val Ser
   115 120
<210> 59
   <211> 119
   <212> PRT
   <213> Artificial Sequence
<220>
   <223> B-lyl-VH - mouse/human chimeric polypeptide
<400> 59
   Glu Val Lys Leu Gln Gln Ser Gly Pro Glu Leu Val Lys Pro Gly Ala
   1 5 10 15
   Ser Val Lys Ile Ser Cys Lys Ala Ser Gly Tyr Ala Phe Ser Tyr Ser
   20 25 30
   Trp Met Asn Trp Val Lys Leu Arg Pro Gly Gln Gly Leu Glu Trp Ile
   35 40 45
   Gly Arg Ile Phe Pro Gly Asp Gly Asp Thr Asp Tyr Asn Gly Lys Phe
   50 55 60
   Lys Gly Lys Ala Thr Leu Thr Ala Asp Lys Ser Ser Asn Thr Ala Tyr
   65 70 75 80
   Met Gln Leu Thr Ser Leu Thr Ser Val Asp Ser Ala Val Tyr Leu Cys
   85 90 95
   Ala Arg Asn Val Phe Asp Gly Tyr Trp Leu Val Tyr Trp Gly Gln Gly
   100 105 110
   Thr Leu Val Thr Val Ser Ala
   115
<210> 60
   <211> 122
   <212> PRT
   <213> Artificial Sequence
<220>
   <223> 2F2-VH - mouse/human chimeric polypeptide
<400> 60
   Glu Val Gln Leu Val Glu Ser Gly Gly Gly Leu Val Gln Pro Gly Arg
   1 5 10 15
   Ser Leu Arg Leu Ser Cys Ala Ala Ser Gly Phe Thr Phe Asn Asp Tyr
   20 25 30
   Ala Met His Trp Val Arg Gln Ala Pro Gly Lys Gly Leu Glu Trp Val
   35 40 45
   Ser Thr Ile Ser Trp Asn Ser Gly Ser Ile Gly Tyr Ala Asp Ser Val
   50 55 60
   Lys Gly Arg Phe Thr Ile Ser Arg Asp Asn Ala Lys Lys Ser Leu Tyr
   65 70 75 80
   Leu Gln Met Asn Ser Leu Arg Ala Glu Asp Thr Ala Leu Tyr Tyr Cys
   85 90 95
   Ala Lys Asp Ile Gln Tyr Gly Asn Tyr Tyr Tyr Gly Met Asp Val Trp
   100 105 110
   Gly Gln Gly Thr Thr Val Thr Val Ser Ser
   115 120
<210> 61
   <211> 122
   <212> PRT
   <213> Artificial Sequence
<220>
   <223> 7D8-VH - mouse/human chimeric polypeptide
<400> 61
   Glu Val Gln Leu Val Glu Ser Gly Gly Gly Leu Val Gln Pro Asp Arg
   1 5 10 15
   Ser Leu Arg Leu Ser Cys Ala Ala Ser Gly Phe Thr Phe His Asp Tyr
               20                  25                  30
   Ala Met His Trp Val Arg Gln Ala Pro Gly Lys Gly Leu Glu Trp Val
           35                  40                  45
   Ser Thr Ile Ser Trp Asn Ser Gly Thr Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Lys Asp Ile Gln Tyr Gly Asn Tyr Tyr Tyr Gly Met Asp Val Trp
               100                 105                 110
   Gly Gln Gly Thr Thr Val Thr Val Ser Ser
           115                 120
<210> 62
   <211> 125
   <212> PRT
   <213> Artificial Sequence
<220>
   <223> 11B8-VH - mouse/human chimeric polypeptide
<400> 62
   Glu Val Gln Leu Val Gln Ser Gly Gly Gly Leu Val His Pro Gly Gly
   1               5                   10                  15
   Ser Leu Arg Leu Ser Cys Thr Gly Ser Gly Phe Thr Phe Ser Tyr His
               20                  25                  30
   Ala Met His Trp Val Arg Gln Ala Pro Gly Lys Gly Leu Glu Trp Val
           35                  40                  45
   Ser Ile Ile Gly Thr Gly Gly Val Thr Tyr Tyr Ala Asp Ser Val Lys
       50                  55                  60
   Gly Arg Phe Thr Ile Ser Arg Asp Asn Val Lys Asn Ser Leu Tyr Leu
   65                  70                  75                  80
   Gln Met Asn Ser Leu Arg Ala Glu Asp Met Ala Val Tyr Tyr Cys Ala
                   85                  90                  95
   Arg Asp Tyr Tyr Gly Ala Gly Ser Phe Tyr Asp Gly Leu Tyr Gly Met
               100                 105                 110
   Asp Val Trp Gly Gln Gly Thr Thr Val Thr Val Ser Ser
           115                 120                 125
<210> 63
   <211> 6
   <212> PRT
   <213> Artificial Sequence
<220>
   <223> Vh Sequence at Kabat positions 8-13 of mouse/human modified antigen binding molecule
<400> 63
   Gly Ala Glu Val Lys Lys
   1               5
<210> 64
   <211> 6
   <212> PRT
   <213> Artificial Sequence
<220>
   <223> Vh Sequence at Kabat positions 8-13 of mouse/human modified antigen binding molecule
<400> 64
   Gly Pro Thr Leu Val Lys
   1               5
<210> 65
   <211> 6
   <212> PRT
   <213> Artificial Sequence
<220>
   <223> Sequence at the Kabat positions of mouse/human modified antigen binding molecule
<400> 65
   Gly Pro Val Leu Val Lys
   1 5
<210> 66
   <211> 6
   <212> PRT
   <213> Artificial Sequence
<220>
   <223> Vh Sequence at Kabat positions 8-13 of mouse/human modified antigen binding molecule
<400> 66
   Gly Pro Ala Leu Val Lys
   1 5
<210> 67
   <211> 6
   <212> PRT
   <213> Artificial Sequence
<220>
   <223> Vh Sequence at Kabat positions 8-13 of mouse/human modified antigen binding molecule
<400> 67
   Gly Gly Gly Leu Val Gln
   1 5
<210> 68
   <211> 6
   <212> PRT
   <213> Artificial Sequence
<220>
   <223> Vh Sequence at Kabat positions 8-13 of mouse/human modified antigen binding molecule
<400> 68
   Gly Gly Gly Leu Val Lys
   1 5
<210> 69
   <211> 6
   <212> PRT
   <213> Artificial Sequence
<220>
   <223> Vh Sequence at Kabat positions 8-13 of mouse/human modified antigen binding molecule
<400> 69
   Gly Gly Gly Leu Val Glu
   1               5
<210> 70
   <211> 6
   <212> PRT
   <213> Artificial Sequence
<220>
   <223> Vh Sequence at Kabat positions 8-13 of mouse/human modified antigen binding molecule
<400> 70
   Gly Gly Gly Val Val Arg
   1               5
<210> 71
   <211> 6
   <212> PRT
   <213> Artificial Sequence
<220>
   <223> Vh Sequence at Kabat positions 8-13 of mouse/human modified antigen binding molecule
<400> 71
   Gly Gly Gly Val Val Gln
   1               5
<210> 72
   <211> 6
   <212> PRT
   <213> Artificial Sequence
<220>
   <223> Vh Sequence at Kabat positions 8-13 of mouse/human modified antigen binding molecule
<400> 72
   Gly Gly Val Val Val Gln
   1               5
<210> 73
   <211> 6
   <212> PRT
   <213> Artificial Sequence
<220>
   <223> Vh Sequence at Kabat positions 8-13 of mouse/human modified antigen binding molecule
<400> 73
   Gly Gly Gly Leu Ile Gln
   1 5
<210> 74
   <211> 6
   <212> PRT
   <213> Artificial Sequence
<220>
   <223> Vh Sequence at Kabat positions 8-13 of mouse/human modified antigen binding molecule
<400> 74
   Arg Gly Val Leu Val Gln
   1 5
<210> 75
   <211> 6
   <212> PRT
   <213> Artificial Sequence
<220>
   <223> Vh Sequence at Kabat positions 8-13 of mouse/human modified antigen binding molecule
<400> 75
   Gly Pro Gly Leu Val Lys
   1 5
<210> 76
   <211> 6
   <212> PRT
   <213> Artificial Sequence
<220>
   <223> Vh Sequence at Kabat positions 8-13 of mouse/human modified antigen binding molecule
<400> 76
   Gly Ser Gly Leu Val Lys
   1 5
<210> 77
   <211> 6
   <212> PRT
   <213> Artificial Sequence
<220>
   <223> Vh Sequence at Kabat positions 8-13 of mouse/human modified antigen binding molecule
<400> 77
   Gly Ala Gly Leu Leu Lys
   1 5
<210> 78
   <211> 6
   <212> PRT
   <213> Artificial Sequence
<220>
   <223> Vh Sequence at Kabat positions 8-13 of mouse/human modified antigen binding molecule
<400> 78
   Gly Ser Glu Leu Lys Lys
   1               5
<210> 79
   <211> 6
   <212> PRT
   <213> Artificial Sequence
<220>
   <223> Vh Sequence at Kabat positions 8-13 of mouse/human modified antigen binding molecule
<400> 79
   Gly His Glu Val Lys Gln
   1               5
<210> 80
   <211> 987
   <212> DNA
   <213> Homo sapiens
<400> 80
   accaagggcc catcggtctt ccccctggca ccctcctcca agagcacctc tgggggcaca 60
   gcggccctgg gctgcctggt caaggactac ttccccgaac cggtgacggt gtcgtggaac 120
   tcaggcgccc tgaccagcgg cgtgcacacc ttcccggctg tcctacagtc ctcaggactc 180
   tactccctca gcagcgtggt gaccgtgccc tccagcagct tgggcaccca gacctacatc 240
   tgcaacgtga atcacaagcc cagcaacacc aaggtggaca agaaagcaga gcccaaatct 300
   tgtgacaaaa ctcacacatg cccaccgtgc ccagcacctg aactcctggg gggaccgtca 360
   gtcttcctct tccccccaaa acccaaggac accctcatga tctcccggac ccctgaggtc 420
   acatgcgtgg tggtggacgt gagccacgaa gaccctgagg tcaagttcaa ctggtacgtg 480
   gacggcgtgg aggtgcataa tgccaagaca aagccgcggg aggagcagta caacagcacg 540
   taccgtgtgg tcagcgtcct caccgtcctg caccaggact ggctgaatgg caaggagtac 600
   aagtgcaagg tctccaacaa agccctccca gcccccatcg agaaaaccat ctccaaagcc 660
   aaagggcagc cccgagaacc acaggtgtac accctgcccc catcccggga tgagctgacc 720
   aagaaccagg tcagcctgac ctgcctggtc aaaggcttct atcccagcga catcgccgtg 780
   gagtgggaga gcaatgggca gccggagaac aactacaaga ccacgcctcc cgtgctggac 840
   tccgacggct ccttcttcct ctacagcaag ctcaccgtgg acaagagcag gtggcagcag 900
   gggaacgtct tctcatgctc cgtgatgcat gaggctctgc acaaccacta cacgcagaag 960
   agcctctccc tgtctccggg taaatga 987
<210> 81
   <211> 328
   <212> PRT
   <213> Homo sapiens
<400> 81
   Thr Lys Gly Pro Ser Val Phe Pro Leu Ala Pro Ser Ser Lys Ser Thr
   1               5                   10                  15
   Ser Gly Gly Thr Ala Ala Leu Gly Cys Leu Val Lys Asp Tyr Phe Pro
               20                  25                  30
   Glu Pro Val Thr Val Ser Trp Asn Ser Gly Ala Leu Thr Ser Gly Val
           35                  40                  45
   His Thr Phe Pro Ala Val Leu Gln Ser Ser Gly Leu Tyr Ser Leu Ser
       50                  55                  60
   Ser Val Val Thr Val Pro Ser Ser Ser Leu Gly Thr Gln Thr Tyr Ile
   65                  70                  75                  80
   Cys Asn Val Asn His Lys Pro Ser Asn Thr Lys Val Asp Lys Lys Ala
                   85                  90                  95
   Glu Pro Lys Ser Cys Asp Lys Thr His Thr Cys Pro Pro Cys Pro Ala
               100                 105                 110
   Pro Glu Leu Leu Gly Gly Pro Ser Val Phe Leu Phe Pro Pro Lys Pro
           115                 120                 125
   Lys Asp Thr Leu Met Ile Ser Arg Thr Pro Glu Val Thr Cys Val Val
       130                 135                 140
   Val Asp Val Ser His Glu Asp Pro Glu Val Lys Phe Asn Trp Tyr Val
   145                 150                 155                 160
   Asp Gly Val Glu Val His Asn Ala Lys Thr Lys Pro Arg Glu Glu Gln
                   165                 170                 175
   Tyr Asn Ser Thr Tyr Arg Val Val Ser Val Leu Thr Val Leu His Gln
               180                 185                 190
   Asp Trp Leu Asn Gly Lys Glu Tyr Lys Cys Lys Val Ser Asn Lys Ala
           195                 200                 205
   Leu Pro Ala Pro Ile Glu Lys Thr Ile Ser Lys Ala Lys Gly Gln Pro
           210                 215                 220
   Arg Glu Pro Gln Val Tyr Thr Leu Pro Pro Ser Arg Asp Glu Leu Thr
   225                 230                 235                 240
   Lys Asn Gln Val Ser Leu Thr Cys Leu Val Lys Gly Phe Tyr Pro Ser
                   245                 250                 255
   Asp Ile Ala Val Glu Trp Glu Ser Asn Gly Gln Pro Glu Asn Asn Tyr
               260                 265                 270
   Lys Thr Thr Pro Pro Val Leu Asp Ser Asp Gly Ser Phe Phe Leu Tyr
           275                 280                 285
   Ser Lys Leu Thr Val Asp Lys Ser Arg Trp Gln Gln Gly Asn Val Phe
       290                 295                 300
   Ser Cys Ser Val Met His Glu Ala Leu His Asn His Tyr Thr Gln Lys
   305                 310                 315                 320
   Ser Leu Ser Leu Ser Pro Gly Lys
                   325
<210> 82
   <211> 618
   <212> DNA
   <213> Homo sapiens
<400> 82
   ctcatgaata tgcaaataac ctgagattta ctgaagtaaa tacagatctg tcctgtgccc      60
   tgagagcatc acccagcaac cacatctgtc ctctagagaa tcccctgaga gctccgttcc     120
   tcaccatgga ctggacctgg aggatcctct tcttggtggc agcagccaca ggtaagaggc     180
   tccctagtcc cagtgatgag aaagaggatt gagtccagtc cagggagatc tcatccactt     240
   ctgtgttctc tccacaggag cccactccca ggtgcagctg gtgcagtctg gggctgaggt     300
   gaagaagcct ggggcctcag tgaaggtctc ctgcaaggct tctggataca ccttcaccgg     360
   ctactatatg cactgggtgc gacaggcccc tggacaaggg cttgagtgga tgggacggat     420
   caaccctaac agtggtggca caaactatgc acagaagttt cagggcaggg tcaccagtac     480
   cagggacacg tccatcagca cagcctacat ggagctgagc aggctgagat ctgacgacac     540
   ggtcgtgtat tactgtgcga gagacacagt gtgaaaaccc acatcctgag ggtgtcagaa     600
   accccaggga ggaggcag                                                   618
<210> 83
   <211> 613
   <212> DNA
   <213> Homo sapiens
<400> 83
   ccagctccac cctcctctgg gttgaaaaag ccgagcacag gtaccagctc agtgactcct     60
   gtgcaccacc atggacacac tttgctccac gctcctgctg ctgaccatcc cttcatgtga    120
   gtgctgtggt cagggactcc ttcacgggtg aaacatcagt tttcttgttt gtgggcttca    180
   tcttcttatg ctttctccac aggggtcttg tcccagatca ccttgaagga gtctggtcct    240
   acgctggtga aacccacaca gaccctcacg ctgacctgca ccttctctgg gttctcactc    300
   agcactagtg gagtgggtgt gggctggatc cgtcagcccc caggaaaggc cctggagtgg    360
   cttgcactca tttattggaa tgatgataag cgctacagcc catctctgaa gagcaggctc    420
   accatcacca aggacacctc caaaaaccag gtggtcctta caatgaccaa catggaccct    480
   gtggacacag ccacatatta ctgtgcacac agaccacaaa gacacagccc agggcacctc    540
   ctgtacaaaa acccaggctg cttctcattg gtgctccctc cccacctctg cagaacagga    600
   aagtctgtct gct                                                       613
<210> 84
   <211> 546
   <212> DNA
   <213> Homo sapiens
<400> 84
   agtgactcct gtgccccacc atggacacac tttgctacac actcctgctg ctgaccaccc     60
   cttcctgtga gtgctgtggt cagggacttc ctcagaagtg aaacatcagt tgtctccttt    120
   gtgggcttca tcttcttatg tcttctccac aggggtcttg tcccaggtca ccttgaagga    180
   gtctggtcct gtgctggtga aacccacaga gaccctcacg ctgacctgca ccgtctctgg    240
   gttctcactc agcaatgcta gaatgggtgt gagctggatc cgtcagcccc cagggaaggc    300
   cctggagtgg cttgcacaca ttttttcgaa tgacgaaaaa tcctacagca catctctgaa    360
   gagcaggctc accatctcca aggacacctc caaaagccag gtggtcctta ccatgaccaa    420
   catggaccct gtggacacag ccacatatta ctgtgcacgg ataccacaga gacacagccc    480
   aggatgcctc ctgtacaaga acctagctgc atctcagtgg tgctccctcc ctacctctgc    540
   agaaca                                                               546
<210> 85
   <211> 460
   <212> DNA
   <213> Homo sapiens
<400> 85
   atggacatac tttgttccac gctcctgcta ctgactgtcc cgtcctgtga gtgctgtggt 60
   caggtagtac ttcagaagca aaaaatctat tctctccttt gtgggcttca tcttcttatg 120
   tcttctccac aggggtctta tcccaggtca ccttgaggga gtctggtcct gcgctggtga 180
   aacccacaca gaccctcaca ctgacctgca ccttctctgg gttctcactc agcactagtg 240
   gaatgtgtgt gagctggatc cgtcagcccc cagggaaggc cctggagtgg cttgcactca 300
   ttgattggga tgatgataaa tactacagca catctctgaa gaccaggctc accatctcca 360
   aggacacctc caaaaaccag gtggtcctta caatgaccaa catggaccct gtggacacag 420
   ccacgtatta ctgtgcacgg ataccacaga gacacaccca 460
<210> 86
   <211> 877
   <212> DNA
   <213> Homo sapiens
<400> 86
   acagcctatt cctccagcat cccactagag cttcttatat agtaggagac atgcaaatag 60
   ggccctccct ctactgatga aaaccaaccc aaccctgacc ctgcaggtct cagagaggag 120
   ccttagccct ggactccaag gcctttccac ttggtgatca gcactgagca cagaggactc 180
   accatggaat tggggctgag ctgggttttc cttgttgcta ttttagaagg tgattcatgg 240
   aaaactagga agattgagtg tgtgtggata tgagtgtgag aaacagtgga tttgtgtggc 300
   agtttctgac cttggtgtct ctttgtttgc aggtgtccag tgtgaggtgc agctggtgga 360
   gtctggggga ggcttggtcc agcctggggg gtccctgaga ctctcctgtg cagcctctgg 420
   attcaccttt agtagctatt ggatgagctg ggtccgccag gctccaggga aggggctgga 480
   gtgggtggcc aacataaagc aagatggaag tgagaaatac tatgtggact ctgtgaaggg 540
   ccgattcacc atctccagag acaacgccaa gaactcactg tatctgcaaa tgaacagcct 600
   gagagccgag gacacggctg tgtattactg tgcgagagac acagtgaggg gaagtcagtg 660
   tgagcccaga cacaaacctc cctgcagggg tcccttggga ccaccagggg gcgacagggc 720
   attgagcact gggctgtctc cagggcaggt gcaggtgctg ctgagggctg gcttcctgtc 780
   gcggtctggg gctgcctcgt cgtcaaattt ccccaggaac ttctccagat ttacaattct 840
   gtactgacat ttcatgtctc taaatgcaat acttttt 877
<210> 87
   <211> 557
   <212> DNA
   <213> Homo sapiens
<400> 87
   ccaggagttt ccattcggtg atcagcactg aacacagagg actcaccatg gagtttgggc 60
   tgagctgggt tttccttgtt gctataataa aaggtgattt atggagaact agagacattg 120
   agtggacgtg agtgagataa gcagtgaata tatgtggcag tttctgacta ggttgtctct 180
   gtgtttgcag gtgtccagtg tcaggtgcag ctggtggagt ctgggggagg cttggtcaag 240
   cctggagggt ccctgagact ctcctgtgca gcctctggat tcaccttcag tgactactac 300
   atgagctgga tccgccaggc tccagggaag gggctggagt gggtttcata cattagtagt 360
   agtggtagta ccatatacta cgcagactct gtgaagggcc gattcaccat ctccagggac 420
   aacgccaaga actcactgta tctgcaaatg aacagcctga gagccgagga cacggccgtg 480
   tattactgtg cgagagacac agtgagggga agtcagtgtg agcccagaca caaacctccc 540
   tgcagggggt cccttgg 557
<210> 88
   <211> 727
   <212> DNA
   <213> Homo sapiens
<400> 88
   agatttaaga accttgcacc tggtacccgt tgctcttctt gtaaccattt gtcttttaag 60
   ttgtttatca ctctgtaact attttgatta ttttgattct tgcatgtttt tacttctgta 120
   aaattattac atttgagtcc ctctcccctt cctaaaccta ggtataaaat ttactcgagc 180
   cccttcctcg tggccgagag aattttgagc atgagctgtc tctttggcag ccggcttaat 240
   aaaggactct taattcgtct caaagtgtgg cgttttctta actcacctgg gtacaacagt 300
   gcagctggtg gagtctgggg gaggcttggt agagcctggg gggtccctga gactctcctg 360
   tgcagcctct ggattcacct tcagtaacag tgacatgaac tgggtccgcc aggctccagg 420
   aaaggggctg gagtgggtat cgggtgttag ttggaatggc agtaggacgc actatgcaga 480
   ctctgtgaag ggccgattca tcatctccag agacaattcc aggaacttcc tgtatcagca 540
   aatgaacagc ctgaggcccg aggacatggc tgtgtattac tgtgtgagaa acactgtgag 600
   aggacggaag tgtgagccca gacacaaacc tcctgcagga acgttggggg aaatcagctg 660
   cagggggcgc tcaagaccca ctcatcagag tcaaccccag agcaggtgca catggaggct 720
   gggtttt 727
<210> 89
   <211> 514
   <212> DNA
   <213> Homo sapiens
<400> 89
   ggactcgcca tggagtttgg gctgagctgg gttttccttg ttgctatttt aaaaggtgat 60
   tcatggatca atagagatgt tgagtgtgag tgaacacgag tgagagaaac agtggatttg 120
   tgtggcagtt tctgaccagg gtgtctctgt gtttgcaggt gtccagtgtg aggtgcagct 180
   ggtggagtct gggggaggtg tggtacggcc tggggggtcc ctgagactct cctgtgcagc 240
   ctctggattc acctttgatg attatggcat gagctgggtc cgccaagctc cagggaaggg 300
   gctggagtgg gtctctggta ttaattggaa tggtggtagc acaggttatg cagactctgt 360
   gaagggccga ttcaccatct ccagagacaa cgccaagaac tccctgtatc tgcaaatgaa 420
   cagtctgaga gccgaggaca cggccttgta tcactgtgcg agagacacag tgaggggaag 480
   ccagtgagag cccagacaca aacgtccctg cagg 514
<210> 90
   <211> 412
   <212> DNA
   <213> Homo sapiens
<400> 90
   tgattcatgg agaaatagag agactgagtg tgagtgaaca tgagtgagaa aaactggatt 60
   tgtgtggcat tttctgataa cggtgtcctt ctgtttgcag gtgtccagtg tcaggtgcag 120
   ctggtggagt ctgggggagg cgtggtccag cctgggaggt ccctgagact ctcctgtgca 180
   gcgtctggat tcaccttcag tagctatggc atgcactggg tccgccaggc tccaggcaag 240
   gggctggagt gggtggcagt tatatggtat gatggaagta ataaatacta tgcagactcc 300
   gtgaagggcc gattcaccat ctccagagac aattccaaga acacgctgta tctgcaaatg 360
   aacagcctga gagccgagga cacggctgtg tattactgtg cgagagacac ag 412
<210> 91
   <211> 870
   <212> DNA
   <213> Homo sapiens
<220>
   <221> misc_feature
   <222> (43)..(43)
   <223> n is a, c, g, or t
<220>
   <221> misc_feature
   <222> (824)..(824)
   <223> n is a, c, g, or t
<400> 91
   catctgttac agaactcatt atatagtagg agacatccaa atngggtccc tccctctgct     60
   gatgaaaacc agcccagccc tgaccctgca gctctgggag aggagcccca gccctgagat    120
   tcccaggtgt ttccattcgg tgatcagcac tgaacacaga gaacgcacca tggagtttgg    180
   actgagctgg gttttccttg ttgctatttt aaaaggtgat tcatggataa atagagatgt    240
   tgagtgtgag tgaacatgag tgagagaaac agtggatatg tgtggcagtg tctgaccagg    300
   gtgtctctgt gtttgcaggt gtccagtgtg aagtgcagct ggtggagtct gggggagtcg    360
   tggtacagcc tggggggtcc ctgagactct cctgtgcagc ctctggattc acctttgatg    420
   attataccat gcactgggtc cgtcaagctc cggggaaggg tctggagtgg gtctctctta    480
   ttagttggga tggtggtagc acatactatg cagactctgt gaagggccga ttcaccatct    540
   ccagagacaa cagcaaaaac tccctgtatc tgcaaatgaa cagtctgaga actgaggaca    600
   ccgccttgta ttactgtgca aaagatacac agtgagggga agtcagcgag agcccagaca    660
   aaaacctcgc tgcaggaaga caggaggggc ctgggctgca gaggccactc aagacacact    720
   gagcataggg ttaactctgg gacaagttgc tcaggaaggt taagagctgg tttcctttca    780
   gagtcttcac aaatttctcc atctaacagt ttccccagga accngtctag atctgtgatc    840
   ttggatctgc tgaaactgcc tgtgtcacct                                     870
<210> 92
   <211> 724
   <212> DNA
   <213> Homo sapiens
<220>
   <221> misc_feature
   <222> (560)..(560)
   <223> n is a, c, g, or t
<400> 92
   ccattcggtg atcagcactg aacacagagg actcaccatg gagttttggc tgagctgggt     60
   tttccttgtt gctattttaa aaggtgattc atggagaact agagatattg agtgtgagtg    120
   aacacgagtg agagaaacag tggatatgtg tggcagtttc taaccaatgt ctctgtgttt    180
   gcaggtgtcc agtgtgaggt gcagctggtg gagtctggag gaggcttgat ccagcctggg    240
   gggtccctga gactctcctg tgcagcctct gggttcaccg tcagtagcaa ctacatgagc    300
   tgggtccgcc aggctccagg gaaggggctg gagtgggtct cagttattta tagcggtggt    360
   agcacatact acgcagactc cgtgaagggc cgattcacca tctccagaga caattccaag    420
   aacacgctgt atcttcaaat gaacagcctg agagccgagg acacggccgt gtattactgt    480
   gcgagagaca cagtgagggg aagtcattgt gcgcccagac acaaacctcc ctgcaggaac    540
   gctgggggga aatcagcggn agggggcgct caggagccac tgatcagagt cagccccgga    600
   ggcaggtgca gatggaggct gatttccttg tcaggatgtg gggacttttg tcttcttctg    660
   acgggttccc caggggaacc tctctaagtt tagcattctg tgcctatgaa cgtcttctct    720
   aagt                                                                 724
<210> 93
   <211> 288
   <212> DNA
   <213> Homo sapiens
<400> 93
   gaggtgcagc tggtggagtc tcggggagtc ttggtacagc ctggggggtc cctgagactc     60
   tcctgtgcag cctctggatt caccgtcagt agcaatgaga tgagctgggt ccgccaggct    120
   ccagggaagg gtctggagtg ggtctcatcc attagtggtg gtagcacata ctacgcagac    180
   tccaggaagg gcagattcac catctccaga gacaattcca agaacacgct gcatcttcaa    240
   atgaacagcc tgagagctga ggacacggct gtgtattact gtaagaaa                 288
<210> 94
   <211> 626
   <212> DNA
   <213> Homo sapiens
<400> 94
   catccctttt cacctcttta tacaaaggca ccacctacat gcaaatcctc acttaggcac     60
   ccacaggaaa ccaccacaca tttccttaaa ttcagggtcc agctcacatg ggaaatactt    120
   tctgagagct catgggcctc ctgcacaaga acatgaaaca cctgtggttc ttcctcctcc    180
   tggtggcagc tcccagatgt gagtgtctca aggctgcaga catgggggta tgggaggtgc    240
   ctctgatccc agggctcact gtgggtctct ctgttcacag gggtcctgtc tcaggtgcag    300
   ctgcaggagt cgggcccagg actggtgaag cctccgggga ccctgtccct cacctgcgct    360
   gtctctggtg gctccatcag cagtagtaac tggtggagtt gggtccgcca gcccccaggg    420
   aaggggctgg agtggattgg ggaaatctat catagtggga gcaccaacta caacccgtcc    480
   ctcaagagtc gagtcaccat atcagtagac aagtccaaga accagttctc cctgaagctg    540
   agctctgtga ccgccgcgga cacggccgtg tattgctgtg cgagagacac agtgagggga    600
   ggtgagtgtg agcccagaca caaacc                                         626
<210> 95
   <211> 299
   <212> DNA
   <213> Homo sapiens
<400> 95
   cagctgcagc tgcaggagtc cggctcagga ctggtgaagc cttcacagac cctgtccctc     60
   acctgcgctg tctctggtgg ctccatcagc agtggtggtt actcctggag ctggatccgg    120
   cagccaccag ggaagggcct ggagtggatt gggtacatct atcatagtgg gagcacctac    180
   tacaacccgt ccctcaagag tcgagtcacc atatcagtag acaggtccaa gaaccagttc    240
   tccctgaagc tgagctctgt gaccgccgcg gacacggccg tgtattactg tgccagaga     299
<210> 96
   <211> 410
   <212> DNA
   <213> Homo sapiens
<400> 96
   gggtcctgtc ccaggtgcag ctacagcagt ggggcgcagg actgttgaag ccttcggaga 60
   ccctgtccct cacctgcgct gtctatggtg ggtccttcag tggttactac tggagctgga 120
   tccgccagcc cccagggaag gggctggagt ggattgggga aatcaatcat agtggaagca 180
   ccaactacaa cccgtccctc aagagtcgag tcaccatatc agtagacacg tccaagaacc 240
   agttctccct gaagctgagc tctgtgaccg ccgcggacac ggctgtgtat tactgtgcga 300
   gaggcacagt gaggggaggt gagtgtgagc ccagacaaaa acctccctgc aggtaggcag 360
   agggggcggg cgcaggtact gctcaagacc agcaggtggc gcgcggcgcc 410
<210> 97
   <211> 700
   <212> DNA
   <213> Homo sapiens
<220>
   <221> misc_feature
   <222> (19) ..(19)
   <223> n is a, c, g, or t
<220>
   <221> misc_feature
   <222> (46)..(46)
   <223> n is a, c, g, or t
<400> 97
   aggttctggg ttataaacnc tgtagactcc tcccttcagg gcaggntgac caactatgca 60
   aatgcaagtg ggggcctccc cacttaaacc cagggctccc ctccacagtg agtctccctc 120
   actgcccagc tgggatctca gggcttcatt ttctgtcctc caccatcatg gggtcaaccg 180
   ccatcctcgc cctcctcctg gctgttctcc aaggtcagtc ctgccgaggg cttgaggtca 240
   cagaggagaa cgggtggaaa ggagcccctg attcaaattt tgtgtctccc ccacaggagt 300
   ctgttccgag gtgcagctgg tgcagtctgg agcagaggtg aaaaagcccg gggagtctct 360
   gaagatctcc tgtaagggtt ctggatacag ctttaccagc tactggatcg gctgggtgcg 420
   ccagatgccc gggaaaggcc tggagtggat ggggatcatc tatcctggtg actctgatac 480
   cagatacagc ccgtccttcc aaggccaggt caccatctca gccgacaagt ccatcagcac 540
   cgcctacctg cagtggagca gcctgaaggc ctcggacacc gccatgtatt actgtgcgag 600
   acacacagtg agagaaacca gccccgagcc cgtctaaaac cctccacacc gcaggtgcag 660
   aatgagctgc tagagactca ctccccaggg gcctctctat 700
<210> 98
   <211> 650
   <212> DNA
   <213> Homo sapiens
<220>
   <221> misc_feature
   <222> (621)..(621)
   <223> n is a, c, g, or t
<400> 98
   agggcagtca ccagagctcc agacaatgtc tgtctccttc ctcatcttcc tgcccgtgct    60
   gggcctccca tggggtcagt gtcagggaga tgccgtattc acagcagcat tcacagactg   120
   aggggtgttt cactttgctg tttccttttg tctccaggtg tcctgtcaca ggtacagctg   180
   cagcagtcag gtccaggact ggtgaagccc tcgcagaccc tctcactcac ctgtgccatc   240
   tccggggaca gtgtctctag caacagtgct gcttggaact ggatcaggca gtccccatcg   300
   agaggccttg agtggctggg aaggacatac tacaggtcca agtggtataa tgattatgca   360
   gtatctgtga aaagtcgaat aaccatcaac ccagacacat ccaagaacca gttctccctg   420
   cagctgaact ctgtgactcc cgaggacacg gctgtgtatt actgtgcaag agacacagtg   480
   aggggaagtc agtgtgagcc cagacacaaa cctccctgca gggatgctca ggaccccaga   540
   aggcacccag cactaccagc gcagggccca gaccaggagc aggtgtggag ttaagcaaaa   600
   atggaacttc ttgctgtgtc ntaaactgtt gttgtttttt tttttttttt              650
<210> 99
   <211> 388
   <212> DNA
   <213> Homo sapiens
<400> 99
   taaggggctc cccagtcact gggctgaggg agaaaccagc acagtcaagt gagacttcat    60
   gcactcccat ctcctctcca caggtgccca ctcccaggtg cagctggtgc aatctgggtc   120
   tgagttgaag aagcctgggg cctcagtgaa ggtttcctgc aaggcttctg gatacacctt   180
   cactagctat gctatgaatt gggtgcgaca ggcccctgga caagggcttg agtggatggg   240
   atggatcaac accaacactg ggaacccaac gtatgcccag ggcttcacag gacggtttgt   300
   cttctccttg gacacctctg tcagcacggc atatctgcag atctgcagcc taaaggctga   360
   ggacactgcc gtgtattact gtgcgaga                                      388
<210> 100
   <211> 294
   <212> DNA
   <213> Homo sapiens
<400> 100
   caggtgcagc tggtgcagtc tggccatgag gtgaagcagc ctggggcctc agtgaaggtc     60
   tcctgcaagg cttctggtta cagtttcacc acctatggta tgaattgggt gccacaggcc    120
   cctggacaag ggcttgagtg gatgggatgg ttcaacacct acactgggaa cccaacatat    180
   gcccagggct tcacaggacg gtttgtcttc tccatggaca cctctgccag cacagcatac    240
   ctgcagatca gcagcctaaa ggctgaggac atggccatgt attactgtgc gaga          294
<210> 101
   <211> 6
   <212> PRT
   <213> Artificial Sequence
<220>
   <223> Vh Sequence at Kabat positions 108-113 of mouse/human modified antigen binding molecule
<400> 101
   Gly Ala Glu Leu Lys Lys
   1               5
<210> 102
   <211> 6
   <212> PRT
   <213> Artificial Sequence
<220>
   <223> Vh Sequence at Kabat positions 108-113 of mouse/human modified antigen binding molecule
<400> 102
   Gly Ala Glu Val Val Lys
   1               5
<210> 103
   <211> 6
   <212> PRT
   <213> Artificial Sequence
<220>
   <223> Vh Sequence at Kabat positions 108-113 of mouse/human modified antigen binding molecule
<400> 103
   Gly Gly Glu Val Lys Lys
   1               5
<210> 104
   <211> 6
   <212> PRT
   <213> Artificial Sequence
<220>
   <223> Vh Sequence at Kabat positions 108-113 of mouse/human modified antigen binding molecule
<210> 105
   <211> 6
   <212> PRT
   <213> Artificial Sequence
<220>
   <223> Vh Sequence at Kabat positions 108-113 of mouse/human modified antigen binding molecule
<400> 105
   Gly Gly Gly Val Val Lys
   1 5
<210> 106
   <211> 6
   <212> PRT
   <213> Artificial Sequence
<220>
   <223> Vh Sequence at Kabat positions 108-113 of mouse/human modified antigen binding molecule
<400> 106
   Leu Val Thr Val Ser Ser
   1 5
<210> 107
   <211> 6
   <212> PRT
   <213> Artificial Sequence
<220>
   <223> Vh Sequence at Kabat positions 108-113 of mouse/human modified antigen binding molecule
<400> 107
   Leu Val Ile Val Ser Ser
   1 5
<210> 108
   <211> 6
   <212> PRT
   <213> Artificial Sequence
<220>
   <223> Vh Sequence at Kabat positions 108-113 of mouse/human modified antigen binding molecule
<400> 108
   Leu Val Thr Val Ile Ser
   1 5
<210> 109
   <211> 6
   <212> PRT
   <213> Artificial Sequence
<220>
   <223> Vh Sequence at Kabat positions 108-113 of mouse/human modified antigen binding molecule
<400> 109
   Leu Val Ile Val Ile Ser
   1 5
<210> 110
   <211> 6
   <212> PRT
   <213> Artificial Sequence
<220>
   <223> Vh Sequence at Kabat positions 108-113 of mouse/human modified antigen binding molecule
<400> 110
   Leu Val Gly Val Ser Ser
   1 5
<210> 111
   <211> 6
   <212> PRT
   <213> Artificial Sequence
<220>
   <223> Vh Sequence at Kabat positions 108-113 of mouse/human modified antigen binding molecule
<400> 111
   Leu Val Thr Val Gly Ser
   1 5
<210> 112
   <211> 6
   <212> PRT
   <213> Artificial Sequence
<220>
   <223> Vh Sequence at Kabat positions 108-113 of mouse/human modified antigen binding molecule
<400> 112
   Leu Val Gly Val Gly Ser
   1 5
<210> 113
   <211> 6
   <212> PRT
   <213> Artificial Sequence
<220>
   <223> Vh Sequence at Kabat positions 108-113 of mouse/human modified antigen binding molecule
<400> 113
   Leu Val Ala Val Ser Ser
   1 5
<210> 114
   <211> 6
   <212> PRT
   <213> Artificial Sequence
<220>
   <223> Vh Sequence at Kabat positions 108-113 of mouse/human modified antigen binding molecule
<400> 114
   Leu Val Thr Val Ala Ser
   1 5
<210> 115
   <211> 6
   <212> PRT
   <213> Artificial Sequence
<220>
   <223> Vh Sequence at Kabat positions 108-113 of mouse/human modified antigen binding molecule
<400> 115
   Leu Val Ala Val Ala Ser
   1 5
<210> 116
   <211> 6
   <212> PRT
   <213> Artificial Sequence
<220>
   <223> Vh Sequence at Kabat positions 108-113 of mouse/human modified antigen binding molecule
<400> 116
   Leu Val Val Val Ser Ser
   1 5
<210> 117
   <211> 6
   <212> PRT
   <213> Artificial Sequence
<220>
   <223> Vh Sequence at Kabat positions 108-113 of mouse/human modified antigen binding molecule
<400> 117
   Leu Val Thr Val Val Ser
   1 5
<210> 118
   <211> 6
   <212> PRT
   <213> Artificial Sequence
<220>
   <223> Vh Sequence at Kabat positions 108-113 of mouse/human modified antigen binding molecule
<400> 118
   Leu Val Val Val Val Ser
   1 5
<210> 119
   <211> 6
   <212> PRT
   <213> Artificial Sequence
<220>
   <223> Vh Sequence at Kabat positions 108-113 of mouse/human modified antigen binding molecule
<400> 119
   Leu Val Leu Val Ser Ser
   1 5
<210> 120
   <211> 6
   <212> PRT
   <213> Artificial Sequence
<220>
   <223> Vh Sequence at Kabat positions 108-113 of mouse/human modified antigen binding molecule
<400> 120
   Leu Val Thr Val Leu Ser
   1 5
<210> 121
   <211> 6
   <212> PRT
   <213> Artificial Sequence
<220>
   <223> Vh Sequence at Kabat positions 108-113 of mouse/human modified antigen binding molecule
<400> 121
   Leu Val Leu Val Leu Ser
   1 5
<210> 122
   <211> 6
   <212> PRT
   <213> Artificial Sequence
<220>
   <223> Vh Sequence at Kabat positions 108-113 of mouse/human modified antigen binding molecule
<400> 122
   Leu Val Ser Val Ser Ser
   1 5
<210> 123
   <211> 6
   <212> PRT
   <213> Artificial Sequence
<220>
   <223> Vh Sequence at Kabat positions 108-113 of mouse/human modified antigen binding molecule
<400> 123
   Leu Val Thr Val Thr Ser
   1 5
<210> 124
   <211> 119
   <212> PRT
   <213> Artificial Sequence
<220>
   <223> B-HH2A construct of a mouse/human modified antigen binding molecule
<400> 124
   Gln Val Gln Leu Val Gln Ser Gly Ala Glu Leu Lys Lys Pro Gly Ser
   1 5 10 15
   Ser Val Lys Val Ser Cys Lys Ala Ser Gly Tyr Ala Phe Ser Tyr Ser
   20 25 30
   Trp Met Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 125
   <211> 119
   <212> PRT
   <213> Artificial Sequence
<220>
   <223> B-HH2B construct of a mouse/human modified antigen binding molecule
<400> 125
   Gln Val Gln Leu Val Gln Ser Gly Ala Glu Val Val Lys Pro Gly Ser
   1 5 10 15
   Ser Val Lys Val Ser Cys Lys Ala Ser Gly Tyr Ala Phe Ser Tyr Ser
   20 25 30
   Trp Met Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 126
   <211> 119
   <212> PRT
   <213> Artificial Sequence
<220>
   <223> B-HH2C construct of a mouse/human modified antigen binding molecule
<400> 126
   Gln Val Gln Leu Val Gln Ser Gly Gly Glu Val Lys Lys Pro Gly Ser
   1 5 10 15
   Ser Val Lys Val Ser Cys Lys Ala Ser Gly Tyr Ala Phe Ser Tyr Ser
   20 25 30
   Trp Met Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ser Ser
   115
<210> 127
   <211> 119
   <212> PRT
   <213> Artificial Sequence
<220>
   <223> B-HH2D construct of a mouse/human modified antigen binding molecule
<400> 127
   Gln Val Gln Leu Val Gln Ser Gly Ala Gly Val Lys Lys Pro Gly Ser
   1               5                   10                  15
   Ser Val Lys Val Ser Cys Lys Ala Ser Gly Tyr Ala Phe Ser Tyr Ser
               20                  25                  30
   Trp Met Asn Trp Val Arg Gln Ala Pro Gly Gln Gly Leu Glu Trp Met
           35                  40                  45
   Gly Arg Ile Phe Pro Gly Asp Gly Asp Thr Asp Tyr Asn Gly Lys Phe
       50                  55                  60
   Lys Gly Arg Val Thr Ile Thr Ala Asp Lys Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asn Val Phe Asp Gly Tyr Trp Leu Val Tyr Trp Gly Gln Gly
               100                 105                 110
   Thr Leu Val Thr Val Ser Ser
           115
<210> 128
   <211> 119
   <212> PRT
   <213> Artificial Sequence
<220>
   <223> B-HH2E construct of a mouse/human modified antigen binding molecule
<400> 128
   Gln Val Gln Leu Val Gln Ser Gly Ala Glu Val Lys Lys Pro Gly Ser
   1               5                   10                  15
   Ser Val Lys Val Ser Cys Lys Ala Ser Gly Tyr Ala Phe Ser Tyr Ser
   20 25 30
   Trp Met Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Ile Val Ser Ser
   115
<210> 129
   <211> 119
   <212> PRT
   <213> Artificial Sequence
<220>
   <223> B-HH2F construct of a mouse/human modified antigen binding molecule
<400> 129
   Gln Val Gln Leu Val Gln Ser Gly Ala Glu Val Lys Lys Pro Gly Ser
   1 5 10 15
   Ser Val Lys Val Ser Cys Lys Ala Ser Gly Tyr Ala Phe Ser Tyr Ser
   20 25 30
   Trp Met Asn Trp Val Arg Gln Ala Pro Gly Gln Gly Leu Glu Trp Met
   35 40 45
   Gly Arg Ile Phe Pro Gly Asp Gly Asp Thr Asp Tyr Asn Gly Lys Phe
   50 55 60
   Lys Gly Arg Val Thr Ile Thr Ala Asp Lys Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Asn Val Phe Asp Gly Tyr Trp Leu Val Tyr Trp Gly Gln Gly
   100 105 110
   Thr Leu Val Thr Val Ile Ser
   115
<210> 130
   <211> 115
   <212> PRT
   <213> Artificial Sequence
<220>
   <223> B-KV10 construct of a mouse/human modified antigen binding molecule
<400> 130
   Asp Ile Val Met Thr Gln Thr Pro Leu Ser Leu Pro Val Thr Pro Gly
   1 5 10 15
   Glu Pro Ala Ser Ile Ser Cys Arg Ser Ser Lys Ser Leu Leu His Ser
   20 25 30
   Asn Gly Ile Thr Tyr Leu Tyr Trp Tyr Leu Gln Lys Ala Gly Gln Ser
   35 40 45
   Pro Gln Leu Leu Ile Tyr Gln Met Ser Asn Leu Val Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Ala Gln Asn
   85 90 95
   Leu Glu Leu Pro Tyr Thr Phe Gly Gly Gly Thr Lys Val Glu Ile Lys
   100 105 110
   Arg Thr Val
   115
<210> 131
   <211> 115
   <212> PRT
   <213> Artificial Sequence
<220>
   <223> B-KV11 construct of a mouse/human modified antigen binding molecule
<400> 131
   Asp Ile Val Met Thr Gln Thr Pro Leu Ser Leu Pro Val Thr Pro Gly
   1 5 10 15
   Glu Pro Ala Ser Ile Ser Cys Arg Ser Ser Lys Ser Leu Leu His Ser
   20 25 30
   Asn Gly Ile Thr Tyr Leu Tyr Trp Tyr Leu Gln Lys Pro Gly Gln Ser
   35 40 45
   Pro Gln Leu Leu Ile Tyr Gln Met Ser Asn Leu Val Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Pro Glu Asp Val Gly Val Tyr Tyr Cys Ala Gln Asn
   85 90 95
   Leu Glu Leu Pro Tyr Thr Phe Gly Gly Gly Thr Lys Val Glu Ile Lys
   100 105 110
   Arg Thr Val
   115
<210> 132
   <211> 115
   <212> PRT
   <213> Artificial Sequence
<220>
   <223> B-KV12 construct of a mouse/human modified antigen binding molecule
<400> 132
   Asp Ile Val Met Thr Gln Thr Pro Leu Ser Leu Pro Val Thr Pro Gly
   1 5 10 15
   Glu Pro Ala Ser Ile Ser Cys Arg Ser Ser Lys Ser Leu Leu His Ser
   20 25 30
   Asn Gly Ile Thr Tyr Leu Tyr Trp Tyr Leu Gln Lys Pro Gly Gln Ser
   35 40 45
   Pro Gln Leu Leu Ile Tyr Gln Met Ser Asn Leu Val Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Phe Gly Val Tyr Tyr Cys Ala Gln Asn
   85 90 95
   Leu Glu Leu Pro Tyr Thr Phe Gly Gly Gly Thr Lys Val Glu Ile Lys
   100 105 110
   Arg Thr Val
   115
<210> 133
   <211> 115
   <212> PRT
   <213> Artificial Sequence
<220>
   <223> B-KV13 construct of a mouse/human modified antigen binding molecule
<400> 133
   Asp Ile Val Met Thr Gln Thr Pro Leu Ser Leu Pro Val Thr Pro Gly
   1 5 10 15
   Glu Pro Ala Ser Ile Ser Cys Arg Ser Ser Lys Ser Leu Leu His Ser
   20 25 30
   Asn Gly Ile Thr Tyr Leu Tyr Trp Tyr Leu Gln Lys Pro Gly Gln Ser
   35 40 45
   Pro Gln Leu Leu Ile Tyr Gln Met Ser Asn Leu Val Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Ala Gln Asn
   85 90 95
   Leu Glu Leu Pro Tyr Thr Phe Gly Gly Gly Thr Lys Val Ala Ile Lys
   100 105 110
   Arg Thr Val
   115
<210> 134
   <211> 115
   <212> PRT
   <213> Artificial Sequence
<220>
   <223> B-KV14 construct of a mouse/human modified antigen binding molecule
<400> 134
   Asp Ile Val Met Thr Gln Thr Pro Leu Ser Leu Pro Val Thr Pro Gly
   1               5                   10                  15
   Glu Pro Ala Ser Ile Ser Cys Arg Ser Ser Lys Ser Leu Leu His Ser
               20                  25                  30
   Asn Gly Ile Thr Tyr Leu Tyr Trp Tyr Leu Gln Lys Pro Gly Gln Ser
           35                  40                  45
   Pro Gln Leu Leu Ile Tyr Gln Met Ser Asn Leu Val Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ala Gln Asn
                   85                  90                  95
   Leu Glu Leu Pro Tyr Thr Phe Gly Gly Gly Thr Lys Val Glu Ala Lys
               100                 105                 110
   Arg Thr Val
           115

## Description

### BACKGROUND OF THE INVENTION

### Field of the Invention

The present invention relates to a modified antigen binding molecule specifically binding to human CD20 comprising
(a) a heavy chain variable region of SEQ ID NO: 125, a heavy chain variable region comprising the replacement of the amino acid residue at Kabat position 11 with a leucine and at Kabat position 12 with lysine in SEQ ID NO: 125, a heavy chain variable region comprising the replacement of the amino acid residue at Kabat position 9 with a glycine in SEQ ID NO: 125, or a heavy chain variable region comprising the replacement of the amino acid residue at Kabat position 10 with a glycine in SEQ ID NO: 125; and
(b) a light chain variable region selected from the group consisting of SEQ ID NO: 48, 130, 131, 132, 133 and 134.

The disclosure generally relates to antigen binding molecules (ABMs). In particular embodiments, the present disclosure relates to recombinant monoclonal antibodies or fragments, including chimeric, primatized or humanized antibodies or fragments, having altered ability to mediate cell signaling activity by a target antigen, and/or altered ability to mediate cross-linking of one or more target antigens. In addition, the present disclosure relates to nucleic acid molecules encoding such ABMs, and vectors and host cells comprising such nucleic acid molecules. The disclosure further relates to methods for producing the ABMs of the invention, and to methods of using these ABMs in treatment of disease. In addition, the present disclosure relates to ABMs with modified glycosylation having improved therapeutic properties, including antibodies with increased Fc receptor binding and increased effector function.

### Background Art

Antibodies, also called immunoglobulins, have a basic structure comprising four polypeptide chains: two identical heavy (H) chains paired with two identical light (L) chains. Each heavy and light chain comprises a variable region (VH and VL, respectively) and a constant region (CH and CL, respectively). The CH region has 3 domains (CH1, CH2, and CH3), while the smaller CL region has only one domain (simply refered to as CL), Bach VH and VL region comprises 3 complementarity determining regions (CDRs) flanked by 4 framework regions in the following order: FR1-CDR1-FR2-CDR2-FR3-CDR3-FR4. The CDRs are the most variable part of the of the V region, and determine the antigen specificity of the antibody. Together, a paired VH and VL form the antigen binding site, and bivalent antibodies have two such antigen binding sites. It should be noted that this basic antibody structure can be modified in various ways (e.g., by generating fragments of the structure) while still retaining or even improving desired functions and/or antigen binding activity.

The interface between the VH and CH1 domains comprises conserved amino acids. This area of contact can be described as a "molecular ball-and-socket joint" This joint determines the "elbow motion" and also the so called "elbow angle" of the VH and VL regions with respect to the CH1 and CL regions, and prevents a rigid contact from forming between the V and C regions (Lesk and Chothia, Nature (1988) 335(8):188-190)).The socket of the ball-and-socket joint is formed by amino acid residues in the VH framework region, specifically those at positions 11, 110, and 112 (according to the numbering system of Kabat et al., (1987) Sequences of Proteins of Immunological Interest, 4th ed. (Public Health Services, NIH, Washington, DC)). (*See* Lesk and Chothia, Nature (1988) 335(8):188-190.) The "ball" of this ball-and-socket joint is found in the CH1 domain, and is formed mainly by the two amino acids at positions 148 and 149 (*See* Landolfi et al., J. Immunol. (2001) 166:1748-1754; Lesk and Chothia, Nature (1988) 335(8):188-190) (wherein the CH1 residues forming the "ball" are numbered 149 and 150, respectively)). Differences in the amino acids at these positions can dictate the elbow angle that is formed between the V andC regions, and therefore the orientation of the VH-VL dimer (*see* Lesk and Chothia, Nature (1988) 335(8):188-190). The amino acid residues that occupy theseVH positions are highly conserved across immunoglobulin sequences (*see e*.*g*., Lesk and Chothia, Nature (1988) 335(8)-188-190). All residues involved in this joint (e.g., positions 11, 110, 112, 148, and 149); are located in the framework regions (e.g., residues 11, 110, and 112) or the constant domain (e.g., 148 and 149 (according to Landolfi et al.; positions 149 and 150 according to Lesk and Chothia), and do not appear to be directly involved in antigen binding. Landolfi et al., J. Immunol. (2001) 166:1748-1754.

In addition to mediating effector functions such as antibody dependent cell-mediated cytotoxicity (ADCC) and complement dependent cytotoxicity (CDC), monoclonal antibodies can modulate cellular functions by inducing or inhibiting cell signaling pathways. For example, monoclonal antibodies have been shown to mediate antigen cross-linking, activate death receptors (e.g., by facilitating oligomerization of receptors or mimicking ligand binding), and blocking of ligand-mediated cell signaling in cell growth differentiation, and/or proliferation pathways (*see*, *e*.*g*., Ludwig et al., Oncogene (2003) 22: 9097-9106).

Apoptosis, or programmed cell death, can be triggered by several different mechanisms. For example, the activation of signaling pathways through cell membrane-bound "death receptors," e.g., members of the tumor necrosis factor receptor (TNFR) superfamily, can lead to induction of apoptosis. Likewise, dimerization or cross-linking of surface antigen, e.g., CD20, can also induce apoptosis (*see*, *e*.*g*., Ludwig et al, Oncogene (2003) 22: 9097-9106).

There remains a need for enhanced therapeutic approaches targeting antigens associated with cell signaling, including but not limited to, the induction of apoptosis, for the treatment of disease in primates, including, but not limited to, humans.

### BRIEF SUMMARY OF THE INVENTION

Recognizing the tremendous therapeutic potential of modified antigen binding molecules (ABMs) that have altered ability to mediate cell signaling activity by a target antigen, and/or altered ability to mediate cross-linking of one or more target antigens, the present inventors developed such ABMs, as well as a method for producing such ABMs. *Inter alia,* this method involves producing recombinant, chimeric antibodies or chimeric fragments thereof. The efficacy of these modified ABMs is further enhanced by engineering the glycosylation profile of the antibody Fc region.

Accordingly, in one aspect, the disclosure is directed to a modified antigen binding molecule comprising a heavy chain or light chain variable region comprising at least one amino acid residue substitution in at least one framework region of said heavy chain or light chain variable region as compared to the heavy chain or light chain variable region of a parent antigen binding molecule, wherein said substitution results in altered cell signaling activity of a target antigen when said modified antigen binding molecule is complexed with said target antigen. In a particular embodiment, the altered cell signaling activity is apoptosis. In one embodiment, the modified antigen binding molecule has an increased ability to induce apoptosis. In another embodiment, the modified antigen binding molecule has a reduced ability to induce apoptosis.

In another aspect, the disclosure is directed to a modified antigen binding molecule comprising a heavy chain or light chain variable region comprising at least one amino acid residue substitution in at least one framework region of said heavy chain or light chain variable region as compared to the heavy chain or light chain variable region of a parent antigen binding molecule, wherein said modified antigen binding molecule has altered ability to mediate cross-linking of one or more target antigens as a result of said substitution.

In one embodiment, the modified antigen binding molecule of the present disclosure comprises a substitution in FR1 of the heavy chain variable region. In another embodiment, the substitution comprises a replacement selected from the group consisting of at least 2, at least 3, or at least 4 amino acids.

In one embodiment, the substitution comprises a replacement of the entire FR1 of the heavy chain variable region. In a further embodiment, the entire FR1 is replaced by a germline VH FR1. In particular embodiments, the germline VH FR1 comprises an amino acid sequence at Kabat positions 8 to 13 selected from the group consisting of SEQ ID NO:63, SEQ ID NO:64, SEQ ID NO: 65, SEQ ID NO:66, SEQ ID NO:67, SBQ ID NO:68, SBQ ID NO:69, SEQ ID NO:70, SEQ ID NO:71, SEQ ID NO:72, SEQ ID NO:73, SEQ ID NO:74, SEQ ID NO:75, SEQ ID NO:76, SEQ ID NO:77, SEQ ID NO:78, SEQ ID NO:79, SEQ ID NO:101, SEQ ID NO:102, SEQ ID NO:103, SEQ ID NO:104, and SEQ ID NO:105.

In one embodiment, the modified ABM comprises a substitution in FR1 of the heavy chain variable region which comprises a replacement of an amino acid residue at one or more of Kabat positions 8, 9, 10, 11, 12, or 13.

In a particular embodiment, the substitution in FR1 of the heavy chain variable region comprises a replacement of the amino acid residue at Kabat position 8. In a more specific embodiment, the substitution in FR1 of the heavy chain variable region comprises a replacement of the amino acid residue at Kabat position 8 with an amino acid residue selected from the group consisting of arginine, and glycine.

In another particular embodiment, the substitution in FR1 of the heavy chain variable region comprises a replacement of the amino acid residue at Kabat position 9. In a more specific embodiment, the substitution in FR1 of the heavy chain variable region comprises a replacement of the amino acid residue at Kabat position 9 with an amino acid residue selected from the group consisting of alanine, proline, glycine, serine, and histidine.

In one particular embodiment, the substitution in FR1 of the heavy chain variable region comprises a replacement of the amino acid residue at Kabat position 10. In a more specific embodiment, the substitution comprises a replacement of the amino acid residue at Kabat position 10 with an amino acid residue selected from the group consisting of glutamate, threonine, glycine, alanine, and valine.

In another particular embodiment, the substitution in FR1 of the heavy chain variable region comprises a replacement of the amino acid residue at Kabat position 11. In specific embodiment, the substitution comprises a replacement of the amino acid residue at Kabat position 11 with any amino acid but leucine. In another specific embodiment, the substitution comprises a replacement of the amino acid residue at Kabat position 11 with a nonpolar amino acid. In another specific embodiment, the substitution comprises a replacement of the amino acid residue at Kabat position 11 with an amino acid residue selected from the group consisting of valine, leucine, isoleucine, serine, and phenylalanine. In a particular embodiment, the substitution comprises a replacement of the amino acid residue at Kabat position 11 with a leucine.

In another particular embodiment, the substitution in FR1 of the heavy chain variable region comprises a replacement of the amino acid residue at Kabat position 12. In a specific embodiment the substitution comprises a replacement of the amino acid residue at Kabat position 12 with an amino acid residue selected from the group consisting of lysine, valine, leucine, and isoleucine.

In another particular embodiment, the substitution in FR1 of the heavy chain variable region comprises a replacement of the amino acid residues at Kabat positions 11 and 12. In a specific embodiment, the substitution comprises a replacement of the amino acid residue at Kabat position 11 with a valine and at Kabat position 12 with a lysine; a replacement of the amino acid residue at Kabat position 11 with a leucine and at Kabat position 12 with a valine; a replacement of the amino acid residue at Kabat position 11 with a valine and at Kabat position 12 with an isoleucine; or a replacement of the amino acid residue at Kabat position 11 with a valine and at Kabat position 12 with a valine.

In another particular embodiment, the substitution in FR1 of the heavy chain variable region comprises a replacement of the amino acid residue at Kabat position 13. In a specific embodiment, the substitution comprises a replacement of the amino acid residue at Kabat position 13 with an amino acid residue selected from the group consisting of lysine, arginine, glutamine and glutamate.

In another aspect of the present disclosure, the substitution in the ABM comprises replacement of at least one amino acid residue in FR4 of the heavy chain variable region. In a particular embodiment, the substitution in FR4 of the heavy chain variable region comprises a replacement of an amino acid residue at one or more of Kabat positions 110 or 112.

In a particular embodiment, the substitution comprises a replacement of the amino acid residue at Kabat position 110 with an amino acid selected from the group consisting of leucine, isolencine, threonine or serine. In a more specific embodiment, the substitution comprises a replacement of the amino acid residue at Kabat position 110 with an isoleucine.

In another embodiment, the substitution comprises a replacement of the amino acid residue at Kabat position 112 with an amino acid selected from the group consisting of valine, leucine, isoleucine, or threonine. In a more specific embodiment, the substitution comprises a replacement of the amino acid residue at Kabat position 112 with an isoleucine.

In one aspect, the present disclosure is further directed to a modified antigen binding molecule comprising a CH1 domain comprising at least one amino acid residue substitution as compared to the CH1 domain of a parent polypeptide, wherein the substitution results in altered cell signaling activity of a target antigen when the modified antigen binding molecule is complexed with the target antigen.

In another aspect, the present disclosure is further directed to a modified antigen binding molecule comprising a CH1 domain comprising at least one amino acid residue substitution as compared to the CH1 domain of a parent polypeptide, wherein the antigen binding molecule has altered ability to mediate cross-linking of one or more target antigens as a result of the substitution.

In a particular embodiment, the substitution in CH1 comprises a replacement of an amino acid residue at one or more of positions 148, 149 or 150. In a more specific embodiment, the substitution comprises a replacement of the amino acid residue position 149 with a leucine. In another embodiment, the substitution comprises a replacement of the entire CH1 domain. In another embodiment, the substitution comprises a replacement of an IgG CH1 domain with an IgM CH1 domain.

In another aspect, the disclosure is directed to a modified antigen binding molecule comprising at least one amino acid substitution, wherein said substitution comprises a replacement of an amino acid residue in the light chain in the region of the interface between the variable and constant regions, wherein the substitution results in altered cell signaling activity of a target antigen binding molecule when said modified antigen binding molecule is complexed with said target antigen.

In another aspect, the disclosure is directed to a modified antigen binding molecule comprising at least one amino acid substitution, wherein said substitution comprises a replacement of an amino acid residue in the light chain in the region of the interface between the variable and constant regions, wherein -said modified antigen binding molecule has altered ability to mediate cross-linking of one or more target antigens as a result of said substitution.

In a particular embodiment, the substitution in the light chain variable region of the ABM comprises a replacement of an amino acid at one or more of Kabat positions 10, 12, 39, 40, 41, 80, 81, 83, 84, 103, 105, 106, and 108. In a particular embodiment, the substitution in the light chain variable region of the ABM comprises a replacement of an amino acid residue at one or more of Kabat positions 40, 80, 83, 105, or 106.

In another particular embodiment, the substitution in the light chain of the ABM comprises a replacement of an amino acid residue at one or more of Kabat positions 40, 80, 83, 105, or 106 with a non-polar amino acid.

In another particular embodiment, the substitution in the light chain of the ABM comprises a replacement of an amino acid residue at Kabat position 40 with an alanine.

In another particular embodiment, the substitution in the light chain of the ABM comprises a replacement of an amino acid residue at Kabat position 80 with a proline.

In another particular embodiment, the substitution in the light chain of the ABM comprises a replacement of an amino acid residue at Kabat position 83 with a phenylalanine.

In another particular embodiment, the substitution in the light chain of the ABM comprises a replacement of an amino acid residue at Kabat position 105 with an alanine.

In another particular embodiment, the substitution in the light chain of the ABM comprises a replacement of an amino acid residue at Kabat position 106 with an alanine. In a more particular embodiment, the substitution in the light chain of the ABM comprises a replacement of an amino acid residue at Kabat position 106, wherein the antigen binding molecule is reduced in its ability to induce apoptosis.

In some embodiments, the substitutions of the present disclosure comprise a combination of any of the amino acid residue replacements in the heavy and/or light chain variable and/or constant regions as described herein.

In one aspect, the amino acid substitution(s) in the modified ABMs of the present disclosure result in altered cell signaling activity of a target antigen when the modified ABM is complexed with the target antigen.

In one aspect of the disclosure, the altered cell signaling activity is increased agonist activity. In one embodiment, the increased agonist activity is selected from the group consisting induction of apoptosis and induction of cell differentiation.

In another aspect of the disclosure, the altered cell signaling activity is increased antagonist activity. In one embodiment, the antagonist activity is blockade of a cell signaling pathway selected from the group consisting of cell survival, cell growth, cell proliferation, and angiogenesis.

In a further aspect, the modified antigen binding molecule of the present disclosure, binds specifically to human CD20. In another aspect, the modified antigen binding molecule binds specifically to a member of the human TNF receptor superfamily. In a particular embodiment, the TNF receptor superfamily is selected from the group consisting of TNFR1, CD95, TRAILR1, TRAILR2, EDAR, and p75NGFR.

In another aspect of the disclosure, the modified antigen binding molecule binds specifically to a receptor tyrosine kinase. In a particular embodiment, the receptor tyrosine kinase is selected from the group consisting of HER1 (EGFR1), HER2/neu, HER3, HER4, IGF-1R, FGFR, PDGFR, VBGFR1, VEGFR2, and VEGFR3. In a more specific embodiment, the receptor tyrosine kinase is HER1 (EGFR1).

In another aspect, the present disclosure is further directed to a modified ABM that can be selected from, but is not limited to, the group consisting of a whole antibody, an Fab fragment or a fusion protein thereof, an F(ab')2 fragment or a fusion protein thereof, a minibody, a diabody, a triabody, and a tetrabody. In a particular embodiment, the modified ABM is chimeric or fully human. In a more particular embodiment, the chimeric modified ABM is humanized. In another particular embodiment, the modified ABM is multispecific. In a more particular embodiment, the modified ABM is bispecific.

In another aspect, the parent antigen binding molecule according to the present disclosure comprises a heavy chain variable region selected from the group consisting of SEQ ID NO:55, SEQ ID NO:56, SBQ ID NO:57, SEQ ID NO:58, SEQ ID NO:59, SEQ ID NO:60, SEQ ID NO:61, and SEQ ID NO:62.

In another aspect, the parent antigen binding molecule according to the present disclosure comprises a light chain selected from the group consisting of SEQ ID NO: 48, SEQ ID NO:130, SEQ ID NO:131, SEQ ID NO:132, SEQ ID NO:133, and SEQ ID NO:134.

In a further aspect, the present disclosure is also directed to a modified ABM further comprising a human Fc region. According to one embodiment, the Fc region of the modified ABM has been modified to have altered oligosaccharides. In a more specific embodiment, the Fc region has been modified to have a decreased proportion of fucose residues compared to a non-modified Fc region. In another specific embodiment, the Fc region has an increased proportion of bisected oligosaccharides compared to a non-modified Fc region. In another specific embodiment, the modified oligosaccharides are bisected complex. In another specific embodiment, the modified oligosaccharides have an increased proportion of bisected, nonfucosylated oligosaccharides in the Fc region compared to a non-modified Fc region. In another specific embodiment, the Fc region has an increased proportion of GlcNAc residues to fucose residues in the modified Fc region compared to a non-modified Fc region. In another specific embodiment, the bisected, nonfucosylated oligosaccharides are hybrid. In another specific embodiment, the bisected, nonfucosylated oligosaccharides are complex.

According to another aspect, the target antigen according to the present disclosure is a cell surface receptor selected from the group consisting of a membrane transport receptor, a G-protein-linked receptor, and an enzyme-linked receptor, In a particular embodiment, the membrane transport receptor is a channel-linked receptor. In another particular embodiment, the enzyme-linked receptor is selected from the group consisting of receptor guanylyl cyclases, receptor tyrosine kinases, tyrosine-kinase associated receptors, receptor tyrosine phosphatases, and receptor serine/threonine kinases.

In another aspect, the disclosure is related to a pharmaceutical composition comprising the modified ABM of the disclosure. It is contemplated that the pharmaceutical composition may further comprise a pharmaceutically acceptable carrier, an adjuvant or a combination thereof.

The present disclosure is also directed to a method of treating a disease treatable by altered cell signaling activity in a patient, the method comprising administering to the patient a therapeutically effective amount of a pharmaceutical composition comprising a modified ABM according to the disclosure and a pharmaceutically acceptable carrier.

In another aspect, the disclosure is directed to an isolated polynucleotide encoding a polypeptide comprising a heavy chain or light chain variable region, wherein the heavy chain or light chain variable region comprises at least one amino acid residue substitution in at least one framework region as compared to a parent heavy chain or light chain variable region, and wherein the substitution results in altered cell signaling activity of a target antigen when the polypeptide is complexed with the target antigen.

In a further aspect, the disclosure is directed to an isolated polynucleotide encoding a polypeptide comprising a heavy chain or light chain variable region, wherein the heavy chain or light chain variable region comprises at least one amino acid residue substitution in at least one framework region as compared to a parent heavy chain or light chain variable region, and wherein the polypeptide has altered ability to mediate cross-linking of one or more target antigens as a result of the substitution.

In one embodiment, a polynucleotide according to the present disclosure encodes a polypeptide, wherein the polypeptide comprises an antibody heavy chain or light chain. In another embodiment, a polynucleotide according to the present disclosure encodes a polypeptide, wherein the polypeptide comprises a fusion protein. The present disclosure is also directed to polypeptides encoded by the polynucleotides of the present disclosure.

The present disclosure is also directed to a vector comprising a polynucleotide according to the disclosure, and a host cell comprising the vector.

The disclosure is further directed to a polynucleotide encoding a polypeptide comprising a heavy chain or light chain variable region which comprises at least one amino acid residue substitution in at least one framework: region of the heavy chain or light chain variable region as compared to the heavy chain or light chain variable region of a parent antigen binding molecule, wherein the polypeptide is a modified ABM according to the disclosure.

The present disclosure is also directed to a host cell engineered to express at least one nucleic acid encoding a polypeptide having β(1,4)-N-acetylglucosaminyltransferase III activity in an amount sufficient to modify the oligosaccharides in the Fc region of a polypeptide produced by the host cell, wherein the polyp eptide is a modified ABM as described herein. In one embodiment, the polypeptide having β(1,4)-N-acetylglucosaminyltransferase III activity is a fusion polypeptide. In a particular embodiment, the fusion polypeptide comprises the catalytic domain of β(1,4)-N-acetylglucosaminyltransferase III. In another embodiment the fusion polypeptide further comprises the Golgi localization domain of a heterologous Golgi resident polypeptide. The Golgi localization domain can be selected from, but is not limited to, the group consisting of the localization domain of mannosidase II, the localization domain of β(1,2)-N-acetylglucosaminyltransferase I, the localization domain of β(1,2)-N-acetylglucosaminyltransferase II, the localization domain of mannosidase I, and the localization domain of α1-6 core fucosyltransferase.

In another embodiment, the modified ABM comprises a region equivalent to the Fc region of a human IgG.

In a further embodiment, the modified ABM produced by the host cell of the present disclosure exhibits increased Fc receptor binding affinity and/or increased effector function as a result of the oligosaccharide modification. According to the present disclosure, the increased effector function is selected from the group consisting of increased Fc-mediated cellular cytotoxicity, increased binding to NK cells, increased binding to macrophages, increased binding to polymorphonuclear cells, increased binding to monocytes, increased direct signaling inducing apoptosis, increased dendritic cell maturation, and increased T cell priming, In one embodiment, the Fc receptor is Fcγ activating receptor. In another embodiment, the Fc receptor is FcγRIIIA receptor.

The host cell of the present disclosure may be selected from the group that includes, but is not limited to, a CHO cell, an HEK293-EBNA cell, a BHK cell, a NSO cell, a SP2/0 cell, a YO myeloma cell, a P3Xb3 mouse myeloma cell, a PER cell, a PER C6 cell or a hybridoma cell.

In another aspect, the present disclosure is directed to a method for producing a modified ABM comprising a heavy chain or light chain variable region comprising at least one amino acid residue substitution in at least one framework region of the heavy chain or light chain variable region as compared to the heavy chain or light chain variable region of a parent ABM, wherein the substitution results in altered cell signaling activity of a target antigen when the modified ABM is complexed with the target antigen, the method comprising: (i) culturing the host cell of the present disclosure under conditions permitting the expression of the polynucleotide; and (ii) recovering the modified ABM from the culture medium.

In a further aspect, the disclosure is directed to a method for producing a modified ABM comprising a heavy chain or light chain variable region comprising at least one amino acid residue substitution in at least one framework region of the heavy chain or light chain variable region as compared to the heavy chain or light chain variable region of a parent antigen binding molecule, wherein the modified antigen binding molecule has altered ability to mediate cross-linking as a result of the substitution, the method comprising: (i) culturing the host cell of the present disclosure under conditions permitting the expression of the polynucleotide; and (ii) recovering the modified ABM from the culture medium.

In a farther aspect, the disclosure is directed to a method of altering the ability of an ABM to facilitate formation of complexes comprising the target antigen of the ABM, the method comprising: replacing at least one amino acid residue in at least one heavy chain or light chain variable region framework region of a parent ABM. In one embodiment, the ABM increases induction of apoptosis in a cell expressing the target antigen. In another embodiment, the ABM increases induction of cell differentiation in a cell expressing the target antigen.

The present disclosure is also directed to a method of inducing apoptosis in a cell, the method comprising contacting the cell with a modified ABM comprising a heavy chain or light chain variable region which comprises at least one amino acid residue substitution in at least one framework region of said heavy chain or light chain variable region as compared to the heavy chain or light chain variable region of a parent ABM, wherein the modified ABM has increased ability to induce apoptosis compared to the parent polypeptide. In a particular embodiment, the cell is a tumor cell. In one embodiment, the contacting occurs in vivo.

In another aspect, the present disclosure is also directed method of treating a disease or disorder that is treatable by altered cell signaling activity of a target antigen, the method comprising administering to a subject in need thereof a therapeutically effective amount of a modified ABM, wherein the modified ABM comprises a heavy chain or light chain variable region comprising at least one amino acid substitution in at least one framework region of the heavy chain or light chain variable region compared to the heavy chain or light chain variable region of a parent ABM, and wherein the substitution results in altered cell signaling activity of a target antigen when the modified ABM is complexed with the target antigen.

In a further aspect, the disclosure is directed to a method of treating a disease or disorder that is treatable by altered ability to mediate cross linking of one or more target antigens, the method comprising administering to a subject in need thereof a therapeutically effective amount of a modified ABM, wherein the modified ABM comprises a heavy chain or light chain variable region comprising at least one amino acid substitution in at least one framework region of the heavy chain or light chain variable region compared to the heavy chain or light chain variable region of a parent ABM, and wherein the modified ABM has altered ability to mediate cross-linking of one or more target antigens as a result of the substitution.

In a particular embodiment, the modified ABM administered according to the present disclosure comprises a heavy chain variable region selected from the group consisting of SEQ ID NO:4, SEQ ID NO:36, and SEQ ID NO:38.

In one aspect, the disease or disorder to be treated with a modified ABM of the present disclosure is a cell proliferation disorder. In one embodiment, cell proliferation disorder is cancer. In another aspect, the disease or disorder to be treated with a modified ABM of the present disclosure is a B cell disorder. In a specific embodiment, the B cell disorder is a B cell lymphoma.

The present disclosure is also directed to use of a modified ABM according to the present disclosure for the manufacture of a medicament for the treatment or prophylaxis of cancer.

In a particular embodiment, the present disclosure is also directed to use of a modified ABM according to the present disclosure for the manufacture of a medicament for the treatment or prophylaxis of cancer, wherein said cancer is selected from the group consisting of B-cell lymphoma, breast cancer, bladder cancer, head and neck cancer, skin cancer, pancreatic cancer, lung cancer, ovarian cancer, colon cancer, prostate cancer, kidney cancer, and brain cancer.

In another particular embodiment, the present disclosure is also directed to use of a modified ABM according to the present disclosure for the manufacture of a medicament for the treatment or prophylaxis of cancer, wherein said antigen binding molecule is used in a therapeutically effective amount from about 1.0 mg/kg to about 15 mg/kg. In a further embodiment, the therapeutically effective amount is from about 1.5 mg/kg to about 12 mg/kg. In a further embodiment, the therapeutically effective amount is from about 1.5 mg/kg to about 4.5 mg/kg. In a further embodiment, the therapeutically effective amount is from about 4.5 mg/kg to about 12 mg/kg. In a further embodiment, the therapeutically effective amount is about 1.5 mg/kg. In a further embodiment, the therapeutically effective amount is about 4.5 mg/kg. In a further embodiment, the therapeutically effective amount is about 12 mg/kg.

The present disclosure is also directed to a method for the treatment or prophylaxis of cancer comprising administering a therapeutically effective amount of a. pharmaceutical composition of the disclosure to a. patient in need thereof. In a particular embodiment, the cancer is selected from the group consisting of B-cell lymphoma, breast cancer, bladder cancer, head and neck cancer, skin cancer, pancreatic cancer, lung cancer, ovarian cancer, colon cancer, prostate cancer, kidney cancer, and brain cancer.

The present disclosure is also directed to a method for the treatment or prophylaxis of a precancerous condition or lesion comprising administering a therapeutically effective amount of the pharmaceutical composition of claim 85 or 158 to a patient in need thereof. In a particular embodiment, the precancerous condition or lesion is selected from the group consisting of oral leukoplakia, actinic keratosis (solar keratosis), precancerous polyps of the colon or rectum, gastric epithelial dysplasia, adenomatous dysplasia, hereditary nonpolyposis colon cancer, syndrome (HNPCC), Barrett's esophagus, bladder dysplasia, and precancerous cervical conditions.

The present disclosure is also directed to a modified antigen binding molecule of the present disclosure for use in the treatment or prophylaxis of cancer. In a particular embodiment, the cancer is selected from the group consisting of B-cell lymphoma, breast cancer, bladder cancer, head and neck cancer, skin cancer, pancreatic cancer, lung cancer, ovarian cancer, colon cancer, prostate cancer, kidney cancer, and brain cancer.

The present disclosure is also directed to a modified antigen binding molecule of the present disclosure for use in the treatment or prophylaxis of a precancerous condition or lesion. In a particular embodiment, the precancerous condition or lesion is selected from the group consisting of oral leukoplakia, actinic keratosis (solar keratosis), precancerous polyps of the colon or rectum, gastric epithelial dysplasia, adenomatous dysplasia, hereditary nonpolyposis colon cancer syndrome (HNPCC), Barrett's esophagus, bladder dysplasia, and precancerous cervical conditions.

The present disclosure is also directed to a modified antigen binding molecule according to the present disclosure for use in therapy of a disorder that is related to altered cell signaling activity and/or altered cross-linking of one or more target antigens.

### BRIEF DESCRIPTION OF THE FIGURES

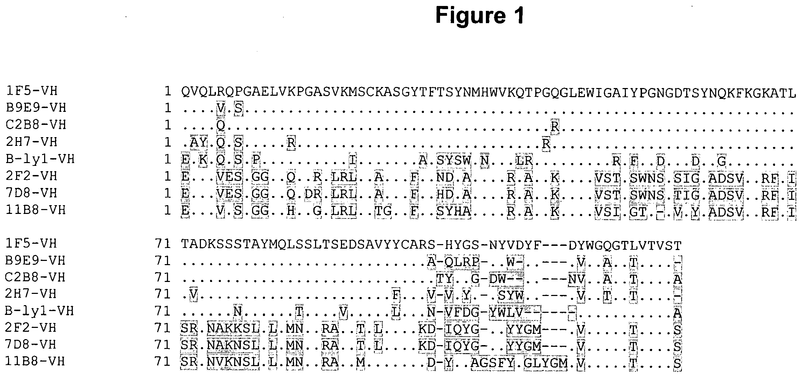

FIG 1. Amino acid sequence alignment of various anti-CD20 antibody heavy chain variable region constructs. The amino acid sequence of the variable chain heavy region of monoclonal antibody 1F5 is used as the reference sequence. Differences in amino acids from 1F5 are shaded.

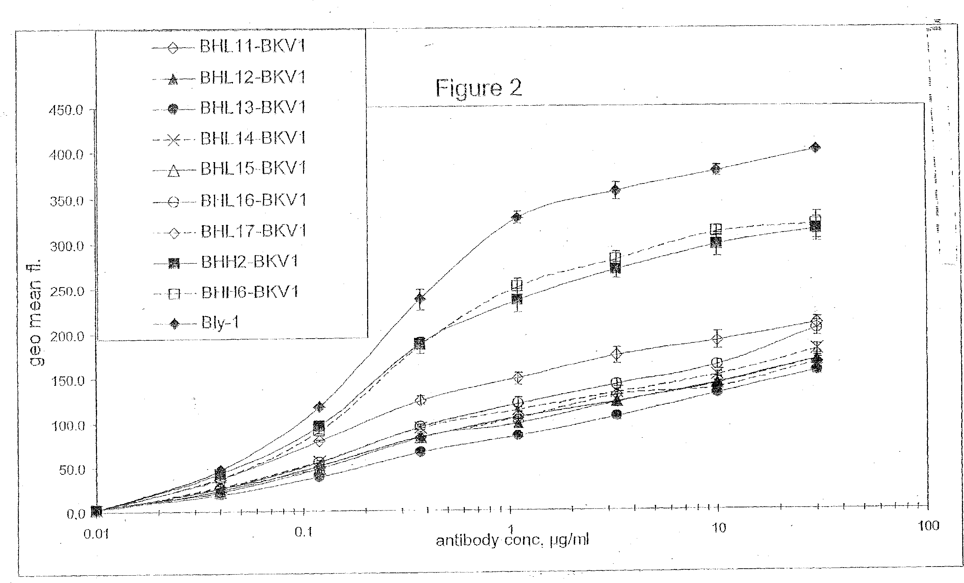

FIG 2. Binding of different humanized anti-CD20 antibodies to Raji B-cells. The parental (chimeric) B-ly1 is compared to those two humanized heavy chain variants that were identified to induce strong apoptosis (BHH2 and BHH6), as well as those derivatives, of the (humanized, nonapoptotic) B-HL8 variant that were hypothesized to restore this effect (B-HL11 to 17). All humanized heavy chain variants were paired with the same BKV1 humanized light chain variant.

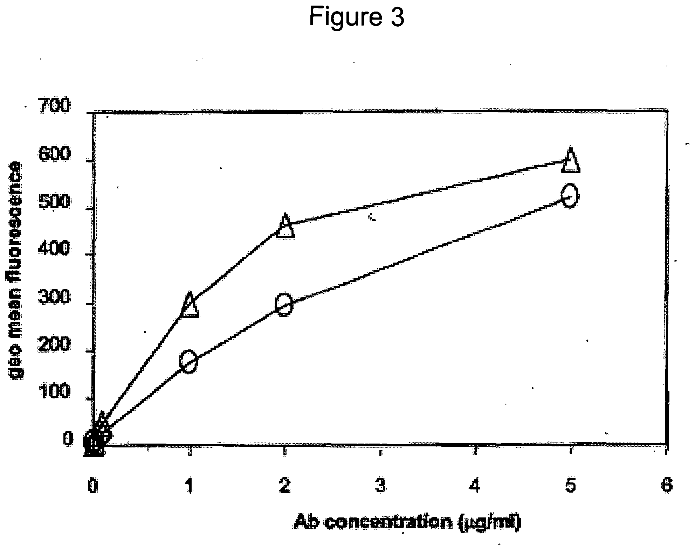

FIG 3. Binding of rituximab (○) and chB-ly1 (Δ) to CD20 on Raji B-lymphoma cells.

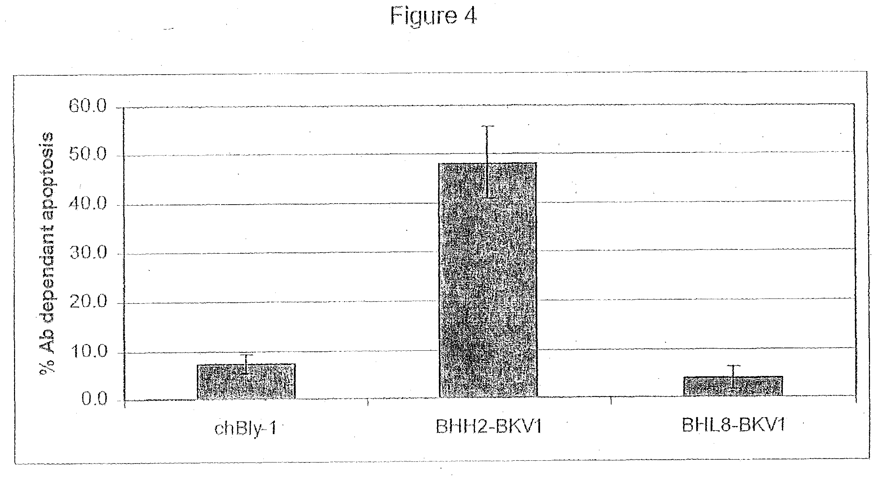

FIG 4. Comparison of antibody-dependent apoptosis by three anti-CD20 antibodies. chB-ly1wt represents a chimeric B-ly1 antibody construct having a murine variable region and a human constant region. BHH2-BKV1 represents a humanized variant comprising murine B-ly1 CDRs and human framework regions derived from VH1 class human germline V genes for the heavy chain and paired with the BKV1 humanized B-ly1 light chain. BHL8-BKV1wt represents a humanized variant comprising murine B-ly1 CDRs and human framework regions derived from two different human germline V genes and paired with the BKV1 humanized B-ly1 light chain.

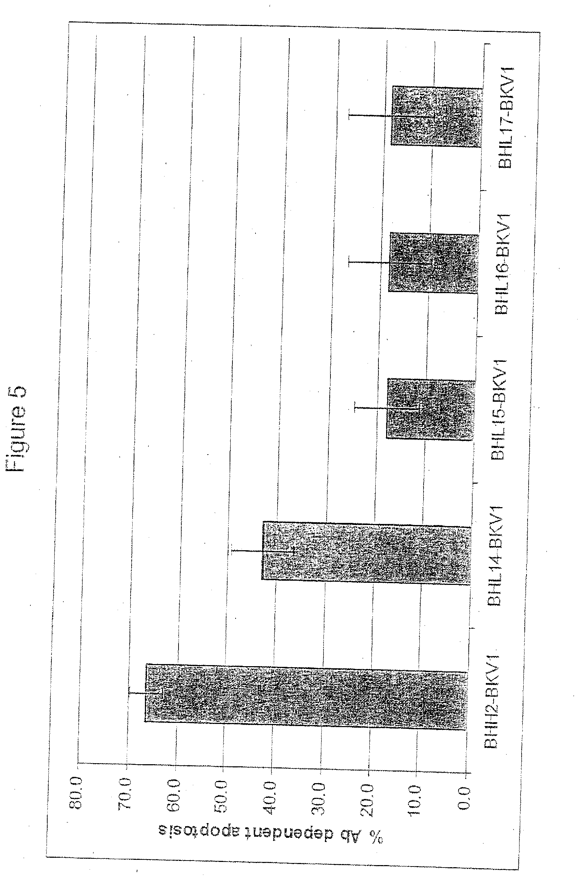

FIG 5. Comparison of antibody-dependent apoptosis by five humanized variants of the B-ly1 anti-CD20 antibody. BHH2-BKV1 represents a humanized variant comprising murine B-ly1 CDRs and human framework regions derived from VH1 class for the heavy chain (BHH2) and paired with the BKV1 humanized B-ly1 light chain. BHL8-BKV1wt represents a humanized variant comprising murine B-ly1 CDRs and human framework regions derived from two different human germline V genes and paired with the BKV1 humanized B-ly1 light chain. BHL14-BKV1 represents a derivative of BHL8, with a valine to lysine substitution at Kabat position 12 and a valine to methionine substitution at Kabat position 48 of the heavy chain variable region, and paired with the BKV1 light chain construct. BHL15-BKV1 W1 is also derived from BHL8, with a glycine to serine substitution at Kabat position 16, and a valine to methionine substitution at Kabat position 48 of the heavy chain variable region and paired with the BKV1 light chain construct. BHL16-BKV1 W1 is derived from BHL8, with a leucine to valine substitution at Kabat position 20, and a valine to methionine substitution at Kabat position 48 of the heavy chain variable region and paired with'the BKV1 light chain construct. BHL17-BKV1 W1 is derived from BHL8, with ε valine to methionine substitution at Kabat position 48 of the heavy chain variable region and paired with the BKV1 light chain construct

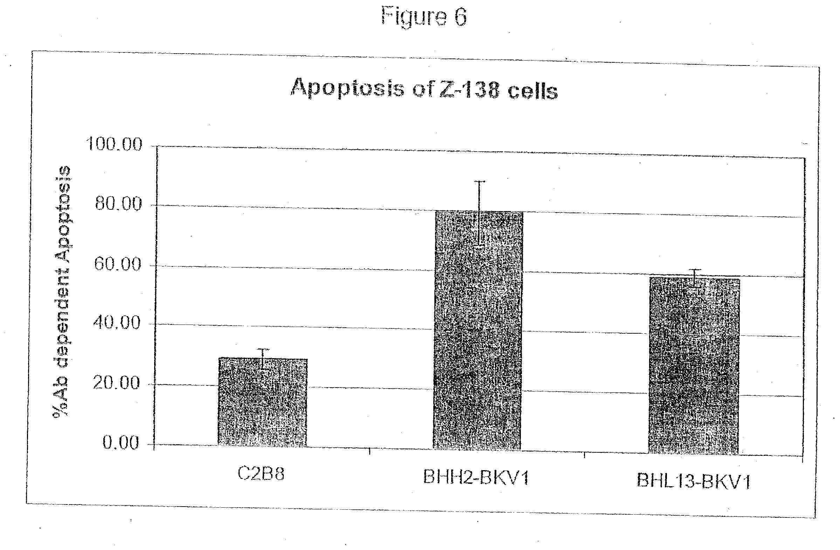

FIG 6. Comparison of antibody-dependent apoptosis in Z-138 cells by C2B8 anti-CD20 monoclonal antibody and two humanized variants of B-lyl antibody, BHH2-BKV1 and BHL13-BKV1. BHH2-BKV1 represents a humanized variant comprising murine B-ly1 CDRs and human framework regions derived from VH1 class human germline V genes for the heavy chain and paired with the BKV1 humanized B-ly1 light chain. BHL13-BKV1 is derived from BHL8 (see Figure 5, above), with a leucine to valine substitution at Kabat position 11 and a valine to methionine substitution at Kabat position 48 in the heavy chain variable region, and paired with a BKV1 light chain.

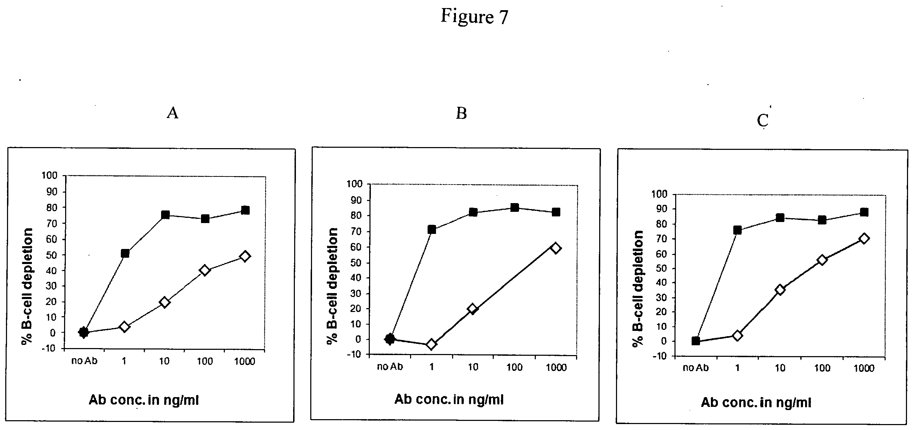

FIG 7. B-Cell depletion by rituximab (◊) and chB-ly1 (■) in whole blood of the three different classes of FcγRIIIa-158V/F genotype: (A) whole blood from a F/F donor, homozygous for the lower affinity receptor, (B) whole blood from a F/V donor, heterozygous for the affinity receptor; and (C) whole blood from a V/V donor, homozygous for the higher affinity receptor.

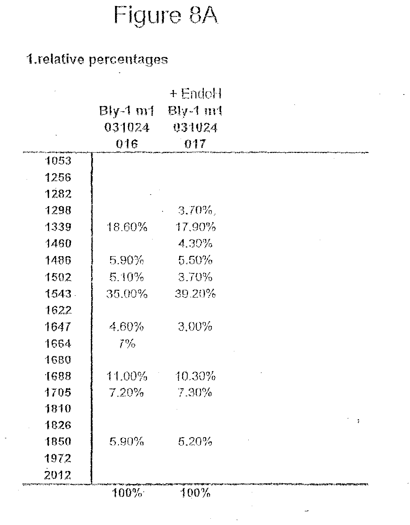

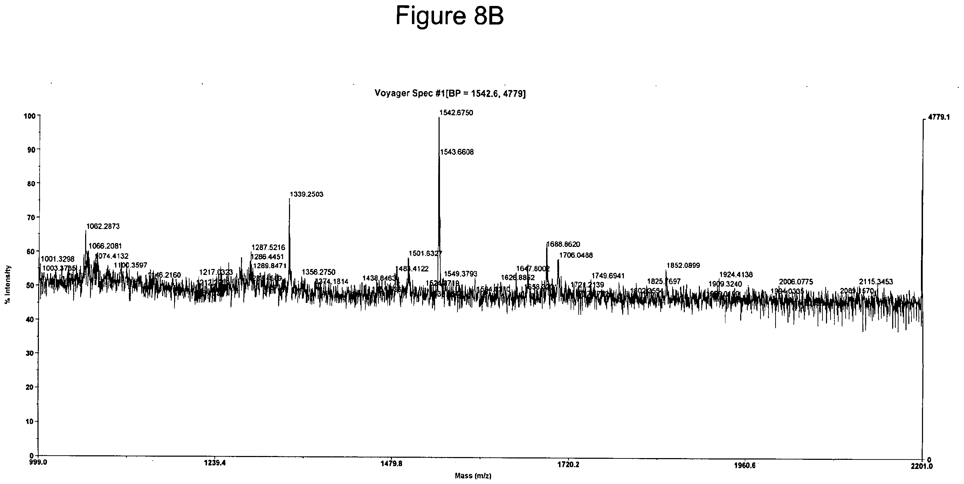

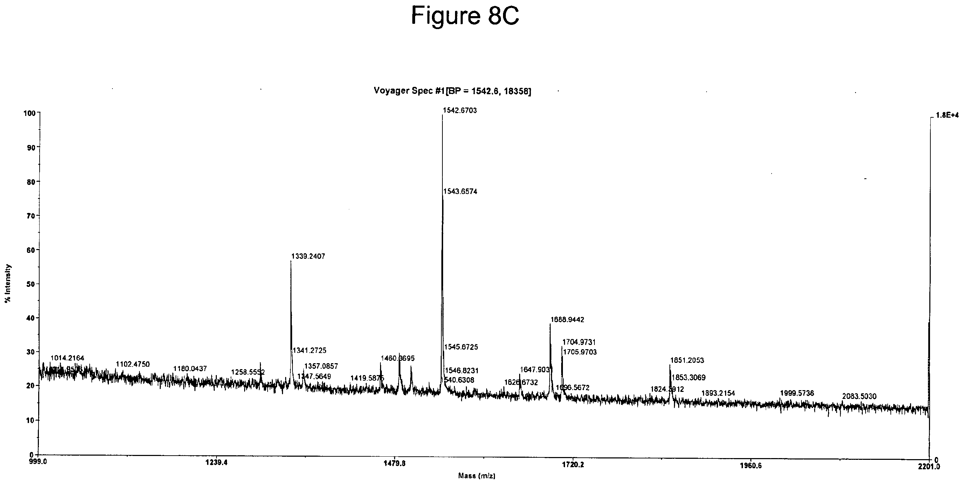

FIG 8. MALDI-TOF profile of a glycoengineered, chimeric B-ly1 antibody. (A) Table detailing the percentages of specific peaks; (B) Spectrum for glycoengineered chimeric B-ly1; (C) Spectrum for glycoengineered chimeric BLy1 treated with Endo-H.

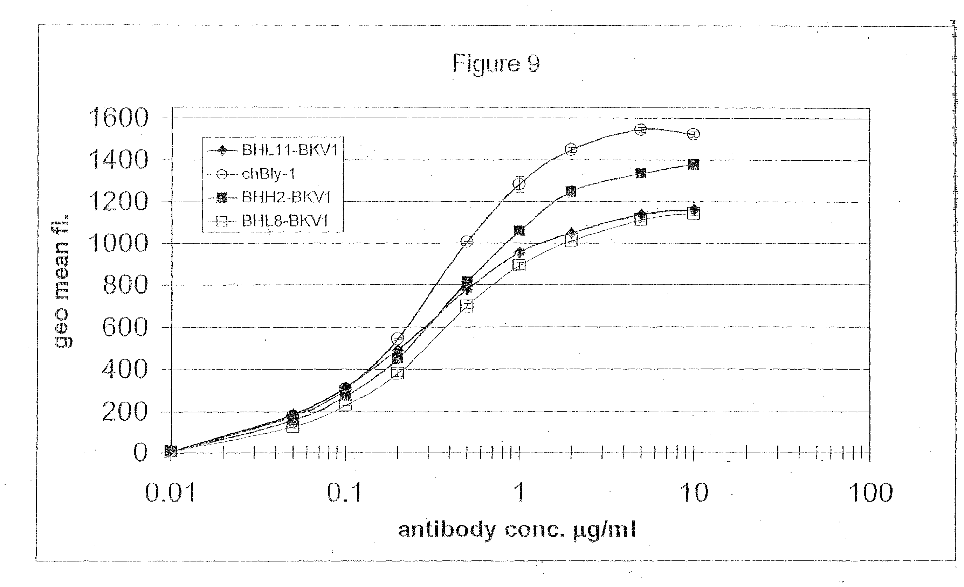

FIG 9. Binding of different humanized anti-CD20 antibodies to Raji B-cells. The differences between the B-HH2 construct and the B-HL8 and B-HL11 constructs are located in the framework 1 and 2 regions with all three CDRs being identical. B-HL8 and B-HL11 have their FR1 andFR2 sequences derived from the human VH3 class, whereas the complete B-HH2 framework is human VH1 derived. B-HL11 is a derivative of B-HL8 with the single mutation Glu1Gln, with Gln being the amino acid residue in the B-HH2 construct. This means that the Glu1Gln exchange does not alter binding affinity or intensity. The other differences between B-HH2 and B-HL8 are 14 FR residues, from which one or more will influence the antigen binding behavior of this antibody.

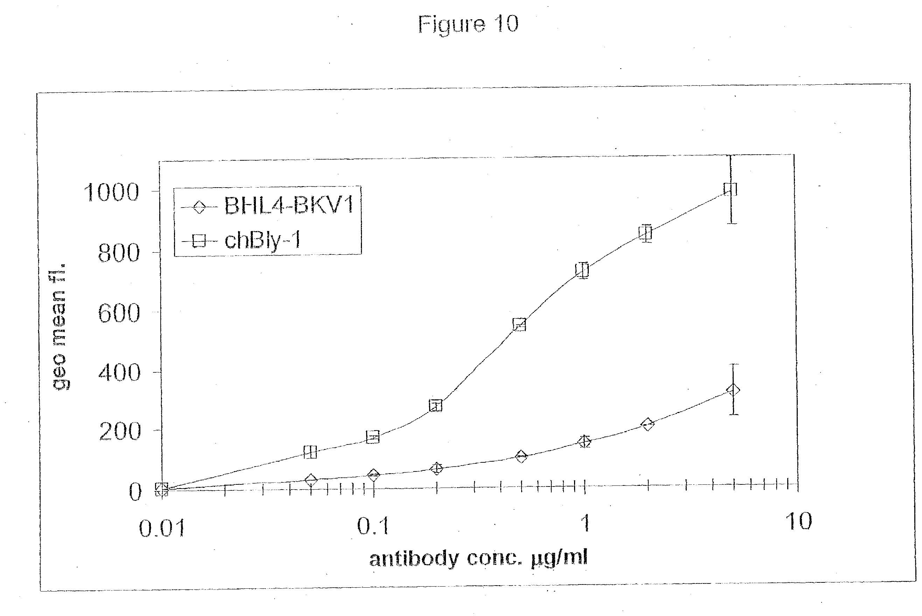

FIG 10. Binding of the humanized anti-CD20 antibody BHL4-BKV1 on Raji target cells. The B-HL4 construct is derived from the B-HH2 antibody by replacing the FR1 of the B-HH2 with that of the human germ line sequence IGHV1-45 (Acc No X92209). This construct shows greatly diminished antigen binding capacity, despite having different amino acids at only four positions within FR1. These residues are located at positions 2, 14, 28 and 30 according to Kabat numbering. Of these, positions 28, and 30 appear to be influential positions, since they are part of the Chothia definition of CDR1.

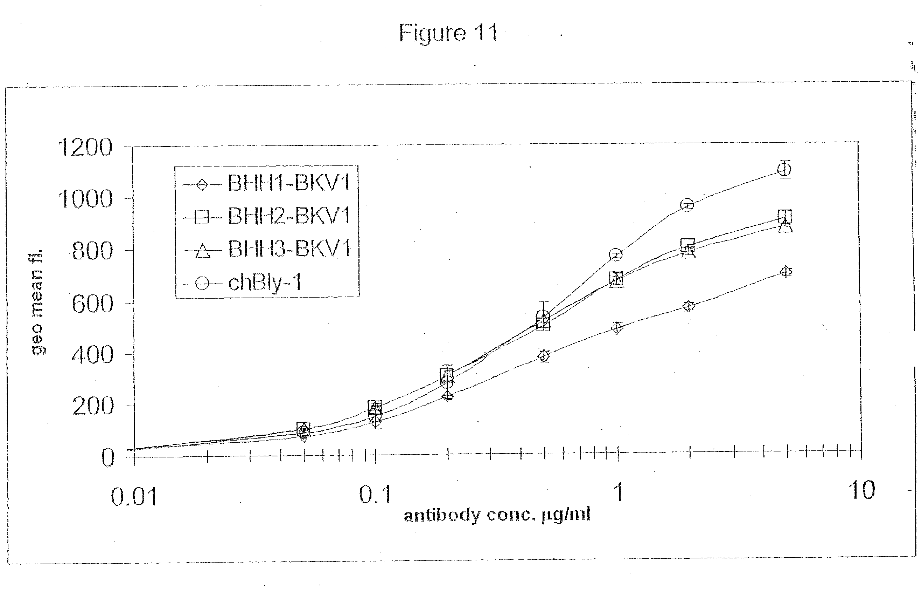

FIG 11. Comparison of the binding behavior between B-HH1, B-HH2, B-HH3 (all paired with the BKV1 humanized B-ly1 light chain), and the parental antibody B-ly1. The data show that all Abs show a similar EC50 value, but the B-HH1 construct binds with a lower intensity/stoichiometry than the the variants B-HH2 and B-HH3. B-HH1 can be distinguished from B-HH2 and B-HH3 by its partially human CDR1 and CDR2 regions (Kabat definition), as well as the Ala/Thr polymorphism at position 28 (Kabat numbering). This indicates that either position 28, the complete CDR1, and/or the complete CDR2 is important for antibody/antigen interaction.

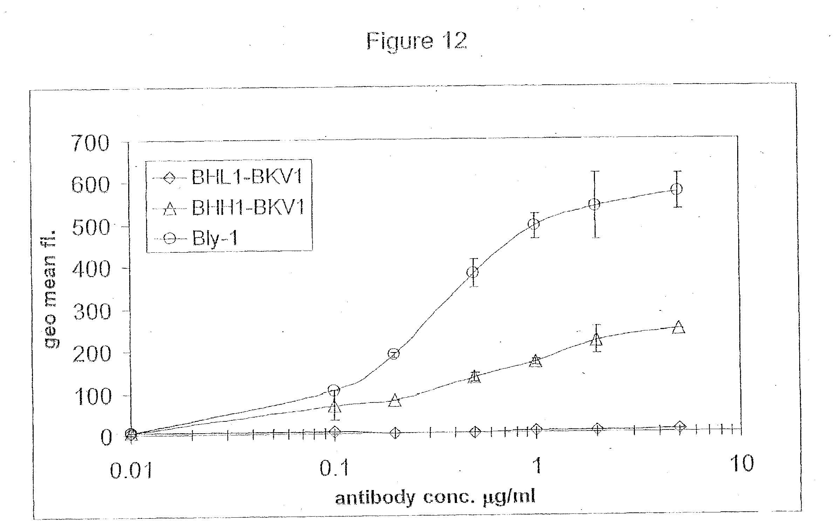

FIG 12. Comparison of the binding behavior between B-HL1, B-HH1, and the B-ly1 parental antibody. The data showed absence of any binding activity in the B-HL1 construct, and about half of the binding intensity /stoichiometry of B-HH1 compared to B-ly1. Both B-HL1, as well as B-HH1, are designed based on acceptor frameworks derived from the human VH1 class. Among other differences, position 71 (Kabat numbering) of the B-HL1 construct is a striking difference, indicating its putative importance for antigen binding.

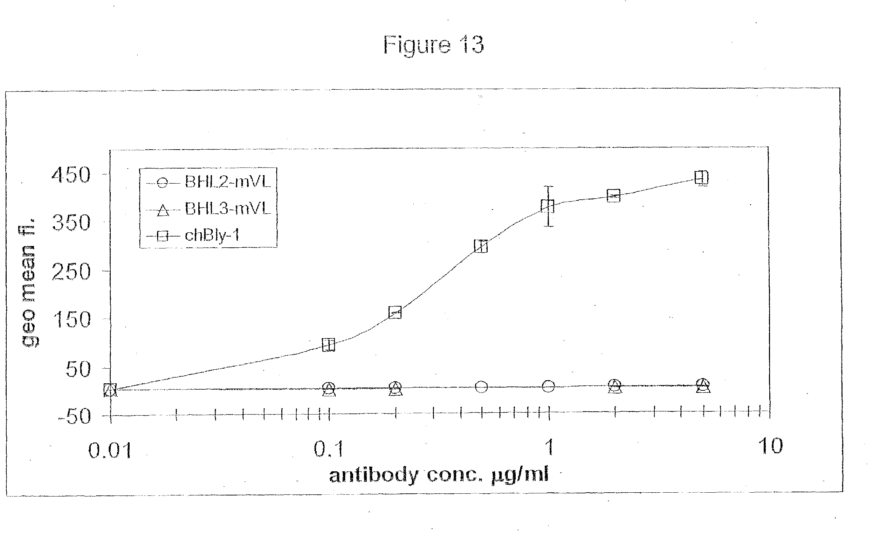

FIG 13. Comparison of the binding behavior between the anti-CD20 antibody heavy chain construct B-HL2, and B-HL3 to its antigen. In both cases the murine VL sequence was combined with the humanized heavy chains The data showed that the B-HL2 and B-HL3 constructs do not display CD-20 binding activity.

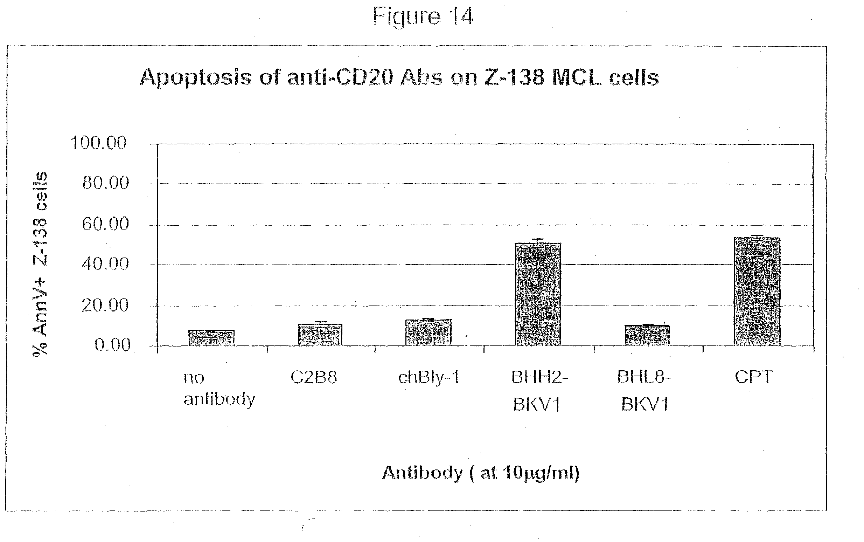

FIG 14. Apoptotic effect of anti-CD20 antibodies on Z-138 MCL cells.

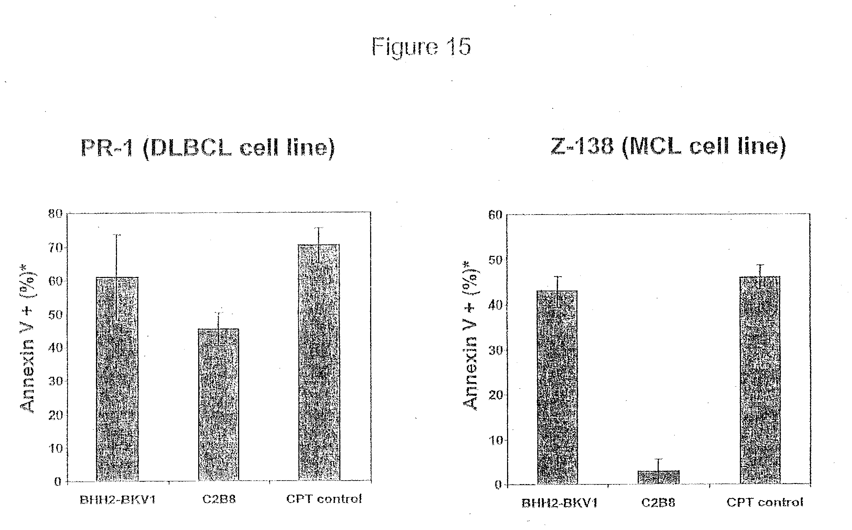

FIG 15. Apoptosis by anti-CD20 antibodies. Assay details: 5 x 10⁵ cells/well were seeded in 24-well plates (5 x 10⁵ cells/ml) in culture medium. 10 µg/ml final concentration of the respective antibody, PBS for the negative control or 5mM Camptothecin (CPT) positive control were added to the wells. Samples were incubated o/n (16 h), stained with AnnV-FITC and analysed by FACS. Assays were performed in triplicate. (*): Signal for PBS alone subtracted (PBS alone gave 8% and 2% AnnV+ for PR-1 and Z-138 cells, respectively). Antibodies used were: C2B8 (chimeric, non-glycoengineered); BHH2-BKV1 (humanized, non-glycoengineered). Note: this assay does not involve any additional effector cells, just targets plus antibody or controls.

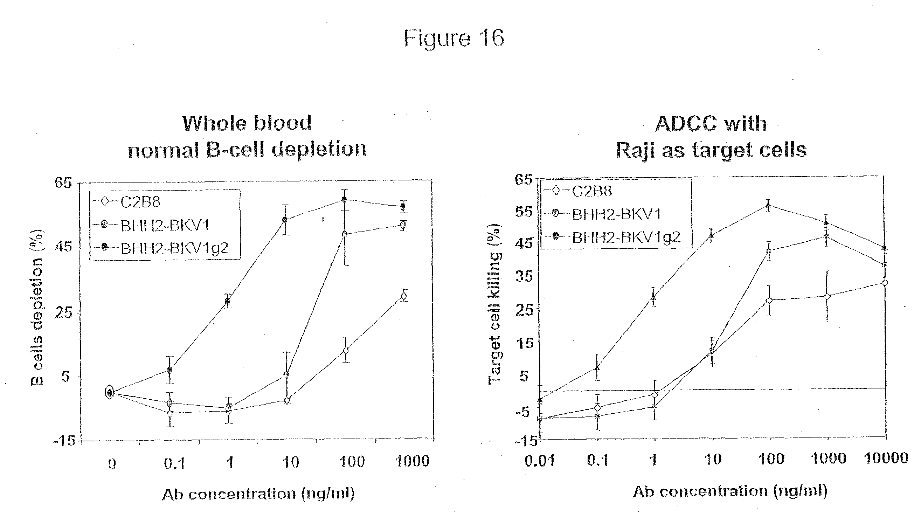

FIG 16. Target-cell killing by anti-CD20 antibodies with immune effector cells. Assay details: B-cell depletion in normal whole blood was determined by overnight incubation and analysis for CD19+/CD3+ by FACS. ADCC was determined using PBMCs as effectors with a 4 h incubation and a 25:1 effector:target ratio. Target-killing was measured by Calcein-retention relative to detergent-lysis (100%) and to lysis without antibody (0%). Antibodies used were: C2B8 (chimeric, non-glycoengineered form); BHH2-BKV1-wt (humanized, non-glycoengineered form of BHH2-BKV1); BHH2-BKV1-GE (humanized, glycoengineered form of BHH2-BKV1).

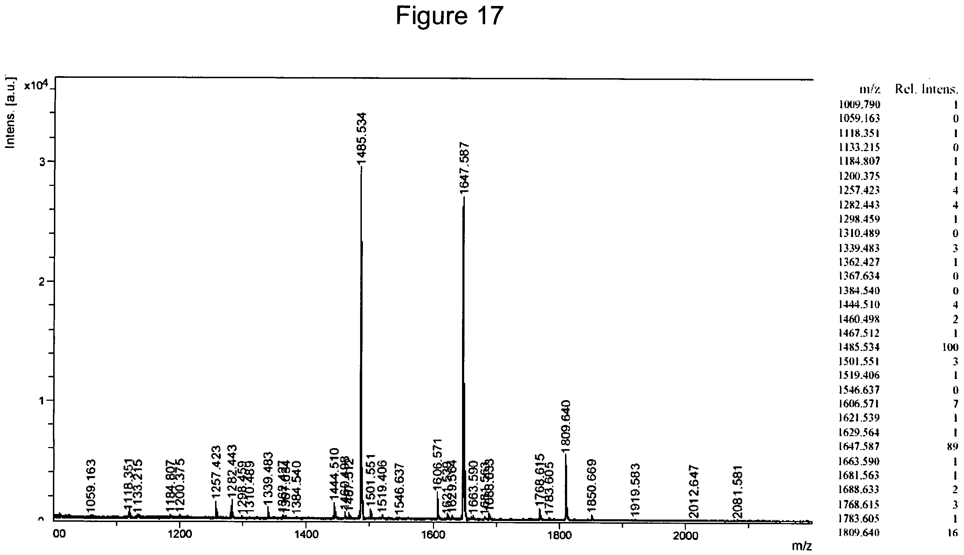

FIG 17. MALDI/TOF-MS profile of PNGaseF-released Fc-oligosaccharides of unmodified, nonglycoengineered BHH2-BKV1 humanized IgG1 B-lyl anti-human CD20 antibody.

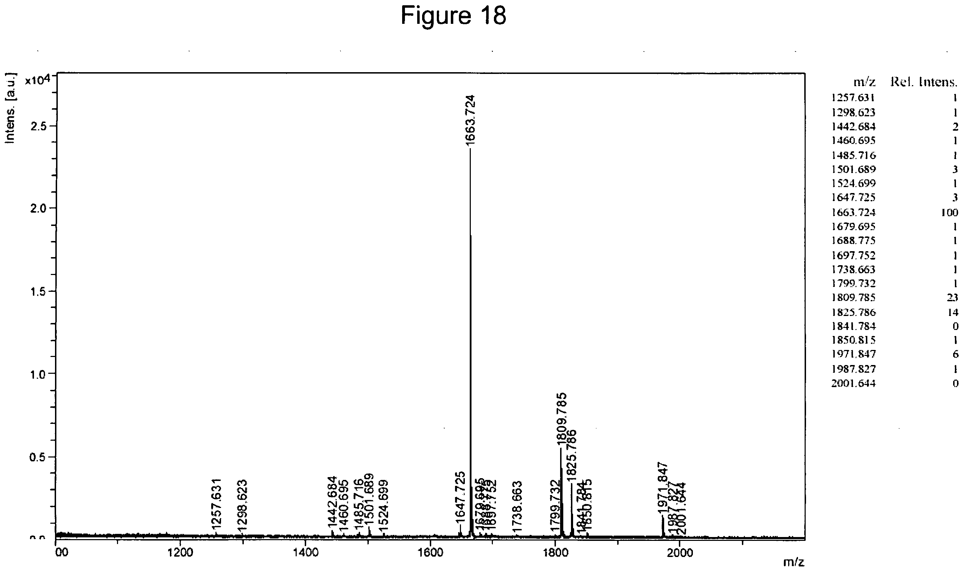

FIG 18. MALDI/TOF-MS profile of PNGaseF-released Fc-oligosaccharides of glycoengineered BHH2-BKV1g1 humanized IgG1 B-ly1 anti-human CD20 antibody. Glycoengineering was performed by co-expression in host cells of antibody genes and a gene encoding an enzyme with β-1,4-N-acetylglucosaminyltransferase III (GnT-III) catalytic activity.

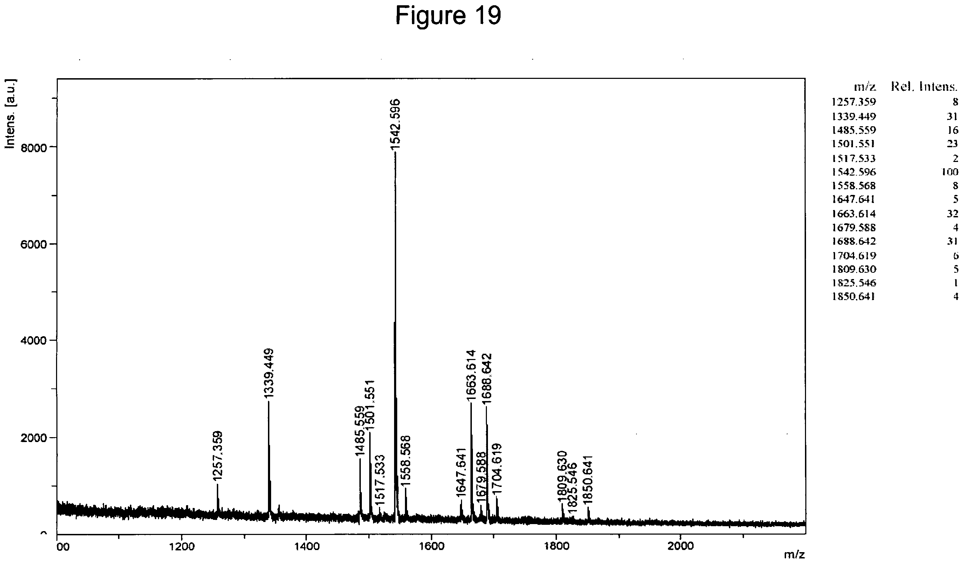

FIG 19. MALDI/TOF-MS profile of PNGaseF-released Fc-oligosaccharides of glycoengineered BEH2-BKV1g2 humanized IgG1 B-ly1 anti-human CD20 antibody. Glycoengineering was performed by co-expression in host cells of antibody genes and genes encoding an enzyme with β-1,4-N-acetylglucosaminyltransferase III (GnT-III) catalytic activity and encoding an enzyme with Golgi α-mannosidase II catalytic activity.

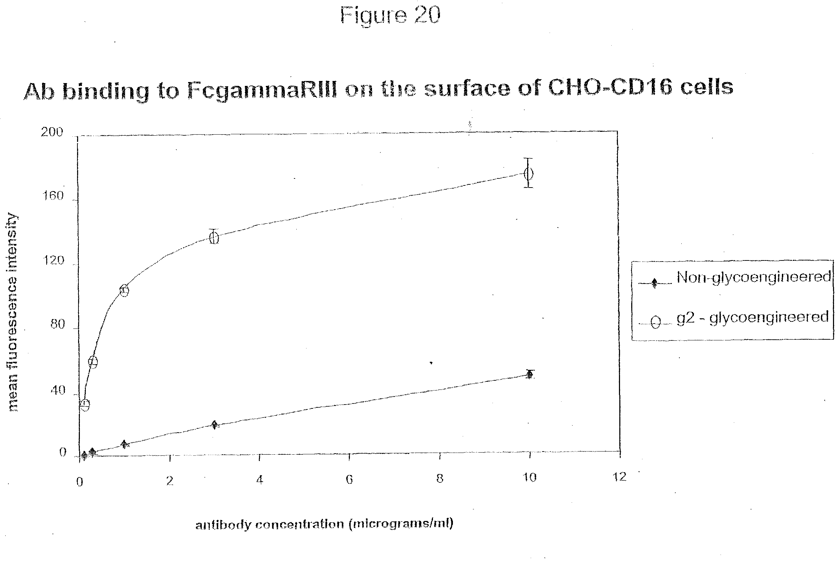

FIG 20. Binding of non-glycoengineered and glycoengineered (g2 version; *see* Figures 17 - 19 for glycosylation profiles) antibodies to human FcgammaRIIIa receptor displayed on the surface of CHO cells expressing recombinant CD16.

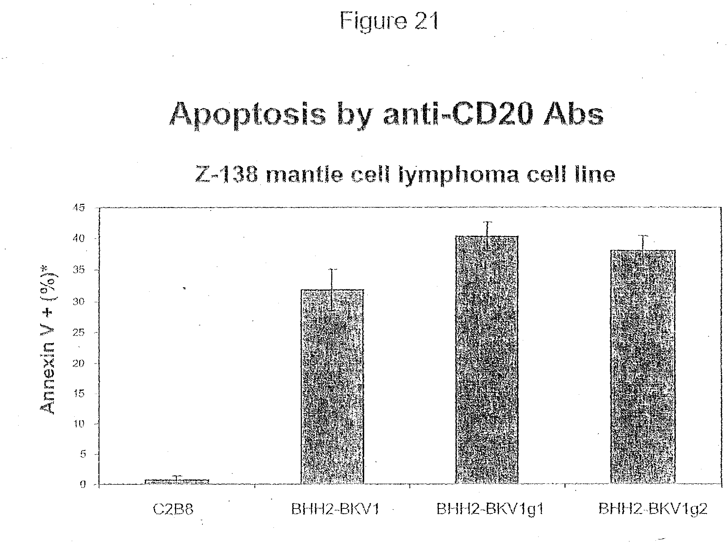

FIG 21. Apoptotic effect of non-Fc engineered and Fc-engineered anti-CD20 antibodies on Z-138 MCL cells. Assay details: 5 x 10⁵ cells/well were seeded in 24-well plates (5 x 10⁵ cells/ml) in culture medium. 10 µg/ml final concentration of the respective antibody, or PBS for the negative control, were added to the wells. Samples were incubated o/n (16 h), stained with AnnV-FITC and analysed by FACS. Assays were performed in triplicate. Antibodies used ware: C2B8 = rituximab (chimeric, non-glycoengineered form); BHH2-BKV1 (humanized, non-glycoengineered-see Figure 17 - 19 for glycosylation profile); BHH2-BKV1g1 (humanized, glycoengineered); BHH2-BKV1g2 (humanized, glycoengineered). Note: this assay does not involve any additional effector cells, just targets plus antibody or controls. (*): Signal for PBS alone was subtracted.

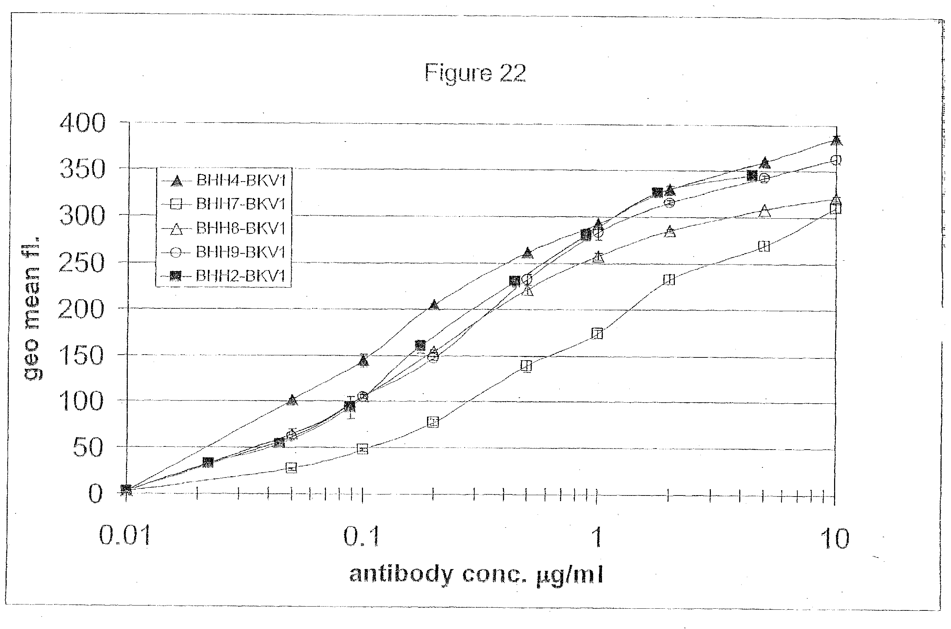

FIG 22. Binding of different humanized anti-CD20 antibodies to Raji B-cells. The humanized heavy chain construct BHH2 is compared to its derivatives BHH4 and BHH7. Also shown are variants that address the influence of Kabat positions 28 and 30 (BHH8 and BHH9).

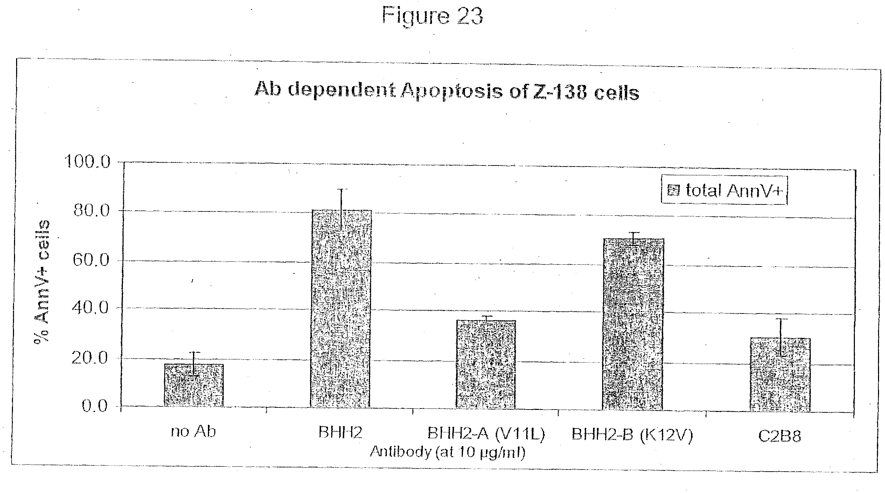

FIG 23. Effect of single amino-acid exchange on apoptosis by anti-CD20 antibodies on Z-138 MCL cells. Assay details: 5 x 10⁵ cells/well were seeded in 24-well plates (5 x 10⁵ cells/ml) in culture medium. 10 µg/ml final concentration of the respective antibody, or PBS for the negative control (no Ab), were added to the wells. Samples were incubated o/n (16 h), stained with AnnV-FITC and analysed by FACS. Assays were performed in triplicate. Antibodies used were: C2B8 (chimeric, non-glycoengineered), BHH2 (humanized, non-glycoengineered), BHH2-A (a derivative of BHH2 with a valine to leucine substitution at Kabat position 11), and BHH2-B (a derivative of BHH2 with a lysine to valine substitution at Kabat position 12), the latter three paired with a BKV1 light chain. K_{D} of antigen binding remains unchanged by substitution. Note: this assay does not involve any additional effector cells, just targets plus antibody or controls.

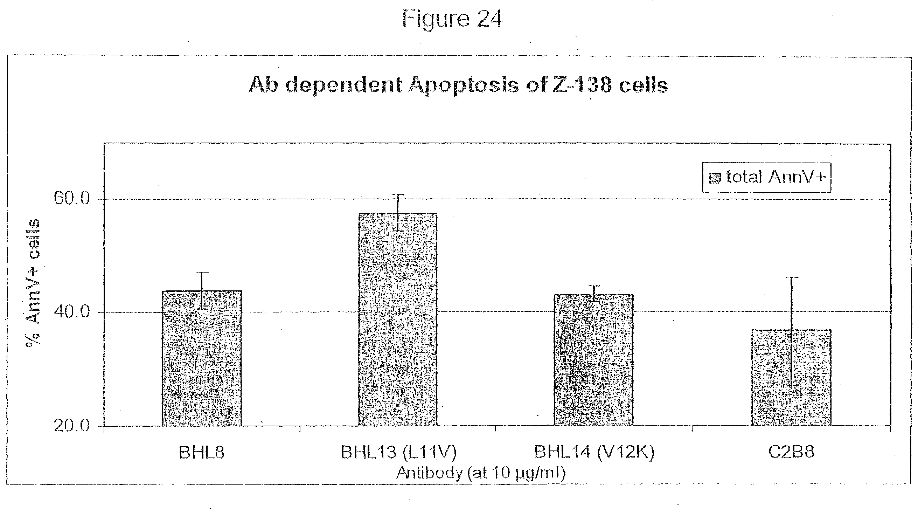

FIG 24. Effect of single amino-acid exchange on apoptosis by previously inactive anti-CD20 antibodies on Z-138 MCL cells. Assay details: 5 x 10⁵ cells/well were seeded in 24-well plates (5 x 10⁵ cells/ml) in culture medium. 10 µg/ml final concentration of the respective antibody, or PBS for the negative control, were added to the wells. Samples were incubated o/n (16h), stained with AnnV-FITC and analysed by FACS. Assays were performed in triplicate. Antibodies used were: C2B8 (chimeric, non-glycoengineered), BHL8 (humanized, non-glycoengineered), BHL13 (a derivative of BHL8 with a leucine to valine substitution at Kabat position 11 and a valine to methionine substitution at Kabat position 48), and BHL14 (a derivative of BHL8 with a valine to lysine substitution at Kabat position 12 and a valine to methionine substitution at Kabat position 48), the latter three paired with a BKV1 light chain. Note: this assay does not involve any additional effector cells, just targets plus antibody or controls.

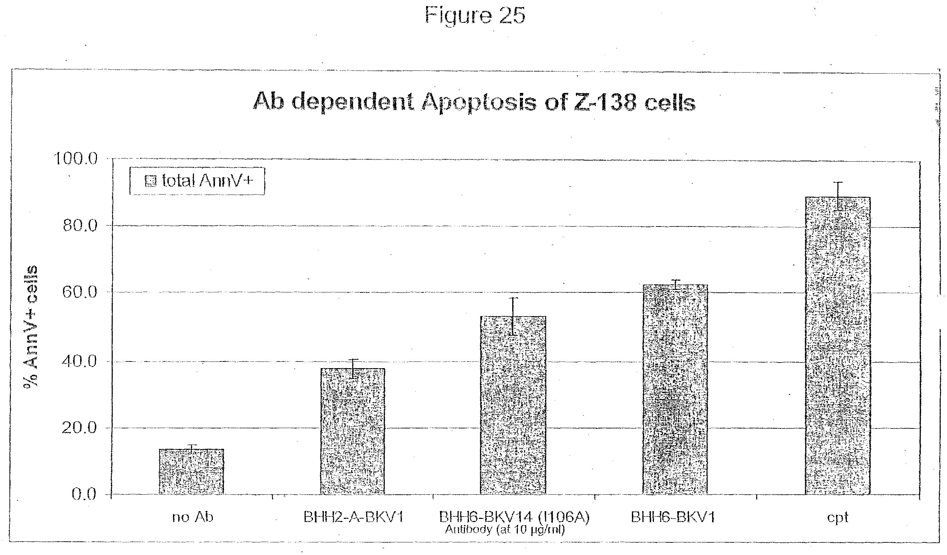

FIG 25. Effect of single amino-acid exchange within the light chain on apoptosis by anti-CD20 antibodies on Z-138 MCL cells. Assay details: 5 x 10⁵ cells/well were seeded in 24-well plates (5 x 10⁵ cells/ml) in culture medium. 10 µg/ml final concentration of the respective antibody, PBS for the negative control (no Ab), or 5mM Camptothecin (CPT) for the positive control were added to the wells. Samples were incubated o/n (16 h), stained with AnnV-FITC and analysed by FACS. Assays were performed in triplicate. Antibodies used were: BHH2-A (a derivative of BHH2 with a valine to leucine substitution at Kabat position 11) paired with a BKV1 light chain, BHH6 (a derivative of BHH2 with a methionine to isoleucine substitution at Kabat position 34) paired with a BKV1 light chain, and BHH6 paired with a BKV14 light chain (a derivative of BKV1 with an isoleucine to alanine substitution at Kabat position 106).

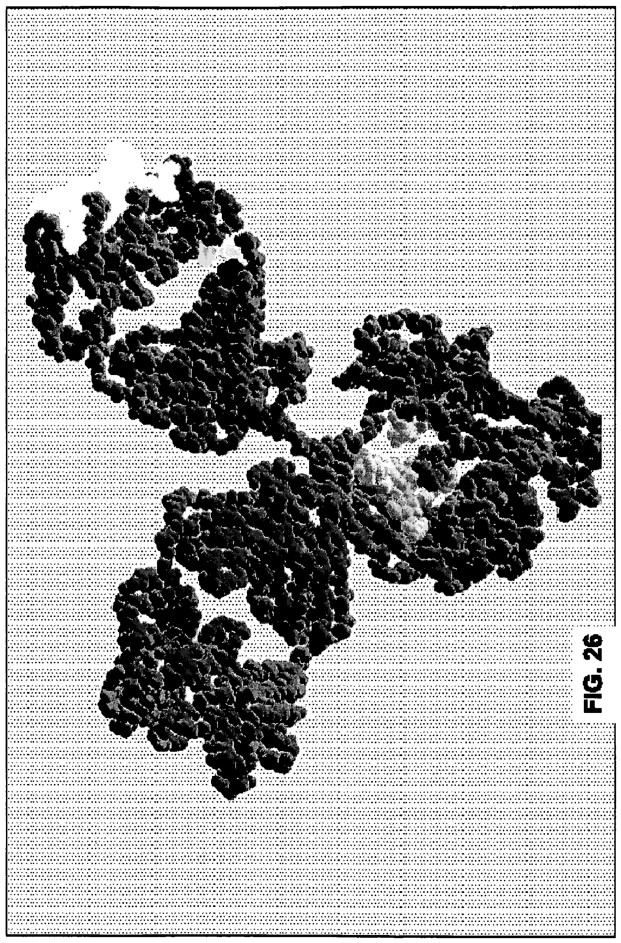

FIG 26. 3-dimensional depiction of a molecular "ball and socket joint" at the interface between the VH and CH1 domains.

### DETAILED DESCRIPTION OF THE INVENTION

Terms are used herein as generally used in the art, unless otherwise defined as follows and herein.

As used herein, the term *antigen binding molecule* (ABM) refers in its broadest sense to amolecule that specifically binds an antigenic determinant. By "specifically binds" is meant that the binding is selective for the antigen and can be discriminated from unwanted or nonspecific interactions. As used herein, the term *modified antigen binding molecule* (or *modified ABM*) is intended to refer to an ABM comprising at least one amino acid residue substitution in the heavy chain variable region and/or CHl region and/or at least one amino acid residue substitution in the light chain variable region and/or CL region.

As used herein, the term *antibody* is intended to include whole antibody molecules, including monoclonal, polyclonal and multispecific (*e.g.*, bispecific) antibodies, as well as antibody fragments having the Fc region and retaining binding specificity, and fusion proteins that include a region equivalent to the Fc region of an immunoglobulin and that retain binding specificity. Also encompassed are antibody fragments that retain binding specificity including, but not limited to, V_{H} fragments, V_{L} fragments, Fab fragments, F(ab')₂ fragments; scFv fragments, Fv fragments, minibodies, diabodies, triabodies, and tetrabodies (*see, e.g.*, Hudson and Souriau, Nature Med. 9: 129-134 (2003), incorporated herein by reference in its entirety). Also encompassed are humanized, primatized and chimeric antibodies. As used herein, *whole antibody* refers to an immunoglobulin molecule comprising two heavy chains and two light chains, each of which comprises a variable and constant region..

As used herein, the term *variable region* is intended to refer to the N-terminal domain of an immunoglobulin heavy or light chain. According to one embodiment of the present disclosure, a modified ABM can comprise a functional fragment of a variable region.

As used herein, the term *heavy chain variable region* is intended to refer to the N-terminal domain of an immunoglobulin heavy chain. In one example, the heavy chain variable region is defined by Kabat positions 1 to 113 (with possible insertions at particular residues as designated by Kabat et al., U.S. Dept. of Health and Human Services, "Sequence of Proteins of Immunological Interest" (1983)). According to one embodiment of the present disclosure, a modified ABM can comprise a functional fragment of a heavy chain variable region.

As used herein, the term *heavy chain constant region* is intended to refer to the C terminal domain of a immunoglobulin heavy chain. There are five naturally-occurring classes of heavy chain constant regions: IgA, IgG, IgE, IgD, and IgM. In one example, the heavy chain oonstant region comprises a CH1 domain, a CH2 domain, and a CH3 domain.

As used herein, the term *CH1 region* is intended to refer to the domain of the heavy chain of an immunoglobulin that is just C-terminal to the variable region and N-terminal to the hinge region. In an immunoglobulin of the IgG type, for example, CH1 is normally defined by Kabat positions 114 to 228.

As used herein, the term *apoptosis* is intended to refer to programmed cell death, which is characterized by certain cellular events such as nuclear fragmentation and/or formation of apoptotic bodies by condensation of cytoplasm, plasma membranes and/or organelles.

As used herein, the term *agonist activity* is intended to refer to activity of an agent (e.g., an antigen binding molecule) when it interacts with (for example, binds to) a molecule associated with a cell surface and initiates or induces a reaction.

As used herein, the term *antagonist activity* is intended to refer to activity of an agent (e.g., an antigen binding molecule) when it interacts with (for example, binds to) a molecule on a cell and prevents initiation or induction of a reaction or discontinues an ongoing reaction.

As used herein, the terms *fusion* and *chimeric,* when used in reference to polypeptides such as ABMs refer to polypeptides comprising amino acid sequences derived from two or more heterologous polypeptides, such as portions of antibodies from different species. For chimeric ABMs, for example, the non-antigen binding components may be derived from a wide variety of species, including primates such as chimpanzees and humans. The constant region of the chimeric ABM is most preferably substantially identical to the constant region of a natural human antibody; the variable region of the chimeric antibody is most preferably derived from a non-human (i.e., donor) antigen binding molecule that specifically binds an antigen of interest. The chimeric ABM may comprise the entire donor variable region; alternatively, the chimeric antibody may comprise a humanized or primatized antibody. Humanized antibodies are a particularly preferred form of fusion or chimeric antibody.

As used herein, the term humanized is used to refer to an antigen binding molecule derived from a non-human antigen-binding molecule, for example, a murine antibody, that retains or substantially retains the antigen-binding properties of the parent molecule but which is less immunogenic in humans. This may be achieved by various methods including (a) grafting the entire non-human variable domains onto human constant regions to generate chimeric antibodies, (b) grafting only the non-human. CDRs onto human framework and constant regions with or without retention of critical framework residues (e.g., those that are important for retaining good antigen binding affinity or antibody functions), or (c) transplanting the entire non-human variable domains, but "cloaking" them with a human-like section by replacement of surface residues. Such methods are disclosed in Jones et al., Morrison et al., Proc. Natl. Acad. Sci., 81.6851-6855 (1984); Morrison and Oi, Adv. Immunol., 44:65-92 (1988); Verhoeyen et al., Science, 239:1534-1536 (1988); Padlan. Molec. Immun., 28:489-498 (1991); Padlan, Molec. Immun., 31(3):169-217 (1994).

There are generally 3 complementarity determining regions, or CDRs, (CDR1, CDR2 and CDR3) in each of the heavy and light chain variable domains of an antibody, which are flanked by four framework subregions (i.e., FR1, FR2, FR3, and FR4) in each of the heavy and light chain variable domains of an antibody: FR1-CDR1-P312-CDR2-FR3-CDR3-FR4. A discussion of humanized antibodies can be found, *inter alia,* in U.S. Patent No. 6,632,927, and in published U.S. Application No. 2003/0175269.

Similarly, as used herein, the term *primatized* is used to refer to an antigen-binding molecule derived from anon-primate antigen-binding molecule, for example, a murine antibody, that retains or substantially retains the antigen-binding properties of the parent molecule but which is less immunogenic in primates.

In the case where there are two or more definitions of a term which is used and/or accepted within the art, the definition of the term as used herein is intended to include all such meanings unless explicitly stated to the contrary. A specific example is the use of the term "complementarity determining region" ("CDR") to describe the non-contiguous antigen combining sites found within the variable region of both heavy and light chain polypeptides. This particular region has been described by Kabat et al., U.S. Dept. of Health and Human Services, "Sequences of Proteins of Immunological Interest" (1983) and by Chothia et al., J. Mol. Biol. 196:901-917 (1987)
where the definitions include overlapping or subsets of amino acid residues when compared against each other. Nevertheless, application of either definition to refer to a CDR of an antibody or variants thereof is intended to be within the scope of the term as defined and used herein. The appropriate amino acid residues which encompass the CDRs as defined by each of the above cited references are set forth below in Table I as a comparison. The exact residue numbers which encompass a particular CDR will vary depending on the sequence and size of the CDR. Those skilled in the art can routinely determine which residues comprise a particular CDR given the variable region amino acid sequence of the antibody.

**TABLE 1. CDR Definitions¹**

| | **Kabat** | **Chothia** | **OxAbM²** |
|---|---|---|---|
| V_{H} CDR1 | 31-35 | 26-32 | 26-35 |
| V_{H} CDR2 | 50-65 | 52-58 | 50-58 |
| V_{H} CDR3 | 95-102 | 95-102 | 95-102 |
| V_{L} CDR1 | 24-34 | 26-32 | 24-34 |
| V_{L} CDR2 | 50-56 | 50-52 | 50-56 |
| V_{L} CDR3 | 89-97 | 91-96 | 89-97 |

| | | | |
|---|---|---|---|
| ¹Numbering of all CDR definitions in Table 1 is according to the numbering conventions set forth by Kabat et al (see below). ²"OxAbM" refers to the CDRs as defined by Oxford Molecular's "AbM" antibody modeling software. | | | |

Kabat *et al.* also defined a numbering system for variable domain sequences that is applicable to any antibody. One of ordinary skill in the art can unambigously assign this system of "Kabat numbering" to any variable domain sequence, without reliance on any experimental data beyond the sequence itself. As used herein, "Kabat numbering" refers to the numbering system set forth by Kabat et al., U.S. Dept of Health and Human Services, "Sequence of Proteins of Immunological Interest" (1983). Unless otherwise specified, references to the numbering of specific amino acid residue positions in an ABM are according to the Kabat numbering system. The sequences of the sequence listing are not numbered according to the Kabat numbering system.

As used herein, *a polypeptide having "GnTIII activity"* refers to polypeptides that are able to catalyze the addition of a N-acetylglocosamine (GlcNAc) residue in β-1-4 linkage to the β-linked marmoside of the trimannosyl core of N-linked oligosaccharides. This includes fusion polypeptides exhibiting enzymatic activity similar to, but not necessarily identical to, an activity of β(1,4)-N-acetylglucosaminyltransferase III, also known as β-1,4-mannosyl-glycoprotein 4-beta-N-acetylglucosaminyl-transferase (EC2.4.1.144), according to the Nomenclature Committee of the International Union of Biochemistry and Molecular Biology (NC-IUBMB), as measured in a particular biological assay, with or without dose dependency. In the case where dose dependency does exist, it need not be identical to that of GnTIII, but rather substantially similar to the dose-dependence in a given activity as compared to the GnTIII (i.e., the candidate polypeptide will exhibit greater activity or not more than about 25-fold less and, preferably, not more than about tenfold less activity, and most preferably, not more than about three-fold less activity relative to the GnTIII.)

As used herein, the term *variant* (or *analog*) refers to a polypeptide differing from a specifically recited polypeptide of the invention by amino acid insertions, deletions, and substitutions, created using, *eg*., recombinant DNA techniques. Variants of the ABMs of the present disclosure include chimeric, primatized or humanized antigen binding molecules wherein one or several of the amino acid residues are modified by replacement, addition and/or deletion in such manner that does not substantially affect antigen binding affinity. Guidance in determining which amino acid residues may be replaced, added or deleted without abolishing activities of interest, maybe found by comparing the sequence of the particular polypeptide with that of homologous peptides and minimizing the number of amino acid sequence changes made in regions of high homology (conserved regions) or by replacing amino acids with consensus sequence.

Alternatively, recombinant variants encoding these same or similar polypeptides may be synthesized or selected by making use of the "redundancy" in the genetic code. Various codon substitutions, such as the silent changes which produce various restriction sites, may be introduced to optimize cloning into a plasmid or viral vector or expression in a particular prokaryotic or eukaryotic system. Mutations in the polynucleotide sequence may be reflected in the polypeptide or domains of other peptides added to the polypeptide to modify the properties of any part of the polypeptide, to change characteristics such as ligand-binding affinities, interchain affinities, or degradation/turnover rate.

As used herein, *amino acid residue substitution* is intended to refer to replacing one or more amino acids in a reference sequence (e.g., a parent molecule, such as an antigen binding molecule). In one embodiment, amino acid residue substitution may be achieved by, for example, a point mutation in the sequence of a nucleic acid encoding a polypeptide as compared to a parent sequence. In another embodiment, substitution of an amino acid residue maybe achieved by replacing the entire framework region of the parent polypeptide with, for example, a framework region from a germline VH sequence that comprises the desired amino acid at the position to be substituted in reference to the parent.

"Conservative" amino acid substitutions are those made by replacing one amino acid with another amino acid having similar structural and/or chemical properties, i.e., conservative amino acid replacements, and may be made on the basis of similarity in polarity, charge, solubility, hydrophobicity, hydrophilicity, and/or the amphipathic nature of the residues involved. For example, nonpolar (hydrophobic) amino acids include glycine, alanine, leucine, isoleucine, valine, proline, phenylalanine, tryptophan, and methionine; polar neutral amino acids include serine, threonine, cysteine, tyrosine, asparagine, and glutamine; positively charged (basic) amino acids include arginine, lysine, and histidine; and negatively charged (acidic) amino acids include aspartic acid and glutamic acid. "Insertions" or "deletions" are preferably in the range of about 1 to 20 amino acids, more preferably 1 to 10 amino acids. The variation allowed may be experimentally determined by systematically making insertions, deletions, or substitutions of amino acids in a polypeptide molecule using recombinant DNA techniques and assaying the resulting recombinant variants for activity.

As used herein, the term *parent antigen binding molecule,* or *parent molecule* refers to a polypeptide having a particular amino acid sequence encoded by a polynucleotide sequence. The sequence of the parent molecule (*i.e*., the *parent sequence*) serves as a reference sequence for making amino acid residue substitutions that alter the ability of the resulting molecule (e.g., a modified antigen binding molecule) to induce or block cell signaling activity and/or cross-linking of antigen. Likewise, the activity of a parent molecule serves as the reference when determining whether a substitution has an effect on cell signaling activity and/or cross-linking of antigen, and, where relevant, the extent of that effect. A sequence containing one or more amino acid substitutions in comparison to its parent (*e.g.*, a modified ABM) may in turn serve as a parent sequence for further substitutions.

As used herein, the term *altered cell signaling activity* is intended to refer to an increase or decrease in the ability of an ABM to induce or inhibit cell signaling activity of a target antigen,

As used herein, the term *altered cross-linking of one or more target antigens* is intended to refer to an increase or decrease in the ability of an ABM to bring into closer proximity to each other, and/or into closer proximity with other membrane-associated molecules, and/orinto a more favorable conformation for interaction target antigens that are capable of forming complexes (e.g., through cross-linking of proteins, or oligomerization of membrane-associated receptors) to initiate cell signaling pafhways.

As used herein, *cell signaling mechanism* or *cell signaling activity* is intended to refer to the entire signaling (i.e., signal transduction) pathway that leads to a particular cellular event or biological function, as well as any signaling steps along the pathway.

By a nucleic acid or polynucleotide having a nucleotide sequence at least, for example, 95% "identical" to a reference nucleotide sequence of the present disclosure, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence.

As a practical matter, whether any particular nucleic acid molecule or polypeptide is at least 80%, 85%, 90%, 95%, 96%, 97%, 98% or 99% identical to a nucleotide sequence or polypeptide sequence of the present disclosure can be determined conventionallyusingknown computer programs. A preferred method for determining the best overall match between a query sequence (a sequence of the present disclosure) and a. subject sequence, also referred to as a global sequence alignment, can be determined using the FASTDB computer program based on the algorithm of Brutlag et al., Comp. App. Biosci, 6:237-245 (1990). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identity are: Matrix=Unitary, k-tuple=4, Mismatch Penalty=1, Joining Panalty=30, Randomization Group Length=0, Cutoff Score=1, Gap Penalty=5, Gap Size Penalty 0.05, Window Size=500 or the length of the subject nucleotide sequence, whichever is shorter.

If the subject sequence is shorter than the query sequence because of 5' or 3' deletions, not because of internal deletions, a manual correction must be made to the results. This is because the FASTDB program does not account for 5' and 3' truncations of the subject sequence when calculating percent identity. For subject sequences truncated at the 5' or 3' ends, relative to the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present disclosure. Only bases outside the 5' and 3' bases of the subject sequence, as displayed by the FASTDB alignment, which are not matched/aligned with the query sequence, are calculated for the purposes of manually adjusting the percent identity soore.

For example, a 90 base subject sequence is aligned to a 100 base query sequence to determine percent identity. The deletions occur at the 5' end of the subject sequence and therefore, the FASTDB alignment does not show a matched/alignment of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' end of the subject sequence which are not matched/aligned with the query. In this case the percent identity calculated by FASTDB is not manually corrected. Once again, only bases on the 5' and 3' end of the subject sequence which are not matched/aligned with the query sequence are manually corrected for. No other manual corrections are to made for the purposes of the present disclosure.

By a polypeptide having an amino acid sequence at least, for example, 95% "identical" to a query amino acid sequence of the present disclosure, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid sequence at least 95% identical to a query amino acid sequence, up to 5% of the amino acid residues in the subject sequence may be inserted, deleted, or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 80%, 85%, 90%, 95%, 96%, 97%, 98% or 99% identical to a reference polypeptide can be determined conventionally using known computer programs. A preferred method for determining the best overall match between a query sequence (a sequence of the present disclosure) and a subject sequence, also referred to as a global sequence alignment, can be determined using the FASTDB computer program based on the algorithm of Brutlag et al., Comp. App. Biosci. 6:237-245 (1990). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty=1, Joining Penalty=20, Randomization Group Longth=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This, is because the FASTDB program does not account for N- and C-terminal truncations of the subject sequence when calculating global percent identity. For subject sequences truncated at the N- and C-termini, relative to the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment This percentage is then subtracted from the percent identity, calculated by the above FASTDB program using the specified parameters, to arrive at a final percent identity score. This final percent identity score is what is used for the purposes of the present disclosure. Only residues to the N- and C-termini of the subject sequence, which are not matched/aligned with the query sequence, are considered for the purposes of manually adjusting the percent identity score, That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C- termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequence are manually corrected for. No other manual corrections are to be made for the purposes of the present disclosure.

As used herein, a nucleic acid that "*hybridizes under stringent conditions*" to a nucleic acid sequence of the disclosure, refers to a polynucleotide that hybridizes in an overnight incubation at 42° C in a solution comprising 50% formamide, 5x SSC (750 mM NaCl, 75 mM sodium citrate), 50 mM sodium phosphate (pH 7.6), 5x Denhardt's solution, 10% dextran sulfate, and 20 µg/ml denatured, sheared salmon sperm DNA, followed by washing the filters in 0.1x SSC at about 65°C.

As used herein, the term *Fc region* is intended to refer to a C-terminal region of an IgG heavy chain. Although the boundaries of the Fc region of an IgG heavy chain might vary slightly, the human IgG heavy chain Fc region is usually defined to stretch from the amino acid residue at position Cys226 to the carboxyl-terminus.

As used herein, the term *region equivalent to the Fc region of an immunoglobulin* is intended to include naturally occurring allelic variants of the Fc region of an immunoglobulin as well as variants having alterations which produce substitutions, additions, or deletions but which do not decrease substantially the ability of the immunoglobulin to mediate effector functions (such as antibody dependent cellular cytotoxicity). For example, one or more amino acids can be deleted from the N-terminus or C-terminus of the Fc region of an immunoglobulin without substantial loss of biological function. Such variants can be selected according to general rules known in the art so as to have minimal effect on activity. (*See, e.g.,* Bowie, J. U. et al., Science 247:1306-10 (1990)). In one embodiment, *a region equivalent to the Fc region* can also form part of a heterologous fusion protein. In some embodiments, a *region equivalent to the Fc region* also encompasses a corresponding region from another class of immunoglobulin heavy chain (e.g., IgA, IgE, IgD, and IgM).

As used herein, the term *Golgi localization domain* refers to the amino acid sequence of a Golgiresident polypeptide which is responsible for anchoring the polypeptide in location within the Golgi complex. Generally, localization domains comprise amino terminal "tails" of an enzyme.

As used herein, the term *effector function* refers to those biological activities attributable to the Fc region (a native sequence Fc region or amino acid sequence variant Fc region) of an antibody. Examples of antibody effector functions include, but are not limited to, Fc receptor binding affinity, antibody-dependent cellular cytotoxicity (ADCC), antibody-dependent cellular phagocytosis (ADCP), cytokine secretion, immune-complex-mediated antigen uptake by antigen-presenting cells, down-regulation of cell surface receptors, etc.

As used herein, the terms *engineer, engineered*, *engineering, glycoengineer*, *glycoengineerea*, *glycoengineering*, and *glycosylation engineering* are considered to include any manipulation of the glycosylation pattern of a naturally occurring or recombinant polypeptide, such as an antigen binding molecule (ABM), or fragment thereof. Glycosylation engineering includes metabolic engineering of the glycosylation machinery of a cell, including genetic manipulations of the oligosaccharide synthesis pathways to achieve altered glycosylation of glycoproteins expressed in cells. In one embodiment, the glycosylation engineering is an alteration in glycosyltransferase activity. In a particular embodiment, the engineering results in altered glucosaminyltransferase activity and/or fucosyltransferase activity.

As used herein, the term *host cell* covers any kind of cellular system which can be engineered to generate the polypeptides and antigen-binding molecules of the present disclosure. Host cells include cultured cells, e.g., mammalian cultured cells, such as CHO cells, BHK cells, HEK293-EBNA cells, NS0 cells, SP2/0 cells, Y0 myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue. In one embodiment, the host cell is engineered to allow the production of an antigen binding molecule with modified glycoforms. In a preferred embodiment, the antigen binding molecule is an antibody, antibody fragment, or fusion protein. In certain embodiments, the host cells have been further manipulated to express increased levels of one or more polypeptides having GnTIII activity. In other embodiments, the host cells have been engineered to have eliminated, reduced or inhibited core α1,6-fucosyltransferase activity. The term *core α1,6-fucosyltransferase activity* encompasses both expression of the core α1,6-fucosyltransferase gene as well as interaction of the core α1,6-βicosyitransferase enzyme with its substrate.

As used herein, the term *Fc-mediated cellular cytotoxicity* includes antibody-dependent cellular cytotoxicity and cellular cytotoxicity mediated by a soluble Fc-fusion protein containing a human Fc-region. It is an immune mechanism leading to the lysis of "antibody-targeted cells" by "human immune effector cells", wherein:

The *human immune effector cells* are a population of leukocytes that display Fc receptors on their surface through which they bind to the Fc-region of antibodies or of Fc-fusion proteins and perform effector functions. Such a population may include, but is not limited to, peripheral blood mononuclear cells (PBMC) and/or natural killer (NK) cells.

The *antibody-targeted cells* are cells bound by the antibodies or Fc-fusion proteins. The antibodies or Fc fusion-proteins bind to target cells via the protein' part N-terminal to the Fc region.

As used herein, the term *increased Fc-mediated cellular cytotoxicity* is defined as either an increase in the number of "antibody-targeted cells" that are lysed in a given time, at a given concentration of antibody, or of Fc-fusion protein, in the medium surrounding the target cells, by the mechanism of Fc-mediated cellular cytotoxicity defined above, and/or a reduction in the concentration of antibody, or of Fc-fusion protein, in the medium surrounding the target cells, required to achieve the lysis of a given number of "antibody-targeted cells", in a given time, by the mechanism of Fc -mediated cellular cytotoxicity. The increase in Fc-mediated cellular cytotoxicity is relative to the cellular cytotoxicity mediated by the same antibody, or Fc-fusion protein, produced by the same type of host cells, using the same standard production, purification, formulation and storage methods, which are known to those skilled in the art, but that has not been produced by host cells engineered to express the glycosyltransferase GnTIII by the methods described herein.

By *antibody having increased antibody dependent cellular cytotoxicity (ADCC)* is meant an antibody, as that term is defined herein, having increased ADCC as determined by any suitable method known to those of ordinary skill in the art. One accepted ADCC assay is described in the Examples set forth herein below. Another accepted *in vitro* ADCC assay is as follows:
1) the assay uses target cells that are known to express the target antigen recognized by the antigen-binding region of the antibody;
2) the assay uses human peripheral blood mononuclear cells (PBMCs), isolated from blood of a randomly chosen Healthy donor, as effector cells;
3) the assay is carried out according to following protocol:
   i) the PBMCs are isolated using standard density centrifugation procedures and are suspended at 5 x 10⁶ cells/ml in RPMI cell culture medium;
   ii) the target cells are grown by standard tissue culture methods, harvested from the exponential growth phase with a viability higher than 90%, washed in RPMI cell culture medium, labeled with 100 micro-Curies of ⁵¹Cr, washed twice with cell culture medium, and resuspended in cell culture medium at a density of 10⁵ cells/ml;
   iii) 100 microliters of the final target cell suspension above are transferred to each well of a 96-well microtiter plate;
   iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;
   v) for the maximum release (MR) controls, 3 additional wells in the plate containing the labeled target cells, receive 50 microliters of a 2% (V/V) aqueous solution of non-ionic detergent (Nonidet, Sigma, St. Louis), instead of the antibody solution (point iv above);
   vi) for the spontaneous release (SR) controls, 3 additional wells in the plate containing the labeled target cells, receive 50 microliters of RPMI cell culture medium instead of the antibody solution (point iv above);
   vii) the 96-well microtiter plate is then centrifuged at 50 x g for 1 minute and incubated for 1 hour at 4°C;
   viii) 50 microliters of the PBMC suspension (point i above) are added to each well to yield an effector:target cell ratio of 25:1 and the plates are placed in an incubator under 5% CO₂ atmosphere at 37°C for 4 hours;
   ix) the cell-free supernatant from each well is harvested and the experimentally released radioactivity (ER) is quantified using a gamma counter;
   x) the percentage of specific lysis is calculated for each antibody concentration according to the formula (ER-MR)/(MR-SR) x 100, where ER is the average radioactivity quantified (see point ix above) for that antibody concentration, MR is the average radioactivity quantified (see point ix above) for the MR controls (see point v above), and SR is the average radioactivity quantified (see point ix above) for the SR controls (see point vi above);
4) "increased ADCC" is defined as either an increase in the maximum percentage of specific lysis observed within the antibody concentration range tested above, and/or a reduction in the concentration of antibody required to achieve one half of the maximum percentage of specific lysis observed within the antibody concentration range tested above. The increase in ADCC is relative to the ADCC, measured with the above assay, mediated by the same antibody, produced by the same type of host cells, using the same standard production, purification, formulation and storage methods, which are known to those skilled in the art, but that has not been produced by host cells engineered to overexpress GnTIII.

### Antigen Binding Molecules with Heavy Chain and/or Light Chain Amino Acid Substitutions

In one aspect, the present disclosure is directed to ABMs comprising modfied heavy chain and/or light chain V regions and/or C regions, and to the discovery that the ability of these ABMs to induce cell signaling activity of a target antigen and/or mediate cross-linking of target antigen can be enhanced (i.e., induced or increased) or reduced (i.e., inhibited or decreased) by such modifications. Thus, the present disclosure provides polypeptides, including ABMs, having modified heavy chain and/or light chain V regions and/or C regions, nucleic acid sequences (e.g. vectors) encoding such polypeptides, methods for generating polypeptides having modified heavy chain and/or light chain V regions and/or C regions, and methods for using same in the treatment of various diseases and disorders.

It is known that several mechanisms are involved in the therapeutic efficacy of antibodies, including antibody dependent cellular cytotoxicity (ADCC), complement-dependant cytotoxicity (CDC), and the induction of growth arrest or apoptosis and the blocking or inhibition of cell growth, cell proliferation, cell survival and/or other cellular events. For example, instances of induction of cell death and other cell signaling events by agonistic monoclonal antibodies have been reported. Cerisano *et al*., showed the induction of caspase-independent cell death characterized by apoptosis-like features (including phosphatidyl-serine (PS) exposure, morphological changes and/or propidium-iodide (PI) uptake), as well as homotypic aggregation of Ewing's sarcoma cells, by stimulation with agonistic antibodies against the transmembrane glycoprotein, CD99 (*e.g*., anti-CD99 013 MAb and 0662 MAb) (Cerisano et al., Oncogene 23: 5664-5674 (2003)). Likewise, Hahn *et al* reported activation of MAPK signaling pathways by engagement of CD99 with anti-CD99 monoclonal antibodies (e.g., DN16 and YG32), which led to homotypic aggregation of cells (Hahn et al., FEBS Letters 470: 350-354 (2000)). Pettersen *et al*. identified a new functional domain of CD99 that could be activated by the anti-CD99 monoclonal antibody, Ad20, which activation induced apoptosis in transformed T cells (Pettersen et al., J. Immunol. 166:4931-4942 (2001)). Monoclonal antibodies against CD47 (e.g., B6H12) can also induce caspase-independent cell death, which is associated with cytoskeletal reorganization signaling pathways (Mateo et al., Blood 100:2882-2890 (2002)).

In other examples, certain antibodies against CD20 (e.g., rituximab and tositumomab) and CD52 (CAMPATH-1H) have been shown to directly induce apoptosis in tumor cells. *See* Ludwig et al., Oncogene 22: 9097-9106 (2003). For rituximab and several other monoclonal antibodies with little or no signaling activity (anti-CD19, CD21, CD22 and Her2), ability to induce apoptosis or growth arrest was enhanced by chemically converting the antibodies into IgG-IgG homodimers. Ghetie et al., Proc. Natl. Acad. Set. 94:7509-14 (1997). It was speculated that the enhancement was due to increased negative signaling and/or hypercrosslinking by the tetravalent antibody homadimers. Ghetie et al., Proc. Natl. Acad. Sci. 94:7509-14 (1997). Cross-linking and increased apoptosis have also been achieved through the use of secondary antibodies or Fc-receptor-bearing accessory cells. *See* Jazhirehi and Bonavida, Oncogene 24:2121-43 (2005).

Without wishing to be bound by theory, the present inventors have determined that modifications to the amino acid residues in the elbow hinge region of an antigen binding molecule can affect the ability of the ABM to induce or inhibit signaling activity and/or cross-linking of target antigen. The angle of the elbow hinge region controls the orientation of the V region with respect to the C region of an immunoglobulin, and as such, facilitates the interactions of' antibodies with antigen and effector proteins. *See* Lesk and Chothia, Nature 335: 188-90 (1988). Lesk and Chothia identified the residues that make up the molecular ball-and-socket joint of the elbow hinge region in antibodies, i.e:, Kabat positions 11, 110, and 112 in the VH region and positions 149 and 150 in the CH1 region, and noted the high degree of conservation across antibodies of residues that make up this joint Lesk and Chothia, Nature 335: 188-90 (1988)
However, they did not make modifications to the ball-and-socket residues or the residues in their vicinity. Landolfi *et al.* showed that modifications to Kabat positions 10-13 in AF2, a neutralizing antibody against human IFNγ, resulted in a significant loss in neutralizing activity of the antibody, but did not affect binding of the antibody to its target antigen. Landolfi et al., J. Immunol 166: 1748-54 (2001).
However, Landolfi *et al.* did not show an effect on the ability of an antibody to induce cell signaling or to mediate antigen cross-linking.

With a multivalent ABM, the ability to change the orientation of the antigen binding sites allows for the adjustment of the proximity of bound antigen units when it is complexed with the multiple antigen binding sites. The proximity of the antigen units to each other facilitates greater or lesser interaction (e.g., cross-linking, dimerization, etc.) between the antigen units. For example, if the elbow angle of each VH/VL-CH1/CL pair in an ABM is oriented such that the antigen binding sites are brought into closer proximity to each other, the bound antigen units (e.g, cell surface receptor molecules) will also be brought into closer proximity to each other or brought into a conformation that is more favorable for interaction. This proximity or conformational change can mediate interactions, for example, cross-linking and oligomerization, between the bound antigens. On the other hand, orientation of the antigen binding sites so that they are farther apart or have a less favorable conformation can prevent them from interacting.

Amino acid residues at the VL-CL interface can also be modified to affect the antigen binding site orientation. For example, Kabat residues 40,80,83,105, and 106 in the light chain variable region frameworks are situated at the VL/CL interface.

The activity of any cell signaling mechanisms can be affected (i.e. induced or inhibited) by the ABMs of the present disclosure. In one aspect of the disclosure, the cell signaling mechanisms involved are those initiated through cell surface receptor proteins including ion-channel linked, G-protein-linked, and enzyme-linked cell-surface receptor proteins. *See generally,* Chapter 15: Cell Signaling in MOLECULAR BIOLOGY OF THE CELL, Alberts et al., eds., (3d ed, 1994) Thus, for example, the cell signaling activities of the present disclosure include, but are not limited to, those which result in apoptosis, cell differentiation, cell growth, cell proliferation, and cell survival, as well as any of the signaling steps along the pathway. In one embodiment, the cell signaling activity occurs through an enzyme-linked receptor; in a particular embodiment, the enzyme-linked receptor is a receptor tyrosine kinase. In another embodiment, the cell signaling activity is through an ion channel-linked receptor.

The modified heavy chain or light chain V regions and/or C regions of the ABMs of the present disclosure differ from the corresponding nonmodified parent polypeptide (e.g., a parent antigen binding molecule) regions by at least one amino acid substitution. The "parent," "starting," or "nonmodified" polypeptide preferably comprises at least a portion of an antibody heavy chain or light chain, and may be prepared using techniques available in the art for generating polypeptides comprising a heavy chain V region or CH1 region or portion thereof and/or a light chain V or C region or portion thereof. In specific embodiments, the parent polypeptide is an antigen binding molecule and comprises at least a portion of a VH or VL region. In certain embodiments, a modified heavy chain and/or light chain V region may be generated (e.g. according to the methods disclosed herein) and can be fused to a heterologous polypeptide of choice, such as an antibody Fc. In one embodiment of the present disclosure, a modified ABM or fragment thereof comprises a fusion protein, wherein a modified heavy chainV region or fragment thereof is fused to a heavy chain constant region selected from the group consisting of IgA, IgG, IgE, IgD, and IgM, or a fragment or derivative thereof. In a particular embodiment, the heavy chain constant region is IgG. In another embodiment of the present disclosure, a modified ABM or fragment thereof comprises a fusion protein, wherein a modified light chain V region or fragment thereof is fused to a light chain constant region selected from the group consisting of IgA, IgG, IgE, IgD, and IgM, or a fragment or derivative thereof In a particular embodiment, the light chain constant region is IgG. In specific embodiments, the polypeptides of the disclosure comprise a whole antibody (e.g., IgG) comprising light chains and heavy chains having a modified heavy chain and/or light chain V region.

Polynucleotides encoding a polypeptide comprising a modified heavy chain V region or CH1 region or light chain V region or CL region may be prepared by methods known in the art using the guidance of the present specification for particular sequences. These methods include, but are not limited to, preparation by site-directed (or oligonucleotide-mediated) mutagenesis, PCR mutagenesis, and cassette mutagenesis of an earlier prepared nucleic acid encoding the polypeptide. Site-direoted mutagenesis is a preferred method for preparing substitution variants. This technique is well known in the art (*see e.g*., Carter et al. Nucleic Acids Res. 13: 4431-4443 (1985) and Kunkel et. al., Proc. Natl. Acad. Sci. USA 82: 488 (1987).

Briefly, in carrying out site directed mutagenesis of DNA, the starting DNA is altered by first hybridizing an oligonucleotide encoding the desired mutation to a single strand of such starting DNA. After hybridization, a DNA polymerase is used to synthesize an entire second strand, using the hybridized oligonucleotide as a primer, and using the single strand of the starting DNA as a template. Thus, the oligonucleotide encoding the desired mutation is incorporated in the resulting double-stranded DNA.

PCR mutagenesis is also suitable for making amino acid sequence variants of the nonmodified starting polypeptide (see, e.g., Vallette et. al., Nuc. Acids Res. 17: 723-733 (1989). Briefly, when small amounts of template DNA are used as starting material in a PCR, primers that differ slightly in sequence from the corresponding region in a template DNA can be used to generate relatively large quantities of a specific DNA fragment that differs from the template sequence only at the positions where the primers differ from the template.

Another method for preparing ABM variants, cassette mutagenesis, is based on the technique described by Wells et al., Gene 34: 315-323 (1985).

The starting material is the plasmid (or other vector) comprising the starting polypeptide DNA to be modified. The codon(s) in the starting DNA to be mutated are identified. There must be a unique restriction endonuclease site on each side of the identified mutation site(s). If no such restriction sites exist, they may be generated using the above-described oligonucleotide-mediated mutagenesis method to introduce them at appropriate locations in the starting polypeptide DNA. The plasmid DNA is cut at these sites to linearize it. A double-stranded oligonucleotide encoding the sequence of the DNA between the restriction sites but containing the desired mutation(s) is synthesized using standard procedures, wherein the two strands of the oligonucleotide are synthesized separately and then hybridized together using standard techniques. This double-stranded oligonucleotide is referred to as the cassette. This cassette is designed to have 5' and 3'ends feat are compatible with the ends of the linearized plasmid, such that it can be directly ligated to the plasmid. This plasmid now contains the mutated DNA sequence.

Alternatively, or additionally, the desired amino acid sequence encoding a polypeptide variant can be determined, and a nucleic acid sequence encoding such an amino acid sequence variant can be generated synthetically.

The amino acid sequence of the parent polypeptide may be modified to generate an ABM having a modified heavy chain V region and/or modified CH1 region, and/or a modified light chain V region and/or modified CL region, with altered ability to induce cell signaling activity of a target antigen when the modified ABM is complexed with (e.g., bound to) the target antigen. The cell signaling activity can be agonist activity or antagonist activity. According to one aspect of the disclosure, agonist activity is induced by a modified antigen binding molecule when it binds to a cell membrane-associated receptor and initiates a cell signaling pathway, In a specific embodiment, the cell signaling pathway is an apoptosis pathway. In another embodiment, the cell signaling pathway is a cell differentiation pathway. According to another aspect of the disclosure, antagonist activity by a modified antigen binding molecule occurs, for example, when the ABM binds to a cell membrane-associated receptor and prevents the induction of a cell signaling pathway or disrupts an ongoing signal. Antagonist activity may be achieved, for example, by blocking the binding and subsequent signal transduction of an endogenous ligand and/or by preventing the cross-linking or oligomerization of receptors or other molecules that would be necessary for induction of a cell signaling pathway. In one embodiment, the cell signaling pathway that is inhibited or disrupted is a cell growth pathway. In another embodiment, the cell signaling pathway that is inhibited or disrupted is a cell division pathway. In another embodiment the cell signaling pathway that is inhibited or disrupted is a cell survival pathway.

Likewise, the amino acid sequence of the parent polypeptide may also be modified to generate an ABM having a modified heavy chain V region or modified C region (e.g., a modified CH1 region), and/or a modified light chain V region and/or modified CL-region, with altered ability to mediate cross-linking of one or more target antigens when the modified ABM is complexed with (e:g., bound to) the target antigen(s). In one embodiment, the bound target antigens (e.g, cell surface receptor molecules) are brought into closer proximity to each other and/or a more favorable conformation for interaction than they would be by the corresponding non-modified parent ABM, thereby increasing cross-linking and oligomerization between the bound antigens. In another embodiment, the bound target antigens (e.g, cell surface receptor molecules) are kept farther apart from each other, and/or in a less favorable conformation for interaction than they would be by the corresponding non-modified parent ABM, thereby reducing or preventing cross-linking and oligomerization between the bound antigens. In a particular embodiment, the increased cross-linking or oligomerization results in increased apoptosis. In another embodiment, the increased cross-linking or oligomerization results in increased cell differentiation. In another embodiment, the reduction in cross-linking or oligomerization results in decreased cell growth, decreased cell division, or decreased cell survival.

Substantial modifications in the biological properties of the heavy chain V region or CH1 region or light chain V region or CL region, may be accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into classes based on common side-chain properties:
(1) hydrophobic: met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for a member of another class. Conservative substitutions will entail exchanging a member of one of these classes for another member of the same class.

### Exemplary Polypeptides Comprising Modified ABMs

In one aspect, the present disclosure is related to antigen binding molecues with amino acid modifications that alter the ability of the ABMs to induce cell signaling activity and/or to mediate cross-linking of antigens. In one embodiment, the modification to the ABM comprises at least one amino acid residue substitution in at least one framework region of the heavy chain or light chain variable region as compared to a parent molecule. In a particular embodiment, the substitution replaces an amino acid residue in heavy chain FR1. In a preferred embodiment, the modification to the ABM comprises a substitution of an amino acid residue at one or more of Kabat positions 8, 9, 10, 11, 12, or 13 in the heavy chain variable region. In another embodiment, the modification to the ABM comprises a substitution of an amino acid residue in heavy chain FR4. In a particular embodiment, the modification to the ABM comprises a substitution of an amino acid residue at one or more of Kabat positions 110 or 112 in the heavy chain variable region. In another embodiment, the modification to the ABM comprises at least one amino acid residue substitution in the light chain at the interface between the V and C regions. In a more particular embodiment, the modification to the ABM comprises a substitution of an amino acid residue at one or more of Kabat positions 40, 80, 83, 105, or 106.

In one embodiment, an amino acid may be substituted by generating a point mutation in the parent sequence that results in the desired change in the amino acid residue(s). Alternatively, the modification to the ABM may comprise replacing an entire framework region of a parent molecule with a framework region that comprises a desired amino acid residue at a particular position. In a particular embodiment, the modification to the ABM comprises replacing the FR1 of a parent molecule with the FR1 encoded by a germline variable gene: sequence.

In another embodiment of the disclosure, the ABM comprises at least a CH1 region and modification of the ABM comprises at least one amino acid residue substitution as compared to a parent polypeptide. In a particular embodiment, the modification to the ABM comprises substitution of one or more of the amino acid residues at positions 148, 149 and/or 150 in the heavy chain constant region.

In another aspect, the disclosure is directed to modified antigen binding molecules comprising one or more truncated CDRs of a parent antigen binding molecule. Such truncated CDRs will contain, at a minimum, the specificity-determining amino acid residues for the given CDR. By "specificity-determining residue" is meant those residues that are directly involved in the interaction with the antigen. In general, only about one-fifth to one-third of the residues in a given CDR participate in binding to antigen. The specificity-determining residues in a particular CDR can be identified by, for example, computation of interatomic contacts from three-dimensional modeling and determination of the sequence variability at a given residue position in accordance with the methods described in Padlan et al., FASEB J. 9(1):133-139 (1995).

Accordingly, the disclosure is also directed to an isolated polynucleotide comprising at least one complementarity determining region of a parent molecule, or a variant or truncated form thereof containing at least the specificity-determining residues for said complementarity determining region, wherein said isolated polynucleotide encodes a fusion polypeptide. Preferably, such isolated polynucleotides encode a fusion polypeptide that is a modified antigen binding molecule. In one embodiment, the polynucleotide comprises three complementarity determining regions of parent molecule, or variants or truncated forms thereof containing at least the specificity-determining residues for each of said three complementarity determining regions. In another embodiment, the polynucleotide encodes the entire variable region of the light or heavy chain of a chimeric. (e.g., humanized) antibody. The disclosure is further directed to the polypeptides encoded by such polynucleotides.

In another embodiment, the disclosure is directed to a modified antigen binding molecule comprising at least one complementarity determining region of a parent molecule, or a variant or truncated form thereof containing at lest the specificity-determining residues for said complementarity determining region, and comprising a sequence derived from a heterologous polypeptide. In one embodiment, the modified antigen binding molecule comprises three complementarity determining regions of the parent moleclue, or variants or truncated forms thereof containing at least the specificity-determining residues for each of said three complementarity determining regions. In another aspect, the modified antigen binding molecule comprises the variable region of an antibody light or heavy chain. In one particularly useful embodiment, the antigen binding molecule is a chimeric, e.g., humanized, antibody. The disclosure is also directed to methods of making such modified antigen binding molecules, and the use of same in the treatment of disease, including cell proliferation disorders.

It is known that several mechanisms are involved in the therapeutic efficacy of antibodies, including antibody dependent cellular cytotoxicity (ADCC), complement-dependent cytotoxicity (CDC), and the induction of growth arrest, cell differentiation, or apoptosis.

The present disclosure is directed to modified ABMs that have increased ability to induce apoptosis compared to the corresponding non-modified parent ABM. For example, a parent ABM that has little or no ability to induce apoptosis may be modified according to the present disclosure to generate a modified ABM that does have the ability to induce apoptosis or that has an increased ability to induce apoptosis. The present disclosure is also directed to modified ABMs that have increased ability to induce growth arrest or cell differentiation as compared to the corresponding non-modified parent ABM. For example, a parent ABM that has little or no ability to induce growth arrest or cell differentiation may be modified according to the present disclosure to generate a modified ABM that does have the ability to induce growth arrest or differentiation or that has an increased ability to induce growth arrest or differentiation.

With respect to anti-CD20 antibodies in particular, for example, the majority of experimental evidence indicates that rituximab operates through conventional effector mechanisms measured by CDC and ADCC assays. Similarly, it has been shown that the resistance of different lymphoma cells to rituximab in vivo is a function of their sensitivity to CDC in vitro. In contrast, the mode of action in vivo of another anti-CD20 antibody that has been approved for therapeutic use, B1, requires neither complement nor natural killer (NK) cell activity. Rather, the efficacy of B1 in vivo is due to its ability to induce potent apoptosis. In general, anti-CD20 monoclonal antibodies fall into two distinct categories based on their mechanism of action in eradicating lymphoma cells. Type I anti-CD20 antibodies primarily utilize complement to kill target cells, while Type II antibodies operate by different mechanisms, primarily apoptosis. Rituximab and 1F5 are examples of Type I anti-CD20 antibodies, whereas B1 is an example of a Type II antibody. *See, e.g*., Cragg, M.S. and Glennie, M.J., Blood 103(7):2738-2743 (April 2004), Teeling, J.L. et al., Blood 104(6):1793-1800 (September 2004) .

U.S. Pat. Appl. Pub. No. 2005/0123546 to Umaña et al.
discloses the first known instance in which a Type I anti-CD20 antibody was engineered to have increased effector functions such as ADCC, and to generate potent apoptosis ability, effectively changing a Type I anti-CD20 antibody into a Type II anti-CD20 antibody. In one embodiment, the present disclosure is directed to a modified anti-CD20 antibody comprising a substitution in a heavy chain or light chain variable region compared to a Type I parent anti-CD20 antibody, wherein the substitution(s) result in increased induction of apoptosis by the modified anti-CD20 antibody. In another embodiment, the present disclosure is directed to engineered Type II anti-CD20 antibodies having increased ADCC as a result of engineering for increased effector function and without loss of substantial ability to induces apoptosis. In one embodiment, the Type II anti-CD20 antibodies comprise a substitution in one or more amino acids in the heavy chain or light chain variable region compared to a parent molecule. In another embodiment, the Type I and/or Type II anti-CD20 antibodies have been engineered to have an altered pattern of glycosylation in the Fc region. In a particular embodiment, the altered glycosylation of the modified ABM comprises an increased level of bisected complex residues in the Fc region. In another particular embodiment, the altered glycosylation of the modified ABM comprises a reduced level of fucose residues in the Fc region. *See* U.S. Pat Appl. Pub. No. 2004 0093 621 to Shitara et al.

In another embodiment, the Type I or Type II anti-CD20 antibodies have undergone polypeptide engineering as taught in U.S. Pat. No. 6,737,056 to Presta or U.S. Pat. Appl. Pub. No. 2004 0185045 (Macrogenics) or U.S. Pat Appl. Pub. No. 2004 0132101 (Xencor). The disclosure is further directed to methods of making such engineered Type I or Type II antibodies and to methods of using such antibodies in the treatment of various B cell disorders, including B cell lymphomas.

### Chimeric and Humanized Modified ABMs

Chimeric mouse/human antibodies have been described. *See e.g,* Morrison, S. L. et al., PNAS I1:6851-6854 (November 1984); European Patent Publication No. 173494; Boulianna, G. L, at al., Nature 312:642 (December 1984); Neubeiger, M. S. et al., Nature 314:268 (March 1985); European Patent Publication No. 125023; Tan et al., J. Immunol. 135:8564 (November 1985); Sun, L. K. et al., Hybridoma 5(1):517 (1986); Sahagan et al., J. Immunol. 137:1066-1074 (1986). See generally, Muron, Nature 312:597 (December 1984); Dickson, Genetic Engineering News 5(3) (March 1985); Marx, Science 229:455 (August 1985); and Morrison, Science 229:1202-1207 (September 1985). Robinson et al., in PCT Publication Number WO/88104936 describe a chimeric antibody with human constant region and murine variable region, having specificity to an epitope of CD20; the murine portion of the chimeric antibody of the Robinson references is derived from the 2H7 mouse monoclonal antibody (gamma 2b, kappa). While the reference notes that the described chimeric antibody is a "prime candidate" for the treatment of B cell disorders, this statement can be viewed as no more than a suggestion to those in the art to determine whether or not this suggestion is accurate for this particular antibody, particularly because the reference lacks any data to support an assertion of therapeutic effectiveness, and importantly, data using higher order mammals such as primates or humans.

Methodologies for generating chimeric antibodies are available to those in the art For example, the light and heavy chains can be expressed separately, using, for example, immunoglobulin light chain and immunoglobulin heavy chains in separate plasmids, or on a single (e.g., polycistronic) vector. These can then be purified and assembled in vitro into complete antibodies; methodologies for accomplishing such assembly have been described, See, for example, Scharff, M., Harvey Lectures 69:125 (1974). In vitro reaction parameters for the formation of IgG antibodies from reduced isolated light and heavy chains have also been described. See, for example, Sears et al., Biochem. 16(9):2016-25 (1977).

In a particularly preferred embodiment, the chimeric ABM of the present disclosure is a humanized antibody. Methods for humanizing non-human antibodies are known in the art. For example, humanized ABMs of the present disclosure can be prepared according to the methods of U.S. Pat. No. 5,225,539 to Winter, U.S. Pat. No. 6,180,370 to Queen et al., or U.S. Pat. No. 6,632,927 to Adair et al., U.S. Pat. Appl. PubL No. 2003/0039649 to Foote; U.S. Pat. Appl. Publ. No. 2004/0044187 to Sato et al., or U.S. Pat. Appl. Publ. No., 2005/0033028 to Leung et al.*.*

Preferably, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting hypervariable region sequences for the corresponding sequences of a human antibody. Accordingly, such "humanized" antibodies are chimeric antibodies (U.S. Pat. No. 4,816,567) wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies. The subject humanized anti-CD20 antibodies will comprise constant regions of human immunoglobulin.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework region (FR) for the humanized antibody (Sims et al., J. Immunol., 151 :2296 (1993); Chothia et al., J. Mol. Biol., 196:901 (1987)). Another method of selecting the human framework sequence is to compare the sequence of each individual subregion of the full rodent framework (i.e., FR1, FR2, FR3, and FR4) or some combination of the individual subregions (e.g., FR1 and FR2) against a library of known human variable region sequences that correspond to that framework subregion (e.g., as determined by Kabat numbering), and choose the human sequence for each subregion or combination that is the closest to that of the rodent (Leung U.S. Patent Application Publication No. 2003/0040606A1, published Feb. 27, 2003). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol., 151:2623 (1993)).

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models can be generated using computer programs familiar to those skilled in the art (e.g. InsightII, accelrys inc (former MSI), or at http://swissmodel.expasy.org/ described by Schwede et al., Nucleic Acids Res. 2003 (13):3381-3385). Inspection of these models permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as maintained affinity for the target antigen(s), is achieved. In general, the hypervariable region residues are directly and most substantially involved in influencing antigen binding.

In another embodiment, the antigen binding molecules of the present disclosure are engineered to have enhanced binding affinity according to, for example, the methods disclosed in U.S. Pat. Appl. Pub. No. 2004/0132066 to Balint et al.*.*

In a preferred embodiment, the present disclosure is directed to an isolated polynucleotide comprising a sequence that encodes a polypeptide having an amino acid sequence as shown in Tables 3 and/or 5, below. The disclosure is further directed to an isolated nucleic acid comprising a sequence at least 80%, 85%, 90%, 95%, 96%, 97%, 98% or 99% identical to a nucleotide sequence shown in Tables 2 and/or 4, below. In another embodiment, the disclosure is directed to an isolated nucleic acid comprising a sequence that encodes a polypeptide having an amino acid sequence at least 80%, 85%, 90%, 95%, 96%, 97%, 98% or 99% identical to an amino acid sequence in Tables 3 and/or 5, below. The disclosure also encompasses an isolated nucleic acid comprising a sequence that encodes a polypeptide having the amino acid sequence of any of the constructs in Tables 3 and/or 5, with conservative amino acid substitutions. In certain embodiments, any of the polynucleotides or polypeptides of Tables 2- 5 maybe excluded. Therefore, for example, in certain embodiments, the modified ABM, and/or the polynucleotide encoding the modified ABM, does not comprise any or all of SBQ ID NO:3, SEQ ID NO:4, SEQ ID NO:35, SEQ ID NO:36, SEQ ID NO:37, or SBQ ID NO:38. In another example, in certain embodiments, the modified ABM of the present disclosure does not comprise any or all of SEQ ID NOs:55-62.

In another embodiment, the present disclosure is directed to an isolated polypeptide comprising an amino acid sequence as shown in Tables 3 and/or 5, below. The disclosure is further directed to an isolated polypeptide comprising a sequence encoded by a nucleotide sequence shown in Tables 2 and/or 4, below. In another embodiment, the disclosure is directed to an isolated polypeptide comprising an amino acid sequence at least 80%, 85%, 90%, 95%, 96%, 97%, 98% or 99% identical to an amino acid sequence in Tables 3 and/or 5, below. The disclosure also encompasses an isolated polypeptide comprising an amino acid sequence of any of the constructs in Tables 3 and/or 5, with conservative amino acid substitutions. In certain embodiments, any of the polynucleotides or polypeptides of Tables 2-5 may be excluded. Therefore, for example, in certain embodiments, the polypeptide does not comprise an amino acid sequence corresponding to or encoded by any or all of SEQ ID NO:3, SEQ ID NO:4, SEQ ID NO:35, SEQ ID NO:36, SEQ ID NO:37, or SBQ ID NO:38. In another example, in certain embodiments, the modified ABM of the present disclosure does not comprise any or all of SEQ ID NOs:55-62.

**TABLE 2**

| CONSTRUCT | NUCLEOTIDE SEQUENCE | SEQ ID NO |
|---|---|---|
| B-HH1 | | 1 |
| B-HH2 | | 3 |
| B-HH3 | | 5 |
| B-HH4 | | 7 |
| B-HH5 | | 9 |
| B-HH6 | | 11 |
| B-HH7 | | 13 |
| B-HH8 | | 15 |
| B-HH9 | | 17 |
| B-HL1 | | 19 |
| B-HL2 | | 21 |
| B-HL3 | | 23 |
| B-HL4 | | 25 |
| B-HL8 | | 27 |
| B-HL10 | | 29 |
| B-HL11 | | 31 |
| B-HL12 | | 33 |
| B-HL13 | | 35 |
| B-HL14 | | 37 |
| B-HL15 | | 39 |
| B-HL16 | | 41 |
| B-HL17 | | 43 |
| VH Signal Sequence | | 45 |
| B-KV1 | | 47 |
| VL Signal Sequence | | 49 |

**TABLE 3**

| CONSTRUCT | AMINO ACID SEQUENCE | SEQ ID NO |
|---|---|---|
| B-HH1 | | 2 |
| B-HH2 | | 4 |
| B-HH2A | | 124 |
| B-HH2B | | 125 |
| B-HH2C | | 126 |
| B-HH2D | | 127 |
| B-HH2E | | 128 |
| B-HH2F | | 129 |
| B-HH3 | | 6 |
| B-HB4 | | 8 |
| B-HH5 | | 10 |
| B-HH6 | | 12 |
| B-HH7 | | 14 |
| B-HH8 | | 16 |
| B-HH9 | | 18 |
| B-HL1 | | 20 |
| B-HL2 | | 22 |
| B-HL3 | | 24 |
| B-HL4 | | 26 |
| B-HL8 | | 28 |
| B-HL10 | | 30 |
| B-HL11 | | 32 |
| B-HL12 | | 34 |
| B-HL13 | | 36 |
| B-HL14 | | 38 |
| B-HL15 | | 40 |
| B-HL16 | | 42 |
| B-HL17 | | 44 |
| VH Signal Sequence | | 46 |
| B-KV1 | | 48 |
| B-KV10 | | 130 |
| B-KV11 | | 131 |
| B-KV12 | | 132 |
| B-KV13 | | 133 |
| B-KV14 | | 134 |
| VL Signal Sequence | MDMRVPAQLLGULLWFPGARC | 50 |

**TABLE 4**

| CONSTRUCT | NUCLEOTIDE SEQUENCE | SEQ ID NO. |
|---|---|---|
| I-HHD | | 51 |
| M-HHA | | 53 |

**TABLE 5**

| CONSTRUCT | AMINO ACID SEQUENCE | SEQ ID NO. |
|---|---|---|
| I-HHD | | 52 |
| M-HHA | | 54 |

In another particular embodiment, the present disclosure is directed to an isolated polynucleotide comprising a sequence that encodes a polypeptide having an amino acid sequence derived from a parent sequence shown in FIG. 1 and Table 6, and comprising at least one amino acid substitution in at least one heavy chain FR region. In another embodiment, the present disclosure is directed to an isolated polypeptide comprising an amino acid sequence derived from a parent sequence shown in FIG. 1 and Table 6, and comprising at least one amino acid substitution in at least one heavy chain FR region.

**TABLE 6**

| SEQUENCE NAME | AMINO ACID SEQUENCE | SEQ ID NO. |
|---|---|---|
| 1F5-VH | | 55 |
| B9E9-VH | | 56 |
| C2B8-VH | | 57 |
| 2H7-VH | | 58 |
| B-ly1-VH | | 59 |
| 2F2-VH | | 60 |
| 7D8-VH | | 61 |
| 11B8-VH | | 62 |

In one aspect, the modified ABMs of the present disclosure can comprise a substitution of an entire framework region compared to a parent ABM. Thus, for example, the present disclosure is further directed to an isolated polynucleotide comprising a sequence that encodes a polypeptide having at least one heavy chain FR derived from a human germline VH sequence. In a preferred embodiment, the human VH germline sequence in the FR1 region, or in the Kabat positions 8-13 is derived from any one of the sequences identified in Table 7, below. These sequences are available from the IMGT database (*http:*//*imgt.cines.fr:8104*/*textes*/ *JMGTreaertoire*), and each sequence can be identified by its accession number.

**TABLE 7**

| IMGT DATABASE NAME | ACCESSION NUMBER | SEQ ID NO (NUCLEOTIDE SEQUENCE) |
|---|---|---|
| IMGT_hVH_1_2 | X07448 | 82 |
| IMGT_hVH_2_5 | X62111 | 83 |
| IMGT_hVH_2_26 | M99648 | 84 |
| IMGT-hVH_2_70 | L21969 | 85 |
| IMGT-hVH_3_7 | M99649 | 86 |
| IMGT_hVH_3_11 | M99652 | 87 |
| IMGT_hVH_3_19 | M99656 | 88 |

| IMGT DATABASE NAME | ACCESSION NUMBER | SEQ ID NO (NUCLEOTIDE SEQUENCES) |
|---|---|---|
| IMGT_hVH_3_20 | M99657 | 89 |
| IMGT_hVH_3_33 | L06618 | 90 |
| IMGT_hVH_3_43 | M99672 | 91 |
| IMGT_hVH_3_53 | M99679 | 92 |
| IMGT_hVH_3_d | Z18898 | 93 |
| IMGT_hVH_4_4 | X05713 | 94 |
| IMGT_hVH_4_30_2 | L10089 | 95 |
| IMGT_hVH_4_34 | X92278 | 96 |
| IMGT_hVH_5_51 | M99686 | 97 |
| IMGT_hVH_6_1 | X92224 | 98 |
| IMGT_hVH_7_4_1 | L10057 | 99 |
| IMGT_hVH_7_81 | Z27509 | 100 |

In another embodiment, the present disclosure is directed to an isolated polynucleotide comprising a sequence that encodes a polypeptide comprising an amino acid sequence at Kabat positions 8 to 13 of the heavy chain variable region, or any subset thereof (e.g., positions 9 to 12, positions 10-12, etc.) according to any one of the sequences presented in Table 8 below. In another embodiment, the present disclosure is directed to an isolated polypeptide comprising an amino acid sequence at Kabat positions 8 to 13, or any subset thereof (*e.g*., positions 9 to 12, 10 to 12, etc.) according to any one of the sequences presented in Table 8 below.

**TABLE 8**

| AMINO ACID SEQUENCE | SEQ ID NO |
|---|---|
| GAEVKK | 63 |
| GPTLVK | 64 |
| GPVLVK | 65 |
| GPALVK | 66 |
| GGGLVQ | 67 |
| GGGLVK | 68 |
| GGGLVE | 69 |
| GGGVVR | 70 |
| GGGVVQ | 71 |
| GGVVVQ | 72 |
| GGGLIQ | 73 |
| RGVLVQ | 74 |
| GPGLVK | 75 |
| GSGLVK | 76 |
| GAGLLK | 77 |
| GSELKK | 78 |
| GHEVKQ | 79 |
| GAELKK | 101 |
| GAEVVK | 102 |
| GGEVKK | 103 |
| GAGVKK | 104 |
| GGGVVK | 105 |

In another embodiment, the present disclosure is directed to an isolated polynucleotide comprising a sequence that encodes a polypeptide comprising an amino acid sequence at Kabat positions 108 to 113 of the heavy chain variable region, or any subset thereof (*e.g*., positions 110 to 112, positions 110 and. 112, etc.). In a particular embodiment, the sequence at positions 108 to 113 is as shown in Table 9 below. In another embodiment, the present disclosure is directed to an isolated polypeptide comprising an amino acid sequence at Kabat positions 108 to 113, or any subset thereof (*e.g*., positions 110 to 112, positions 110 and 112, etc.) according to any one of the sequences presented in Table 9 below.

**TABLE 9**

| AMINO ACID SEQUENCE | SEQ ID NO |
|---|---|
| LVTVSS | 106 |
| LVIVSS | 107 |
| LVTVIS | 108 |
| LVIVIS | 109 |
| LVGVSS | 110 |
| LVTVGS | 111 |
| LVGVGS | 112 |
| LVAVSS | 113 |
| LVTVAS | 114 |
| LVAVAS | 115 |
| LVVVSS | 116 |
| LVTVVS | 117 |
| LVVVVS | 118 |
| LVLVSS | 119 |
| LVTVLS | 120 |
| LVLVLS | 121 |
| LVSVSS | 122 |
| LVTVTS | 123 |

In another embodiment, the present disclosure is directed to an expression vector and/or a host cell which comprise one or more isolated polynucleotides of the present disclosure.

Generally, any type of cultured cell line can be used to express the ABMs of the present invention. In a preferred embodiment, CHO cells, HEK293-EBNA cells, BHK cells, NS0 cells, SP2/0 cells, YO myeloma cells, P3X63 mouse myeloma cells, PER cells, PER.C6 cells or hybridoma cells, other mammalian cells, yeast cells, insect cells, or plant cells are used as the background cell line to generate the engineered host cells described herein.

### Modified ABMs Further Comprising Fc Regions and Fc Region Variants

In one embodiment, the ABMs of the present disclosure comprising one or more amino acid substitutions in the heavy chain V and/or CH1 regions and/or the light chain V and/or C regions may further comprise a human Fc region. In a specific embodiment, the human constant region is IgG1, as set forth in SEQ ID NOs 80 and 81, and set forth below:
IgG1 Nucleotide Sequence (SEQ ID NO:80)
IgG1 Amino Acid Sequence (SEQ ID NO:81)

However, variants and isoforms of the human Fc region are also encompassed by the present disclosure. For example, variant Fc regions suitable for use in the present disclosure can be produced according to the methods taught in U.S. Pat. No. 6,737,056 to Presta (Fc region variants with altered effector function due to one or more amino acid modifications); or in U.S. Pat. Appl. Nos. 60/439,498; 60/456,041; 60/514,549; or WO 2004/063351 (variant Fc regions with increased binding affinity due to amino acid modification); or in U.S. Pat Appl. No. 10/672,280 or WO 2004/099249 (Fc variants with altered binding to FcgammaR due to amino acid modification).

In another aspect of the disclosure, the ABMs comprising one or more amino acid substitutions in the heavy chain V and/or CH1 regions and/or the light chain V and/or C regions may further comprise an Fc region variant One can engineer an Fc region to produce a variant with altered binding affinity for one or more FcRs. One may, for example, modify one or more amino acid residues of the Fc region in order to alter (e.g. increase or decrease) binding to an FcR, as described in U.S. Provisional Pat. Appl. No. 60/678,776.

Generally, one will make an amino acid substitution at one or more of the Fc region residues identified as affecting FcR binding in order to generate such an Fc region variant. In preferred embodiments, no more than one to about ten Fc region residues will be deleted or substituted. The Fc regions herein comprising one or more amino acid modifications (e.g. substitutions) will preferably retain at least about 80%, and preferably at least about 90%, and most preferably at least about 95%, of the parent Fc region sequence or of a native sequence human Fc region.

One may also make amino acid insertion modified Fc regions, which variants have altered effector function. For example, one may introduce at least one amino acid residue (e.g. one to two amino acid residues and generally no more than ten residues) adjacent to one or more of the Fc region positions identified herein as impacting FcR binding. By adjacent is meant within one to two amino acid residues of anFc region residue identified herein. Such Fc region variants may display enhanced or diminished FcR binding and/or effector function. In order to generate such insertion variants, one may evaluate a co-crystal structure of a polypeptide comprising a binding region of an FcR (e.g. the extracellular domain of the FcR of interest) and the Fc region into which the amino acid residue(s) are to be inserted (see, e.g., Sondermann et al. Nature 406:267 (2000); Deisenhofer, Biochemistry 20 (9): 2361-2370 (1981); and Burmeister et al., Nature 3442: 379-383, (1994))
in order to rationally design a modified Fc region that exhibits, e.g., improved FcR binding ability.

By introducing the appropriate amino acid sequence modifications in a parent Fc region, one can generate a variant Fc region which (a) mediates one or more effector functions in the presence of human effector cells more or less effectively and/or (b) binds an Fc γ receptor (FcγR) or Fc neonatal receptor (FcRn) with better affinity than the parent polypeptide. Such modified Fc regions will generally comprise at least one amino acid modification in the Fc region.

In preferred embodiments, the parent polypeptide Fc region is a human Fc region, e.g. a native human Fc region human IgG1. (A and non-A allotypes), IgG2, IgG3, IgG4, and all allotypes known or discovered from any species. Fc region. Such regions have sequences such as those disclosed in U.S. Provisional Patent Application No. 60/678,776.

In certain embodiments, in order to generate an ABM comprising one or more amino acid substitutions in the heavy chain V and/or CH1 regions and/or the light chain V and/or C regions and further comprising a modified Fc region with improved effector function (e.g., ADCC), the parent polypeptide preferably has pre-existing ADCC activity (e.g., the parent polypeptide comprises a human IgG1 or human IgG3 Fc region). In some embodiments, a modified Fc region with improved ADCC mediates ADCC substantially more effectively than an antibody with a native sequence IgG1 or IgG3 Fc region.

In particular embodiments, amino acid modification(s) are introduced into the CH2 domain of the parent Fc region.

The polypeptides of the disclosure having modified Fc regions may be subjected to one or more further modifications, depending on the desired or intended use of the polypeptide. Such modifications may involve, for example, further alteration of the amino acid sequence (substitution, insertion and/or deletion of amino acid residues), fusion to heterologous polypeptide(s) and/or covalent modifications. Such farther modifications may be made prior to, simultaneously with, or following, the amino acid modification(s) disclosed above which result in an alteration of Fc receptor binding and/or effector function.

Alternatively or additionally, it may be useful to combine amino acid modifications with one or more further amino acid modifications that alter Clq binding and/or complement dependent cytoxicity function of the Fc region. The starting polypeptide of particular interest in this regard herein is one that binds to Clq and displays complement dependent cytotoxicity (CDC). Amino acid substitutions described herein may serve to alter the ability of the starting polypeptide to bind to Clq and/or modify its complement dependent cytotoxicity function (e.g. to reduce and preferably abolish these effector functions). However, polypeptides comprising substitutions at one or more of the described positions with improved Clq binding and/or complement dependent cytotoxicity (CDC) function are contemplated herein. For example, the starting polypeptide may be unable to bind Clq and/or mediate CDC and may be modified according to the teachings herein such that it acquires these further effector functions. Moreover, polypeptides with pre-existing Clq binding activity, optionally further having the ability to mediate CDC may be modified such that one or both of these activities are enhanced. Amino acid modifications that alter Clq and/or modify its complement dependent cytotoxicity function are described, for example, in WO00/42072.

As disclosed above, one can design an Fc region or portion thereof with altered effector function, e.g., by modifying Clq binding and/or FcR binding and thereby changing CDC activity and/or ADCC activity. For example, one can generate a modified Fc region with improved Clq binding and improved FcγRIII binding (e.g. having both improved ADCC activity and improved CDC activity). Alternatively, where one desires that effector function be reduced or ablated, one may engineer a modified Fc region with reduced CDC activity and/or reduced ADCC activity. In other embodiments, one may increase only one of these activities, and optionally also reduce the other activity, e.g. to generate a modified Fc region with improved ADCC activity, but reduced CDC activity and vice versa.

Another type of amino acid substitution serves to alter the glycosylation pattern of the polypeptide. This may be achieved, for example, by deleting one or more carbohydrate moieties found in the polypeptide, and/or adding one or more glycosylation sites that are not present in the polypeptide. Glycosylation of polypeptides is typically either N-linked or O-linked. N-linked refers to the attachment of the carbohydrate moiety to the side chain of an asparagine residue. The peptide sequences asparagine-X-serine and asparagine-X-threonine, where X is any amino acid except proline, are the recognition sequences for enzymatic attachment of the carbohydrate moiety to the asparagine side chain. Thus, the presence of either of these peptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

In some embodiments, the present disclosure provides compositions comprising a modification of a parent polypeptide having an Fc region, wherein the modified Fc region comprises at least one surface residue amino acid modification (*See e.g*., Deisenhofer, Biochemistry, 28;20(9):2361-70, April 1981, and WO00/42072). In other embodiments, the present disclosure. provides compositions comprising, a modification of a parent polypeptide having an Fc region, wherein the modified Fc region comprises at least one non-surface residue amino acid modification. In further embodiments, the present disclosure comprises a variant of a parent polypeptide having an Fc region, wherein the variant comprises at least one surface amino acid modification and at least one non-surface amino acid modification.

The therapeutic efficacy of the modified ABMs of the present disclosure can be further enhanced by producing them in a host cell that has been glycoengineered, to have altered expression of at least one glycoprotein-modifying glcyosyltransferase. In one embodiment, the glycoengineered host cell further expresses one or more of the following: a polynucleotide encoding a polypeptide having GnTIII activity, a polynucleotide encoding a polypeptide having ManII activity, or a polynucleotide encoding a polypeptide having GalT activity. In a preferred embodiment, the host cell expresses a polynucleotide encoding a polypeptide having GnTIII activity or ManII activity. In another preferred embodiment, the host cell expresses a polynucleotide encoding a polypetide having GnTIII activity as well as a polynucleotide encoding a polypeptide having ManII activity. In yet another preferred embodiment, the polypeptide having GnTIII activity is a fusion polypeptide comprising the Golgi localization domain of a Golgi resident polypeptide. In another preferred embodiment, the expression of the modified ABMs of the present disclosure in a host cell that expresses a polynucleotide encoding a polypeptide having GnTIII activity results in modified ABMs with increased Fc receptor binding affinity and increased effector function. Accordingly, in one embodiment, the present disclosure is directed to a host cell comprising (a) an isolated nucleic acid comprising a sequence encoding a polypeptide having GnTIII activity; and (b) an isolated polynucleotide encoding an ABM of the present disclosure, such as a chimeric, primatized or humanized antibody that binds human CD20. In a preferred embodiment, the polypeptide having GnTIII activity is a fusion polypeptide comprising the catalytic domain of GnTIII and the Golgi localization domain is the localization domain of mannosidase II. Methods for generating such fusion polypeptides and using them to produce antibodies with increased effector functions are disclosed in U.S. Provisional Pat Appl. No. 60/495,142 and U.S. Pat. Appl. Publ. No. 2004/0241817.

In another preferred embodiment, the chimeric ABM is a chimeric antibody or a fragment thereof, having the binding specificity of the murine B-Lyl antibody. In a particularly preferred embodiment, the chimeric antibody comprises a human Fc. In another preferred embodiment, the antibody is primatized or humanized.

In one embodiment, one or several polynucleotides encoding an ABM of the present disclosure may be expressed under the control of a constitutive promoter or, alternately, a regulated expression system. Suitable regulated expression systems include, but are not limited to, a tetracycline-regulated expression system, an ecdysone-inducible expression system, a lac-switch expression system, a glucocorticoid-inducible expression system, a temperature-inducible promoter system, and a metallothionein metal-inducible expression system. If several different nucleic acids encoding an ABM of the present disclosure are comprised within the host cell system, some of them may be expressed under the control of a constitutive promoter, while others are expressed under the control of a regulated promoter. The maximal expression level is considered to be the highest possible level of stable polypeptide expression that does not have a significant adverse effect on cell growth rate, and will be determined using routine experimentation. Expression levels are determined by methods generally known in the art, including Western blot analysis using an antibody specific for the ABM or an antibody specific for a peptide tag fused to the ABM; and Northern blot analysis. In a further alternative, the polynucleotide maybe operatively linked to a reporter gene; the expression levels of a modified ABM having substantially the same binding specificity of a parent antibody are determined by measuring a signal correlated with the expression level of the reporter gene. The reporter gene may be transcribed together with the nucleic acid(s) encoding said fusion polypeptide as a single mRNA molecule; their respective coding sequences may be linked either by an internal ribosome entry site (IRES) or by a cap-independent translation enhancer (CITE). The reporter gene may be translated together with at least one nucleic acid encoding a modified ABM having substantially the same binding specificity of a parent antibody such that a single polypeptide chain is formed. The nucleic acids encoding the AMBs of the present disclosure may be operatively linked to the reporter gene under the control of a single promoter, such that the nucleic acid encoding the fusion polypeptide and the reporter gene are transcribed into an RNA molecule which is alternatively spliced into two separate messenger RNA (mRNA) molecules; one of the resulting mRNAs is translated into said reporter protein, and the other is translated into said fusion polypeptide,

### Expression of Modified ABMs

Methods which are well known to those skilled in the art can be used to construct expression vectors containing the coding sequence of a modified ABM having substantially the same binding specificity of a parent antibody along with appropriate transcriptional/translational control signals. These methods include *in vitro* recombinant DNA techniques, synthetic techniques and *in vivo* recombination/genetic recombination. *See*, for example, the techniques described in Maniatis et al., MOLECULAR CLONING A LABORATORY MANUAL, Cold Spring Harbor Laboratory, N.Y. (1989) and Ausubel et al, CURRENT PROTOCOLS IN MOLECULAR BIOLOGY, Greene Publishing Associates and Wiley Interscience, N.Y (1989).

A variety of host-expression vector systems may be utilized to express the coding sequence of the ABMs of the present disclosure. Preferably, mammalian cells are used as host cell systems transfected with recombinant plasmid DNA or cosmid DNA-expression vectors containing the coding sequence of the protein of interest and the coding sequence of the fusion polypeptide. Most preferably, CHO cells, HEK293-EBNA cells, BHK cells, NS0 cells, SP2/0 cells, YO myeloma cells, P3X63 mouse myeloma cells, PER cells, PER.C6 cells or hybridoma cells, other mammalian cells, yeast cells, insect cells, or plant cells are used as host cell system. Some examples of expression systems and selection methods are described in the following references, and references therein: Borth et al., Biotechnol. Bioen. 71(4):266-73 (2000-2001), in Werner et al., Arzreimittelforschung/Drug Res. 48(8):870-80 (1998), in Andersen and Krummen, Curr. Op. Biotechnol 13:117-123 (2002), in Chadd and Chamow, Curr. Op. Biotechnol. 12:188-194 (2001), and in Giddings, Curr. Op. Biotechnol. 12: 450-454 (2001). In alternate embodiments, other eukaryotic host cell systems may be contemplated, including yeast cells transformed with recombinant yeast expression vectors containing the coding sequence of an ABM of the present invention, such as the expression systems taught in U.S. Pat. Appl. No. 60/344,169 and WO 03/056914 (methods for producing human-like glycoprotein in a non-human eukaryotic host cell)
; insect cell systems infected with recombinant virus expression vectors (*e.g*., baculovirus) containing the coding sequence of a modified ABM having substantially the same binding specificity of a parent antibody; plant cell systems infected with recombinant virus expression vectors (*e.g*., cauliflower mosaic virus, CaMV; tobacco mosaic virus, TMV) or transformed with recombinant plasmid expression vectors (*e.g*., Ti plasmid) containing the coding sequence of the ABM of the disclosure including, but not limited to, the expression systems taught in U.S. Pat. No. 6,815,184 (methods for expression and secretion of biologically active polypeptides from genetically engineered duckweed); WO 2004/057002 (production of glycosylated proteins in bryophyte plant cells by introduction of a glycosyl transferase gene) and WO 2004/024927 (methods of generating extracellular heterologous, non-plant protein in moss protoplast); and U.S. Pat. AppL Nos. 60/365,769, 60/368,047, and WO 2003/078614 (glycoprotein processing in transgenic plants comprising a functional mammalian GnTIII enzyme)
; or animal cell systems infected with recombinant virus expression vectors (*e.g*., adenovirus, vaccinia virus) including cell lines engineered to contain multiple copies of the DNA encoding a modified ABM having substantially the same binding specificity of a parent antibody either stably amplified (CHO/dhfr) or unstably amplified in double-minute chromosomes (*e.g*., murine cell lines). In one embodiment, the vector comprising the polynuclootide(s) encoding the ABM of the disclosure is polycistronic. Also, in one embodiment the ABM discussed above is an antibody or a fragment thereof. In a preferred embodiment, the ABM is a humanized antibody.

For the methods of this disclosure, stable expression is generally preferred to transient expression because it typically achieves more reproducible results and also is more amenable to large-scale production. Rather than using expression vectors which contain viral origins of replication, host cells can be transformed with the respective coding nucleic acids controlled by appropriate expression control elements (*e*.*g*., promoter, enhancer, sequences, transcription terminators, polyadenylation sites, etc.), and a selectable marker. Following the introduction of foreign DNA, engineered cells may be allowed to grow for 1-2 days in an enriched media, and then are switched to a selective media. The selectable marker in the recombinant plasmid confers resistance to the selection and allows selection of cells which have stably integrated the plasmid into their chromosomes and grow to form foci which in turn can be cloned and expanded into cell lines.

A number of selection systems may be used, including, but not limited to, the herpes simplex virus thymidine kinase (Wigler et al., Cell 11:223 (1977)), hypoxanthine-guamne phosphoribosyltransferase (Szybalska & Szybalski, Proc. Natl. Acad. Sci. USA 48:2026 (1962)), and adenine phosphoribosyltransferase (Lowy et al., Cell 22:817 (1980)) genes, which can be employed in tk⁻, hgprt⁻ or aprt⁻ cells, respectively. Also, antimetabolite resistance can be used as the basis of selection for dhfr, which, confers resistance to methotrexate (Wigler et al., Natl. Acad. Sci. USA 77:3567 (1989); O'Hare et αl., Proc. Natl. Acad. Sci. USA 78:1527 (1981)); gpt, which confers resistance to mycophenolic acid (Mulligan & Berg, Proc. Natl. Acad. Sci. USA 78:2072 (1981)); neo, which confers resistance to the aminoglycoside G-418 (Colberre-Garapin et al., J. Mol. Biol. 150:1 (1981)); and hygro, which confers resistance to hygromycin (Santerre et al., Gene 30:147 (1984) genes. Recently, additional selectable genes have been described, namely trpB, which allows cells to utilize indole in place of tryptophan; hisD, which allows cells to utilize histinol in place of histidine (Hartman & Mulligan, Proc. Natl. Acad. Sci. USA 85:8047 (1988)); the glutamine synthase system; and ODC (ornithine decarboxylase) which confers resistance to the ornithine decarboxylase inhibitor, 2-(difluoromethyl)-DL-ornithine, DFMO (McConlogue, in: Current Communications in Molecular Biology, Cold Spring Harbor Laboratory ed. (1987)).

### Expression of Modified ABMs comprising Fc Regions with Altered Glycosylation

In another aspect, the present disclosure is further directed to a method for modifying the glycosylation profile of the modified ABMs comprising at least one amino acid substitution in the V or CH1 region that are produced by a host cell, comprising expressing in said host cell a nucleic acid encoding a modified ABM of the invention and a nucleic acid encoding a polypeptide with GnTIII activity, or a vector comprising such nucleic acids. Preferably, the modified polypeptide is IgG or a fragment thereof comprising the Fc region. In a particularly preferred embodiment the ABM is a humanized antibody or a fragment thereof In another embodiment, the host cell is engineered to coexpress an ABM of the disclosure, GnTIII and mannosidase II (ManII).

The modified ABMs produced by the host cells of the disclosure exhibit increased Fc receptor binding affinity and/or increased effector function as a result of the modification. In a particularly preferred embodiment the modified ABM is a humanized antibody or a fragment thereof containing the Fc region. Preferably, the increased Fc receptor binding affinity is increased binding to a Fcγ activating receptor, such as the FcγRIIIa receptor. The increased effector function is preferably an increase in one or more of the following: increased antibody-dependent cellular cytotoxicity, increased antibody-dependant cellular phagocytosis (ADCP), increased cytokine secretion, increased immune-complex-mediated antigen uptake by antigen-presenting cells, increased Fc-mediated cellular cytotoxicity, increased binding to NK cells, increased binding to macrophages, increased binding to polymorphonuclear cells (PMNs), increased binding to monocytes, increased crosslinking of target-bound antibodies, increased direct signaling inducing apoptosis, increased dendritic cell maturation, and increased T cell priming.

Effector functions can be measured and/or determined by various assays known to those of skill in the art. Various assays for measuring effector functions, including Fc receptor binding affinity and complement dependent cytotoxicity, are described in US Application Publication No. 2004/0241817A.

Cytokine secretion can be measured, for example, using a sandwich ELISA, see, e.g., McRAe et al., J. Immunol. 164: 23-28 (2000) and the cytokine sandwich ELISA protocol available at www.bdbiosciences.com/pharmingen/protocols, or by the methods described in Takahashi et al., British J. Pharmacol. 137: 315-322 (2002), each of which is herein incorporated by reference in its entirety. Dendritic cell maturation, for example, can be determined using assays as set forth by Kalergis and Ravetch, J. Exp. Med. 195: 1653-59 (2002).

Examples of phagocytosis and antigen uptake/presentation assays are provided by Gresham et al., J Exp. Med. 191: 515-28 (2000); Krauss et al., J. Immunol. 153: 1769-77 (1994); and Rafiq et al., J. Clin. Invest. 110: 71-79 (2002), and Hamano et al., J. Immunol. 164: 6113-19 (2000), each of which is herein incorporated by reference in its entirety. Down regulation of cell-surface receptors can be measured, for example, by methods set forth by Liao et al., Blood 83: 2294-2304 (1994).

General methods, protocols and assays, canbe found in CELL BIOLOGY: A LABORATORY HANDBOOK, Celis, J.E., ed., (2d ed., 1998).

It is within the skill of one in the art to adapt the above-referenced methods, protocols and assays for use with the present disclosure,

The present disclosure is also directed to a method for producing an ABM of the present disclosure, having modified oligosaccharides in a host cell comprising (a) culturing a host cell engineered to express at least one nucleic acid encoding a polypeptide having GnTIII activity under conditions which permit the production of an ABM according to the present disclosure, wherein said polypeptide having GnTIII activity is expressed in an amount sufficient to modify the oligosaccharides in the Fc region of said ABM produced by said host cell; and (b) isolating said ABM. In a preferred embodiment, the polypeptide having GnTIII activity is a fusion polypeptide comprising the catalytic domain of GnTIII. In a particularly preferred embodiment, the fusion polypeptide further comprises the Golgi localization domain of a Golgi resident polypeptide.

Preferably, the Golgi localization domain is the localization domain of mannosidase II or GnTI. Alternatively, the Golgi localization domain is selected from the group consisting of: the localization domain of mannosidase I, the localization domain of GnTII, and the localization domain of α 1-6 core fucosyltransferase. The ABMs produced by the methods of the present disclosure have increased Fc receptor binding affinity and/or increased effector function. Preferably, the increased effector function is one or more of the following: increased Fc-mediated cellular cytotoxicity (including increased antibody-dependent cellular cytotoxicity), increased antibody-dependent cellular phagocytosis (ADCP), increased cytokine secretion, increased immune-complex-mediated antigen uptake by antigen-presenting cells, increased binding to NK cells, increased binding to macrophages, increased binding to monocytes, increased binding to polymorphonuclear cells, increased direct signaling inducing apoptosis, increased crosslinking of target-bound antibodies, increased dendritic cell maturation, or increased T cell priming. The increased Fc receptor binding affinity is preferably increased binding to Fc activating receptors such as FcγRIIIa. In a particularly preferred embodiment the ABM is a humanized antibody or a fragment thereof.

In another embodiment, the present disclosure is directed to a modified ABM having substantially the same binding specificity of a parent antibody produced by the methods of the disclosure which has an increased proportion of bisected oligosaccharides in the Fc region of said polypeptide. It is contemplated that such an ABM encompasses antibodies and fragments thereof comprising the Fc region. In a preferred embodiment, the ABM is a humanized antibody. In one embodiment, the percentage of bisected oligosaccharides in the Fc region of the ABM is at least 50%, more preferably, at least 60%, at least 70%, at least 80%, or at least 90%, and most preferably at least 90-95% of the total oligosaccharides. In yet another embodiment, the ABM produced by the methods of the disclosure has an increased proportion of nonfucosylated oligosaccharides in the Fc region as a result of the modification of its oligosaccharides by the methods of the present disclosure. In one embodiment, the percentage of nonfucosylated oligosaccharides is at least 50%, preferably at least 60% to 70%, most preferably at least 75%. The nonfucosylated oligosaccharides may be of the hybrid or complex type. In a particularly preferred embodin disclosure BM produced by the host cells and methods of the invention has an increased proportion of bisected, nonfucosylated oligosaccharides in the Fc region. The bisected, nonfucosylated oligosaccharides may be either hybrid or complex. Specifically, the methods of the present disclosure maybe used to produce ABMs in which at least 15%, more preferably at least 20%, more preferably at least 25%, more preferably at least 30%, more preferably at least 35% of the oligosaccharides in the Fc region of the ABM are bisected, nonfucosylated. The methods of the present disclosure may also be used to produce polypeptides in which at least 15%, more preferably at least 20%, more preferably at least 25%, more preferably at least 30%, more preferably at least 3 5% of the oligosaccharides in the Fc region of the polypeptide are bisected hybrid nonfucosylated.

In another embodiment, the present disclosure is directed to a modified ABM having substantially the same binding specificity of a parent antibody engineered to have increased effector function and/or increased Fc receptor binding affinity, produced by the methods of the disclosure. Preferably, the increased effector function is one or more of the following: increased Fc-mediated cellular cytotoxicity (including increased antibody-dependent cellular cytotoxicity), increased antibody-dependent cellular phagocytosis (ADCP), increased cytokine secretion, increased immune-complex-mediated antigen uptake by antigen-presenting cells, increased binding to NK cells, increased binding to macrophages, increased binding to monocytes, increased binding to polymorphonuclear cells, increased direct signaling inducing apoptosis, increased crosslinking of target-bound antibodies, increased dendritic cell maturation, or increased T cell priming. In a preferred embodiment, the increased Fc receptor binding affinity is increased binding to a Fc activating receptor, most preferably FcγRIIIa. In one embodiment, the modified ABM is an antibody, an antibody fragment containing the Fc region, or a fusion protein that includes a region equivalent to the Fc region of an immunoglobulin. In a particularly preferred embodiment, the ABM is a humanized antibody.

### Pharmaceutical Compositions Comprising Modified ABMs

The present disclosure is further directed to pharmaceutical compositions comprising the modified ABMs of the present disclosure and a pharmaceutically acceptable carrier.

Any conventional carrier material can be utilized. The carrier material can be an organic or inorganic one suitable for eteral, percutaneous or parenteral administration. Suitable carriers include water, gelatin, gum arabic, lactose, starch, magnesium stearate, talc, vegetable oils, polyalkylene-glycols, petroleum jelly and the like. Furthermore, the pharmaceutical preparations may contain other pharmaceutically active agents. Additional additives such as flavoring agents, stabilizers, emulsifying agents, buffers and the like may be added in accordance with accepted practices of pharmaceutical compounding.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The present disclosure is further directed to such pharmaceutical compositions for use in the treatment or prohpylaxis of cancer. The present disclosure is further directed to a method for the treatment or prophylaxis of cancer comprising administering a therapeutically effective amount of the pharmaceutical composition of the described herein.

Preferably, the cancer is selected from the group consisting of breast cancer, bladder cancer, head and neck cancer, skin cancer, pancreatic cancer, lung cancer, ovarian cancer, colon cancer, prostate cancer, kidney cancer, and brain cancer.

The present disclosure is further directed to such pharmaceutical compositions for use in the treatment or prophylaxis of a precancerous condition or lesion. The present disclosure is further directed to a method for the treatment or prophylaxis of a precancerous condition or lesion comprising administering a therapeutically effective amount of the pharmaceutical composition of the disclosure.

Preferably, the precancerous condition or lesion is selected from the group consisting of oral leukoplakia, actinic keratosis (solar keratosis), precancerous polyps of the colon or rectum, gastric epithelial dysplasia, adenomatous dysplasia, hereditary nonpolyposis colon cancer syndrome (HNPCC), Barrett's esophagus, bladder dysplasia, and precancerous cervical conditions.

The present disclosure further provides methods for the generation and use of host cell systems for the production of glycoforms of the modified ABMs of the present disclosure, having increased Fc receptor binding affinity, preferably increased binding to Fc activating receptors, and/or having increased effector functions, including antibody-dependent cellular cytotoxicity. The glycoengineering methodology that can be used with the modified ABMs of the present disclosure has been described in greater detail in U.S. Pat. No. 6,602,684 and Provisional U.S. Patent Application No. 60/441,307 and WO 2004/065540.

The modified ABMs of the present disclosure can alternatively be glycoengineered to have reduced fucose residues in the Fc region according to the techniques disclosed in EP 1 176 195 A1.

### Generation Of Cell Lines For The Production Of Modified ABMs With Altered Glycosylation Pattern

The present disclosure describes host cell expression systems for the generation of the modified ABMs of the present disclosure having modified glycosylation patterns. In particular, the present disclosure provides host cell systems for the generation of glycoforms of the modified ABMs of the present disclosure having an improved therapeutic value. Therefore, the disclosure describes host cell expression systems selected or engineered to express a polypeptide having GnTIII activity. In one embodiment, the polypeptide having GnTIII activity is a fusion polypeptide comprising the Golgi localisation domain of a heterologous Golgi resident polypeptide. Specifically, such host cell expression systems may be engineered to comprise a recombinant nucleic acid molecule encoding a polypeptide having GnTIII, operatively linked to a constitutive or regulated promoter system.

In one specific embodiment, the present disclosure describes a host cell that has been engineered to express at least one nucleic acid encoding a fusion polypeptide having GnTIII activity and comprising the Golgi localization domain of a heterologous Golgi resident polypeptide. In one aspect, the host cell is engineered with a nucleic acid molecule comprising at least one gene encoding a fusion polypeptide having GnTIII activity and comprising the Golgi localization domain of a heterologous Golgi resident polypeptide.

Generally, any type of cultured cell line, including the cell lines discussed above, can be used as a background to engineer the host cell lines of the present disclosure. In a preferred embodiment, CHO cells, BHK cells, NS0 cells, SP2/0 cells, YO myeloma cells, P3X63 mouse myeloma cells, PER cells, PER-C6 cells or hybridoma cells, other mammalian cells, yeast cells, insect cells, or plant cells are used as the background cell line to generate the engineered host cells of the disclosure.

The disclosure is contemplated to encompass any engineered host cells expressing a polypeptide having GnTIII activity, including a fusion polypeptide that comprises the Golgi localization domain of a heterologous Golgi resident polypeptide as defined herein.

One or several nucleic acids encoding a polypeptide having GnTIII activity may be expressed under the control of a constitutive promoter or, alternately, a regulated expression system. Such systems are well known in the art, and include the systems discussed above. If several different nucleic acids encoding fusion polypeptides having GnTIII activity and comprising the Golgi localization domain of a heterologous Golgi resident polypeptide are comprised within the host cell system, some of them may be expressed under the control of a constitutive promoter, while others are expressed under the control of a regulated promoter. Expression levels of the fusion polypeptides having GnTIII activity are determined by methods generally known in the art, including Western blot analysis, Northern blot analysis, reporter gene expression analysis or measurement of GnTIII activity. Alternatively, a lectin may be employed which binds to biosynthetic products of the GnTIII, for example, E₄-PHA lectin. Alternatively, a functional assay which measures the increased Fc receptor binding or increased effector function mediated by antibodies produced by the cells engineered with the nucleic acid encoding a polypeptide with GnTIII activity may be used.

### Identification Of Transfectants Or Transformants That Express The Protein Having A Modified Glycosylation Pattern

The host cells which contain the coding sequence of a modified ABM of the present disclosure and which express the biologically active gene products may be identified by at least four general approaches; (a) DNA-DNA or DNA-RNA hybridization; (b) the presence or absence of "marker" gene functions; (c) assessing the level of transcription as measured by the expression of the respective mRNA transcripts in the host cell; and (d) detection of the gene product as measured by immunoassay or by its biological activity.

In the first approach, the presence of the coding sequence of a modified ABM of the present disclosure and the coding sequence of the polypeptide having GnTIII activity can be detected by DNA-DNA or DNA-RNA hybridization using probes comprising nucleotide sequences that are homologous to the respective coding sequences, respectively, or portions or derivatives thereof.

In the second approach, the recombinant expression vector/host system can be identified and selected based upon the presence or absence of certain "marker" gene functions (*e.g*., thymidine kinase activity, resistance to antibiotics, resistance to methotrexate, transformation phenotype, occlusion body formation in baculovirus, etc.). For example, if the coding sequence of the modified ABM of the disclosure, or a fragment thereof, and the coding sequence of the polypeptide having GnTIII activity are inserted within a marker gene sequence of the vector, recombinants containing the respective coding sequences can be identified by the absence of the marker gene function. Alternatively, a marker gene can be placed in tandem with the coding sequences under the control of the same or different promoter used to control the expression of the coding sequences. Expression of the marker in response to induction or selection indicates expression of the coding sequence of the modified ABM of the disclosure and the coding sequence of the polypeptide having GrnTIII activity.

In the third approach, transcriptional activity for the coding region of the modified ABM of the disclosure, or a fragment thereof, and the coding sequence of the polypeptide having GnTIII activity can be assessed by hybridization assays. For example, RNA can be isolated and analyzed by Northern blot using a probe homologous to the coding sequences of the modified ABM of the disclosure, or a fragment thereof and the coding sequence of the polypeptide having GnTIII activity or particular portions thereof. Alternatively, total nucleic acids of the host cell may be extracted and assayed for hybridization to such probes.

In the fourth approach, the expression of the protein products can be assessed immunologically, for example by Western blots, immunoassays such as radioimmuno-precipitaiion, enzyme-linked immuno assays and the like. The ultimate test of the success of the expression system, however, involves the detection of the biologically active gene products.

### Generation And Use Of Modified ABMs Having Increased Effector Function Including Antibody-Dependent Cellular Cytotoxicity

In preferred embodiments, the present disclosure describes glycoforms of chimeric modified ABMs having substantially the same binding specificity of the murine B-Ly1 antibody and having increased effector function including antibody-dependent cellular cytotoxicity. Glycosylation engineering of antibodies has been previously described. See, *e.g*., U.S. Patent No. 6,602,684.

Clinical trials of unconjugated monoclonal antibodies (mAbs) for the treatment of some types of cancer have recently yielded encouraging results. Dillman, Cancer Biother. & Radiopharm. 12:223-25 (1997); Deo et al., Immunology Today 18:127 (1997). A chimeric, unconjugated IgG1 has been approved for low-grade or follicular B-cell non-Hodgkin's lymphoma. Dillman, Cancer Biother. & Radiopharm. 12:223-25 (1997), while another unconjugated mAb, a humanized IgG1 targeting solid breast tumors, has also been showing promising results in phase III clinical trials. Deo et al., Immunology Today 18:121 (1997). The antigens of these two mAbs are highly expressed in their respective tumor cells and the antibodies mediate potent tumor destruction by effector cells *in vitro* and *in vivo*. In contrast, many other unconjugated mAbs with fine tumor specificities cannot trigger effector functions of sufficient potency to be clinically useful. Frost et al., Cancer 80:317-33 (1997); Surfus et al., J. Immunother. 19:184-91 (1996). For some of these weaker mAbs, adjunct cytokine therapy is currently being tested. Addition of cytokines can stimulate antibody-dependent cellular cytotoxicity (ADCC) by increasing the activity and number of circulating lymphocytes. Frost et al., Cancer 80:317-33 (1997); Surfus et al., J. Immunother. 19:184-91 (1996). ADCC, a lytic attack on antibody-targeted cells, is triggered upon binding of leukocyte receptors to the constant region (Fc) of antibodies. Deo et al., Immunology Today 18:127 (1997).

A different, but complementary, approach to increase ADCC activity of unconjugated IgG1s is to engineer the Fc region of the antibody. Protein engineering studies have shown that FcγRs interact with the lower hinge region of the IgG CH2 domain. Lund et al., J. Immunol. 157:4963-69 (1996). However, FcγR binding also requires the presence of oligosaccharides covalently attached at the conserved Asn 297 in the CH2 region. Lund et al., J. Immunol. 157:4963-69 (1996); Wright and Morrison, Trends Biotech. 15:26-31 (1997), suggesting thai either oligosaccharide and polypeptide both directly contribute to the interaction site or that the oligosaccharide is required to maintain an active CH2 polypeptide conformation. Modification of the oligosaccharide structure can therefore be explored as a means to increase the affinity of the interaction.

An IgG molecule carries two N-linked oligosaccharides in its Fc region, one on each heavy chain. As any glycoprotein, an antibody is produced as a population of glycoforms which share the same polypeptide backbone but have different oligosaccharides attached to the glycosylation sites. The oligosaccharides normally found in the Fo region of serum IgG are of complex bi-antennary type (Wormald et al., Biochemistry 36:130-38 (1997), with a low level of terminal sialic acid and bisecting N-acetylglucosamine (GlcNAc), and a variable degree of terminal galactosylation and core fucosylation. Some studies suggest that the minimal carbohydrate structure required for FcγR binding lies within the oligosaccharide core. Lund et al., J. Immunol. 157:4963-69 (1996)

The mouse- or hamster-derived cell lines used in industry and academia for production of unconjugated therapeutic mAbs normally attach the required oligosaccharide determinants to Fc sites. IgGs expressed in these cell lines lack, however, the bisecting GlcNAc found in low amounts in serum IgGs. Lifely et al., Glycobiology 318:813-22 (1995). In contrast, it was recently observed that a rat myeloma-produced, humanized IgG1 (CAMPATH-1H) carried a bisecting GlcNAc in some of its glycoforms. Lifely et al., Glycobiology 318:813-22 (1995). The rat cell-derived antibody reached a similar maximal *in vitro* ADCC activity as CAMPATH-1H antibodies produced in standard cell lines, but at significantly lower antibody concentrations.

The CAMPATH antigen is normally present at high levels on lymphoma cells, and this chimeric mAb has high ADCC activity in the absence of a bisecting GlcNac. Lifely et al., Glycobiology 318:813-22 (1995). In the N-linked glycosylation pathway, a bisecting GlcNAc is added by GnTIII Schachter, Biochem. Cell Biol 64:163-81 (1986).

Previous studies used a single antibody-producing CHO cell line, that was previously engineered to express, in an externally-regulated fashion, different levels of a cloned GnTIII gene enzyme (Umana, P., et al., Nature Biotechnol. 17:176-180 (1999)). This approach established for the first time a rigorous correlation between expression of GnTIII and the ADCC activity of the modified antibody. Thus, the disclosure contemplates a recombinant, chimeric antibody or a fragment thereof with the binding specificity of the murine B-Ly1 antibody, having altered glycosylation resulting from increased GnTIII activity. The increased GnTIII activity results in an increase in the percentage of bisected oligosaccharides, as well as a decrease in the percentage of fucose residues, in the Fc region of the modified ABM. This antibody, or fragment thereof, has increased Fc receptor binding affinity and increased effector function. In addition, the disclosure is directed to antibody fragment and fusion proteins comprising a region that is equivalent to the Fc region of immunoglobulins.

### Therapeutic Applications of Modified ABMs According to the Methods of the Disclosure.

In the broadest sense, the modified ABMs of the present disclosure can be used to target cells in vivo or in vitro that express a target antigen, in particular, where said target antigen is expressed on the cell surface. The cells expressing a target antigen can be targeted for diagnostic or therapeutic purposes. In one aspect, the modified ABMs of the present disclosure can be used to alter cell signaling activity in cells expressing a target antigen. In another aspect, the modified ABMs of the present disclosure can be used to alter the cross-linking and/or oligomerization of one or more target antigens. Target antigens for the moified ABMs of the present disclosure can be cell surface receptors including, but not limited to CD20, CD21, CD22, CD19, CD47, CD99, CD2, CD45, Her1 (EGFR), Her2/neu, Her3, Her4, TRAIL receptors (e.g., TRAILR1, TRAILR2), TNFR, FGF receptors (e.g., FGFR1), IGF receptors, PDGF receptors, VEGF receptors, and other cell-surface associated receptors. In a particular embodiment, the target antigen is CD20. The modified ABMs of the disclosure also act to arrest the cell cycle, cause apoptosis of the target cells (e.g., tumor cells), inhibit angiogenesis and/or cause differentiation of target cells.

In another aspect, the disclosure is directed to a method for treating a disease that is treatable by altered cell signaling activity of a target antigen and/or by altered ability to mediate cross-linking of one or more target antigens comprising administering a therapeutically effective amount of a modified ABM of the present disclosure to a subject in need thereof. In a specific embodiment the modified ABM is an antibody. In a more specific embodiment, the antibody is humanized. Examples of diseases for which the modified ABMs can be administered include, but are not limited to, cell proliferation diseases or disorders, autoimmune diseases or disorders, and diseases or disorders related to bacterial or viral infection.

In one embodiment, the disease or disorder is a cell proliferation disorder, Examples of cell proliferation disorders that can be treated by an ABM of the present disclosure include, but are not limited to, neoplasms, cancers, malignancies and/or tumors located in the abdomen, bone, breast, digestive system, liver, pancreas, peritoneum, endocrine glands (adrenal, parathyroid, pituitary, testicles, ovary, thymus, thyroid), eye, head and neck, nervous system (central and peripheral), lymphatic system, pelvic, skin, soft tissue, spleen, thoracic region, and urogenital system. Particular neoplasms, cancers, malignancies, and/or tumors that can be treated with the ABMs of the disclosure include, but are not limited to, epidermal and squamous cell carcinomas, gliomas, pancreatic cancer, ovarian cancer, prostate cancer, breast cancer, bladder cancer, head and neck cancer, renal cell carcinomas, colon cancer, colorectal cancer, lung cancer, brain tumor, malignant melanoma, leukemia, lymphomas, T cell lymphomas, multiple myeloma, gastric cancer, cervical cancer, endometrial carcinoma, esophageal cancer, liver cancer, cutaneous cancer, urinary tract carcinoma, choriocarcinoma, pharyngeal cancer, laiyngeal cancer, thecomatosis, androblastoma, endometrium hyperplasy, endometriosis, embryoma, fibrosarcoma, Kaposi's sarcoma, hemangioma, cavernous hemangioma, angioblastoma, retinoblastoma, astrocytoma, neurofibroma, oligodendroglioma, medulloblastoma, ganglioneuroblastoma, glioma, rhabdomyosarcoma, hamartoblastoma, osteogenic sarcoma, leiomyosarcoma, thyroid sarcoma, Ewing's sarcoma, and Wilms tumor.

Similarly, other cell proliferation disorders can also be treated with the modified ABMs of the present disclosure. Examples of such cell proliferation disorders include, but are not limited to hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other cell proliferation disease, besides neoplasia, located in an organ system listed above.

Examples of autoimmune diseases or disorders include, but are not limited to, immune-mediated thrombocytopenias, such as acute idiopathic thrombocytopenic purpurea and chronic idiopathic thrombocytopenic purpurea, dermatomyositis, Sydenham's chorea, lupus nephritis, rheumatic fever, polyglandular syndromes, Henoch-Schonlein purpura, post-streptococcal nephritis, erythema nodosum, Takayasu's arteritis, Addison's disease, erythema multiforme, polyarteritis nodosa, ankylosing spondylitis, Goodpasture's syndrome, thromboangitis ubiterans, primary biliary cirrhosis, Hashimoto's thyroiditis, thyrotoxicosis, chronic active hepatitis, polymyositis/dermatomyositis, polychondritis, pamphigus vulgaris, Wegener's granulomatosis, membranous nephropathy, amyotrophic lateral sclerosis, tabes dorsalis, polymyaglia, pernicious anemia, rapidly progressive glomerulonephritis and fibrosing alveolitis, inflammatory responses such as inflammatory skin diseases including psoriasis and dermatitis (*e*.*g*. atopic dermatitis); systemic scleroderma and sclerosis; responses associated with inflammatory bowel disease (such as Crohn's disease and uloerative colitis); respiratory distress syndrome (including adult respiratory distress syndrome; ARDS); dermatitis; meningitis; encephalitis; uveitis; colitis; glomerulonephritis; allergic conditions such as eczema and asthma and other conditions involving infiltration of T cells and chronic inflammatory responses; atherosclerosis; leukocyte adhesion deficiency; rheumatoid arthritis; systemic lupus erythematosus (SLE); diabetes mellitus (*e*.*g*. Type 1 diabetes mellitus or insulin dependent diabetes mellitus); multiple sclerosis; Reynaud's syndrome; autoimmune thyroiditis; allergic encephalomyclitis; Sjorgen's syndrome; juvenile onset diabetes; and immune responses associated with acute and delayed hypersensitivity mediated by cytokines and T-lymphocytes typically found in tuberculosis, sarcoidosis, polymyositis, granulomatosis and vasculitis; pernicious amenia (Addison's disease); diseases involving leukocyte diapedesis; central nervous system (CNS) inflammatory disorder; multiple organ injury syndrome; hemolytic anemia (including, but not limited to cryoglobinemia or Coombs positive anemia); myasthenia gravis; antigen-antibody complex mediated diseases; anti-glomerular basement membrane disease; antiphospholipid syndrome; allergic neuritis; Graves disease; Lambert-Eaton myasthenic syndrome; pemphigoid bullous; pemphigus; autoimmune polyendocrinopathies; Reiter's disease; stiff-man syndrome; Behcet disease; giant cell arteritis; immune complex nephritis; IgA nephropathy; IgM polyneuropathies; immune thrombocytopenic purpura (ITP) or autoimmune thrombocytopenia etc.

The modified ABMs of the present disclosure can be used alone or in combination with other treatments or therapeutic agents to treat disorders that are treatable by increasing or decreasing cell signaling activity and/or cross-linking of one or more target antigens. In one embodiment, modified ABMs of the present disclosure can be used alone to target and kill tumor cells in vivo. The modified ABMs can also be used in conjunction with an appropriate therapeutic agent to treat human carcinoma. For example, the modified ABMs can be used in combination with standard or conventional treatment methods such as chemotherapy, radiation therapy or can be conjugated or linked to a therapeutic drug, or toxin, as well as to a lymphokine or a tumor-inhibitory growth factor, for delivery of the therapeutic agent to the site of the carcinoma. In particular embodiments, the conjugates of the modified ABMs of this disclosure include (1) immunotoxins (conjugates of the modified ABM and a cytotoxic moiety) and (2) labeled (e.g. radiolabeled, enzyme-labeled, or fluorochrome-labeled) modified ABMs in which the label provides a means for identifying immune complexes that include the labeled ABM. The modified ABMs can also be used to induce lysis through the natural complement process, and to interact with antibody dependent cytotoxic cells normally present.

The cytotoxic moiety of the immunotoxin may be a cytotoxic drug or an enzymatically active toxin of bacterial or plant origin, or an enzymatically active fragment ("A chain") of such a toxin. Enzymatically active toxins and fragments thereof used are diphtheria A chain, nonbinding active fragments of diphtheria toxin, exotoxin A chain (from Pseudomonas aeruginosa), ricin A chain, abrin A chain, modeccin A chain, alpha-sacrin, Aleurites fordii proteins, dianthin, proteins, Phytolacca americana proteins (PAPI, PAPII, and PAP-S), momordica charantia inhibitor, curcin, crotin, sapaonaria officinalis inhibitor, gelonin, mitogellin, restrictocin, phenomycin, and enomycin. In another embodiment, the modified ABMs are conjugated to small molecule anticancer drugs. Conjugates of the modified ABM and such cytotoxic moieties are made using a variety of bifunctional protein coupling agents. Examples of such reagents are SPDP, IT, bifunctional derivatives of imidoesters such a dimethyl adipimidate HCl, active esters such as disuccinimidyl suberate, aldehydes such as glutaraldehyde, bis-azido compounds such as bis (p-azidobenzoyl) hexanediamine, bis-diazonium derivatives such as bis-(p-diazoniumbenzoyl)-ethylenediamine, diisocyanates such as tolylene 2,6-diisocyanate, and bis-active fluorine compounds such as 1,5-difluoro-2,4-dinitrobenzene, The lysing portion of a toxin may be joined to the Fab fragment of the modified ABMs. Additional appropriate toxins are knownin the art, as evidenced in e.g., published U.S. Patent Application No. 2002/0128448.

In one embodiment, the antigen binding molecule of the present disclosure is conjugated to an additional moiety, such as a radiolabel or a toxin. Such conjugated modified ABMs can be produced by numerous methods that are well known in the art.

A variety of radionuclides are applicable to the present disclosure and those skilled in the art are credited with the ability to readily determine which radionuclide is most appropriate under a variety of circumstances. For example, ¹³¹iodine is a well known radionuclide used for targeted immunotherapy. However, the clinical usefulness of ¹³¹iodine can be limited by several factors including: eight-day physical half-life; dehalogenation of iodinated antibody both in the blood and at tumor sites; and emission characteristics (eg; large gamma component) which can be suboptimal for localized dose deposition in tumor. With the advent of superior chelating agents, the opportunity for attaching metal chelating groups to proteins has increased the opportunities to utilize other radionuclides such as ¹¹¹indium and ⁹⁰yttrium. ⁹⁰Yttrium provides several benefits for utilization in radioimmunotherapeutic applications: the 64 hour half-life of ⁹⁰yttrium is long enough to allow antibody accumulation by tumor and, unlike eg, ¹³¹iodine, ⁹⁰yttrium is a pure beta emitter of high energy with no accompanying gamma irradiation in its decay, with a range in tissue of 100 to 1000 cell diameters. Furthermore, the minimal amount of penetrating radiation allows for outpatient administration of ⁹⁰yttrium-labeled antibodies. Additionally, internalization of labeled antibody is not required for cell killing, and the local emission of ionizing radiation should be lethal for adjacent tumor cells lacking the target antigen.

Effective single treatment dosages (i.e., therapeutically effective amounts) of ⁹⁰yttrium labeled modified ABMs of the present disclosure range from between about 5 and about 75 mCi, more preferably between about 10 and about 40 mCi. Effective single treatment non-marrow ablative dosages of ¹³¹iodine labeled ABMs of the present disclosure range from between about 5 and about 70 mCi, more preferably between about 5 and about 40 mCi. Effective single treatment ablative dosages (i.e., may require autologous bone marrow transplantation) of ¹³¹iodine labeled antibodies of the present invention range from between about 30 and about 600 mCi, more preferably between about 50 and less than about 500 mCi. In conjunction with a chimeric antibody according to the present disclosure, owing to the longer circulating half life vis-a-vis murine antibodies, an effective single treatment non-marrow ablative dosages of ¹³¹iodine labeled chimeric antibodies range from between about 5 and about 40 mCi, more preferably less than about 30 mCi. Imaging criteria for, e.g., the ¹¹¹indium label, are typically less than about 5 mCi.

With respect to radiolabeled antibodies of the present disclosure, therapy therewith can also occur using a single therapy treatment or using multiple treatments. Because of the radionuclide component, it is preferred that prior to treatment, peripheral stem cells ("PSC") or bone marrow ("BM") be "harvested" for patients experiencing potentially fatal bone marrow toxicity resulting from radiation. BM and/or PSC are harvested using standard techniques, and then purged and frozen for possible reinfusion. Additionally, it is most preferred that prior to treatment a diagnostic dosimetry study using a diagnostic labeled antibody (e.g., using ¹¹¹indium) be conducted on the patient, a purpose of which is to ensure that the therapeutically labeled antibody (eg, using ⁹⁰yttrium) will not become unnecessarily "concentrated" in any normal organ or tissue.

In one embodiment, a chimeric, glycoengineered modified ABM of the present disclosure, is conjugated to ricin A chain. Most advantageously, the ricin A chain is deglycosylated and produced through recombinant means. An advantageous method of making the ricin immunotoxin is described in Vitetta et al., Science 238, 1098 (1987).

When used to kill human cancer cells in vitro for diagnostic purposes, the conjugates will typically be added to the cell culture medium at a concentration of at least about 10 nM. The formulation and mode of administration for in vitro use are not critical. Aqueous formulations that are compatible with the culture or perfusion medium will normally be used. Cytotoxicity may be read by conventional techniques to determine the presence or degree of cancer.

As discussed above, a cytotoxic radiopharmaceutical for treating cancer may be made by conjugating a radioactive isotope (e.g., I, Y, Pr) to a chimeric, glycoengineered and/or modified ABM of the present disclosure. The term "cytotoxic moiety" as used herein is intended to include such isotopes.

In another embodiment, liposomes are filled with a cytotoxic drug and the liposomes are coated with the ABMs of the present disclosure. Because many of the target molecules for the modified ABMs of the present disclosure expressed on the cell surface (e.g., there are many CD20 molecules on the surface of the malignant B-cell), this method permits delivery of large amounts of drug to the correct cell type.

Techniques for conjugating such therapeutic agents to antibodies are well known (*see*, *e.g*., Amon et al., "Monoclonal Antibodies for Immunotargeting of Drugs in Cancer Therapy", in Monoclonal Antibodies and Cancer Therapy, Reisfeld et al. (eds.), pp. 243-56 (Alan R. Liss, Inc. 1985); Hellstrom et al., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications, Pinchera et al. (eds.), pp. 475-506 (1985); and Thorpe et al., "The Preparation And Cytotoxic Properties Of Antibody-Toxin Conjugates", Immunol. Rev., 62:119-58 (1982) .

Still other therapeutic applications for the ABMs of the disclosure include conjugation or linkage, e.g., by recombinent DNA techniques, to an enzyme capable of converting a prodrug into a cytotoxic drug and the use of that antibody-enzyme conjugate in combination with the prodrug to convert the prodrug to a cytotoxic agent at the tumor site (see, e.g., Sector et al., "Anti-Tumor Effects of Antibody-alkaline Phosphatase", Proc. NatL Acad Sci. USA 85:4842-46 (1988); "Enhancement of the in vitro and in vivo Antitumor Activites of Phosphorylated Mitocycin C and Etoposide Derivatives by Monoclonal Antibody-Alkaline Phosphatase Conjugates", Cancer Research 49:5789-5792 (1989); and Senter, "Activation of Prodrugs by Antibody-Enzyme Conjugates: A New Approach to Cancer Therapy," FASEB J. 4:188-193 (1990)).

Still another therapeutic use for the ABMs of the disclosure involves use, either unconjugated, in the presence of complement, or as part of an antibody-drug or antibody-toxin conjugate, to remove tumor cells from the bone marrow of cancer patients. According to this approach, autologous bone marrow may be purged ex vivo by treatment with the antibody and the marrow infused back into the patient (*see*, *e.g*., Ramsay et al., "Bone Marrow Purging Using Monoclonal Antibodies", J. Clin. Immunol., 8(2):81-88 (1988)).

Furthermore, it is contemplated that the disclosure comprises a single-chain immunotoxin comprising antigen binding domains that allow substantially the same specificity of binding as a parent antibody (e.g., polypeptides comprising the CDRs of the parent antibody) and further comprising a toxin polypeptide. The single-chain immunotoxins of the disclosure may be used to treat human carcinoma *in vivo.*

Similarly, a fusion protein comprising at least the antigen-binding region of an ABM of the invention joined to at least a functionally active portion of a second protein having anti-tumor acitivty, *e.g*., a lymphokine or oncostatin, can be used to treat human carcinoma in vivo.

The present disclosure provides a method for selectively killing tumor cells expressing cell surface receptors including, but not limited to CD20, Her1 (EGFR), Her2/neu, Her3, Her4, TRAIL receptors (e.g., TRAILR1, TRAILR2), TNFR, FGF receptors (e.g., FGFR1), IGF receptors, PDGF receptors, VEGF receptors, and other cell-surface associated receptors. This method comprises reacting the modified ABM of the disclosure (conjugated, e.g., as an immunotoxin, or unconjugated) with said tumor cells. These tumor cells may be from a human carcinoma.

Additionally, this disclosure describes a method of treating carcinomas (for example, human carcinomas) *in vivo.* This method comprises administering to a subject a pharmaceutically effective amount of a composition containing at least one of the modified ABMs of the disclosure (conjugated, e.g., as an immunotoxin, or unconjugated).

In a further aspect, the disclosure is directed to an improved method for treating B-cell proliferative disorders including B-cell lymphoma, as well as an autoimmune disease produced in whole or in part by pathogenic autoantibodies, based on B-cell depletion comprising administering a therapeutically effective amount of an ABM of the present disclosure to a human subject in need thereof. In a preferred embodiment, the ABM is a glycoengineered anti-CD20 antibody with a binding specificity substantially the same as that of the murine B-Ly1 antibody. In another preferred embodiment the antibody is humanized. In this aspect of the disclosure, the ABMs of the disclosure are used to deplete the blood of normal B-cells for an extended period.

In accordance with the practice of this disclosure, the subject may be a human, equine, porcine, bovine, murine, canine, feline, and avian subjects. Other warm blooded animals are also included in this disclosure.

The disclosure further discloses methods for inhibiting the growth of human tumor cells, treating a tumor in a subject, and treating a proliferative type disease in a subject. These methods comprise administering to the subject an effective amount of the composition of the disclosure.

In one embodiment, the disclosure relates to an ABM according to the present disclosure for use in the treatment or prophylaxis of cancer, a precancerous condition or lesion, or an autoimmune disorder. In yet another embodiment, the disclosure relates to an ABM according to the present disclosure for use as a medicament for the treatment or prophylaxis of cancer, a precancerous condition or lesion, or an autoimmune disease. The cancer may be, for example, B-cell lymphoma, lung cancer, non small cell lung (NSCL) cancer, bronchioalviolar cell lung cancer, bone cancer, pancreatic cancer, skin cancer, cancer of the head or neck, cutaneous or intraocular melanoma, uterino cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, chronic or acute leukemia, lymphocytic lymphomas, neoplasms of the central nervous system (CNS), spinal axis tumors, brain stem glioma, glioblastoma multiforme, astrocytomas, schwannomas, ependymomas, medulloblastomas, meningiomas, squamous cell carcinomas, pituitary adenomas, including refractory versions of any of the above cancers, or a combination of one or more of the above cancers. The precancerous condition or lesion includes, for example, the group consisting of oral leukoplakia, actinic keratosis (solar keratosis), precancerous polyps of the colon or rectum, gastric epithelial dysplasia, adenomatous dysplasia, hereditary nonpolyposis colon cancer syndrome (HNPCC), Barrett's esophagus, bladder dysplasia, and precancerous cervical conditions.

Preferably, the cancer is selected from the group consisting of B-cell lymphoma, breast cancer, bladder cancer, head and neck cancer, skin cancer, pancreatic cancer, lung cancer, ovarian cancer, colon cancer, prostate cancer, kidney cancer, and brain cancer. Examples of autoimmune disorders are provided, above.

Yet another embodiment is the use of the ABM according to the present disclosure for the manufacture of a medicament for the treatment or prophylaxis of cancer or for the treatment or prophylaxis of a precancerous condition or lesion. Cancer and precancerous condition or lesion are defined as above.

It is apparent, therefore, that the present disclosure encompasses pharmaceutical compositions, combinations, uses, and methods for treating human carcinomas. For example, the invention includes pharmaceutical compositions for use in the treatment of human carcinomas comprising a pharmaceutically effective amount of an antibody of the present disclosure and a pharmaceutically acceptable carrier.

The ABM compositions of the disclosure can be administered using conventional modes of administration including, but not limited to, intravenous, intraperitoneal, oral, intralymphatic or administration directly into the tumor. Intravenous administration is preferred.

In one aspect of the disclosure, therapeutic formulations containing the ABMs of the disclosure are prepared for storage by mixing an antibody having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

Exemplary anti-CD20 ABM formulations are described in WO98/56418. This publication describes a liquid multidose formulation comprising 40 mg/mL rituximab, 25 mM acetate, 150 mM trehalose, 0.9% benzyl alcohol, 0.02% polysorbate 20 at pH 5.0 that has a minimum shelf life of two years storage at 2-8°C. Another anti-CD20 formulation of interest comprises 10 mg/mL rituximab in 9.0 mg/mL sodium chloride, 7.35 mg/mL sodium citrate dihydrate, 0.7 mg/mL polysorbate 80, and Sterile Water for Injection, pH6.5. In the present disclosure, rituximab will be substituted by a modified ABM of the present disclosure.

Lyophilized formulations adapted for subcutaneous administration are described in WO97/04801. Such lyophihized formulations may be reconstituted with a suitable diluent to a high protein concentration and the reconstituted formulation may be administered subcutaneously to the mammal to be treated herein.

The formulation herein may also contain more than one active compound as necessary for the particular indication being treated, preferably those with complementary activities that do not adversely affect each other. For example, it may be desirable to further provide a cytotoxic agent, chemotherapeutic agent, cytokine or immunosuppressive agent (e.g. one which acts on T cells, such as cyclosporin or an antibody that binds T cells, e.g., one which binds LFA-1). The effective amount of such other agents depends on the amount of antagonist present in the formulation, the type of disease or disorder or treatment, and other factors discussed above. These are generally used in the same dosages and with administration routes as used hereinbefore or about from 1 to 99% of the heretofore employed dosages.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions, Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antagonist, which matrices are in the form of shaped articles, e.g., films, or microcapsules. Examples of sustained-release matrices include polyesters, hydrogels (for example, poly(2-hydroxyethylmethacrylate), or poly(vinylalcahol)), polylactides (U.S. Pat No. 3,773,919), copolymers of L-glutamic acid and γethyl-L-glutamate, non-degradable ethylenevinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes:

The compositions of the disclosure may be in a variety of dosage forms which include, but are not limited to, liquid solutions or suspension, tablets, pills, powders, suppositories, polymeric microcapsules or microvesicles, liposomes, and injectable or infusible solutions. The preferred form depends upon the mode of administration and the therapeutic application.

The compositions of the disclosure also preferably include conventional pharmaceutically acceptable carriers and adjuvants known in the art such as human serum albumin, ion exchangers, alumina, lecithin, buffer substances such as phosphates, glycine, sorbic acid, potassium sorbate, and, salts or electrolytes such as protamine sulfate.

The most effective mode of administration and dosage regimen for the pharmaceutical compositions of this disclosure depends upon the severity and course of the disease, the patient's health and response to treatment and the judgment of the treating physician. Accordingly, the dosages of the compositions should be titrated to the individual patient. Nevertheless, an effective dose of the compositions of this disclosure will generally be in the range of from about 0.01 to about 2000 mg/kg.

The molecules described herein may be in a variety of dosage forms which include, but are not limited to, liquid solutions or suspensions, tablets, pills, powders, suppositories, polymeric microcapsules or microvesicles, liposomes, and injectable or infusible solutions. The preferred form depends upon the mode of administration and the therapeutic application.

The dosages of the present disclosure may, in some cases, be determined by the use of predictive biomarkers. Predictive biomarkers are molecular markers that are used to determine (i.e., observe and/or quanitate) a pattern of expression and/or activation of tumor related genes or proteins, or cellular components of a tumor-related signaling pathway. Elucidating the biological effects of targeted therapies in tumor tissue and correlating these effects with clinical response helps identify the predominant growth and survival pathways operative in tumors, thereby establishing a profile of likely responders and conversely providing a rationale for designing strategies to overcoming resistance to therapy. For example, where the modified ABM is an antibody specific for EGFR, biomarkers for anti-EGFR therapy may comprise one or more molecules that are in the EGFR downstream signaling pathway leading to a cell proliferation disorder including, but not limited to, Akt, RAS, RAF, MAPK, ERK1, ERK2, PKC, STAT3, STAT5 (Mitchell, Nature Biotech. 22: 363-364 (2004); Becker, Nature Biotech 22: 15-18 (2004); Tsao and Herbst, Signal 4: 4-9 (2003)). Biomarkers for anti-EGFR therapy may also comprise growth factor receptors such as EGFR, ErbB-2 (HBR2/neu), and ErbB-3 (HER3), and maybe positive or negative predictors of patient response to anti-EGFR therapy. For example, the growth factor receptor ErbB-3 (HER3) was determined to be a negative predictive biomarker for the anti-BGFR antibody ABX-EGF (U.S. Pat. Appl. Pub. No. 2004/0132097 A1).

Predictive biomarkers may be measured by cellular assays that are well known in the art including, but not limited to immunohistochemistry, flow cytometry, immunofluorescence, capture-and-detection assays, and reversed phase assays, and/or assays set forth in U.S. Pat. Appl. Pub. No. 2004/0132097 A1. Predictive biomarkers of anti-EGFR therapy, themselves, can be identified according to the techniques set forth in U.S. Pat. Appl. Pub. No. 2003/0190689A1.

Thus, in one aspect, the present disclosure describes a method for treating a disorder that is related to altered or dysregulated cell signaling by a target antigen and/or altered ability to mediate cross-linking and/or oligomerization of one or more target antigens comprising predicting a response to therapy with a modified ABM in a human subject in need of treatment by assaying a sample from the human subject prior to therapy with one or a plurality of reagents that detect expression and/or activation of predictive biomarkers for disorder that is related to altered or dysregulated cell signaling by a target antigen and/or altered ability to mediate cross-linking and/or oligomerization of one or more target antigens (such as cancer); determining a pattern of expression and/or activation of one or more of the predictive biomarkers, wherein the pattern predicts the human subject's response to the modified ABM therapy; and administering to a human subject who is predicted to respond positively to modified ABM treatment a therapeutically effective amount of a composition, comprising a modified ABM of the present disclosure. As used herein, *a human subject who is predicted to respond positively to modified ABM treatment* is one for whom the modified ABM will have a measurable effect on the disease or disorder that is related to altered or dysregulated cell signaling by a target antigen and/or altered ability to mediate cross-linking and/or oligomerization of one or more target antigens (e.g., tumor regression/shrinkage) and for whom the benefits of modified ABM therapy are not outweighed by adverse effects (e.g., toxicity). As used herein, a sample means any biological sample from an organism, particularly a human, comprising one or more cells, including single cells of any origin, tissue or biopsy samples which has been removed from organs such as breast, lung, gastrointestinal tract, skin, cervix, ovary, prostate, kidney, brain, head and neck, or any other other organ or tissue of the body, and other body samples including, but not limited to, smears, sputum, secretions, cerebrospinal fluid, bile, blood, lymph fluid, urine and feces.

The composition comprising a modified ABM of the present disclosure will be formulated, dosed, and administered in a fashion consistent with good medical practice. Factors for consideration in this context include the particular disease or disorder being treated, the particular mammal being treated, the clinic condition of the individual patient, the cause of the disease or disorder, the site of delivery of the agent, the method of administration, the scheduling of administration, and other factors known to medical practitioners. The therapeutically effective amount of the antagonist to be administered will be governed by such considerations.

As a general proposition, the therapeutically effective amount of the antibody administered parenterally per dose will be in the range of about 0.1 to 20 mg/kg of patient body weight per day, with the typical initial range of antagonist used being in the range of about 2 to 10 mg/kg.

Also preferably, the modified ABM is used in a therapeutically effective amount from about 1.0 mg/kg to about 15 mg/kg.

Also more preferably, the modified ABM is used in a therapeutically effective amount from about 1.5 mg/kg to about 12 mg/kg.

Also more preferably, the modified ABM is used in a therapeutically effective amount from about 1.5 mg/kg to about 4.5 mg/kg.

Also more preferably, the modified ABM is used in a therapeutically effective amount from about 4.5 mg/kg to about 12 mg/kg.

Most preferably, the modified ABM is used in a therapeutically effective amount of about 1.5 mg/kg.

Also most preferably, the modified ABM is used in a therapeutically effective amount of about 4.5 mg/kg.

Also most preferably, the modified ABM is used in a therapeutically effective amount of about 12 mg/kg.

In a preferred embodiment, the modified ABM is an antibody, preferably a humanized antibody. Suitable dosages for such an unconjugated antibody are, for example, in the range from about 20 mg/m² to about 1000 mg/m². In one embodiment, the dosage of the antibody differs from that presently recommended for rituximab. For example, one may administer to the patient one or more doses of substantially less than 375 mg/m² of the antibody, e.g., where the dose is in the range from about 20 mg/m² to about 250 mg/m², for example from about 50 mg/m² to about 200 mg/m².

Moreover, one may administer one or more initial dose(s) of the antibody followed by one or more subsequent dose(s), wherein the mg/m² dose of the antibody in the subsequent dose(s) exceeds the mg/m² dose of the antibody in the initial dose(s). For example, the initial dose may be in the range from about 20 mg/m² to about 250 mg/m² (e.g., from about 50 mg/m² to about 200mg/m²) and the subsequent dose may be in the range from about 250 mg/m² to about 1000 mg/m².

As noted above, however, these suggested amounts of modified ABM are subject to a great deal of therapeutic discretion. The key factor in selecting an appropriate dose and scheduling is the result obtained, as indicated above. For example, relatively higher doses may be needed initially for the treatment of ongoing and acute diseases. To obtain the most efficacious results, depending on the disease or disorder, the antagonist is administered as close to the first sign, diagnosis, appearance, or occurrence of the disease or disorder as possible or during remissions of the disease or disorder,

The modified ABM of the present disclosure is administered by any suitable means, including parenteral, subcutaneous, intraperitoneal, intrapulinonary, and intranasal, and, if desired for local immunosuppressive treatment, intralesional administration. Parenteral infusions include intramuscular, intravenous, intraarterial, intraperitoneal, or subcutaneous administration. In addition, the antagonist may suitably be administered by pulse infusion, e.g., with declining doses of the antagonist Preferably the dosing is given by injections, most preferably intravenous or subcutaneous injections, depending in part on whether the administration is brief or chronic.

One may administer other compounds, such as cytotoxic agents, chernotherapeutic agents, immunosuppressive agents and/or cytokines with the antagonists herein. The combined administration includes coadministration, using separate formulations or a single pharmaceutical formulation, and consecutive administration in either order, wherein preferably there is a time period while both (or all) active agents simultaneously exert their biological activities.

It would be clear that the dose of the composition of the disclosure required to achieve cures may be further reduced with schedule optimization.

In accordance with the practice of the disclosure, the pharmaceutical carrier may be a lipid carrier. The lipid carrier may be a phospholipid. Further, the lipid carrier may be a fatty acid. Also, the lipid carrier may be a detergent. As used herein, a detergent is any substance that alters the surface tension of a liquid, generally lowering it

In one example of the disclosure, the detergent may be a nonionic detergent Examples of nonionic detergents include, but are not limited to, polysorbate 80 (also known as Twean 80 or (polyoxyethylenesorbitan monooloate), Brij, and Triton (for example Triton WR-1339 and Triton A-20).

Alternatively, the detergent maybe an ionic detergent. An example of an ionic detergent includes, but is not limited to, alkyltrimethylammonium bromide,

Additionally; in accordance with the disclosure, the lipid carrier may be a liposome. As used in this application, a "liposome" is any membrane bound vesicle which contains any molecules of the disclosure or combinations thereof

The examples below explain the invention in more detail. The following preparations and examples are given to enable those skilled in the art to more clearly understand and to practice the present invention. The present invention, however, is not limited in scope by the exemplified embodiments, which are intended as illustrations of single aspects of the invention only, and methods which are functionally equivalent are within the scope of the invention. Indeed, various modifications of the invention in addition to those described herein will become apparent to those skilled in the art from the foregoing description and accompanying drawings. Such modifications are intended to fall within the scope of the appended claims.

### EXAMPLES

Unless otherwise specified, references to the numbering of specific amino acid residue positions in the following Examples are according to the Kabat numbering system.

### EXAMPLE 1

### Materials and Methods

### Cloning and Expression of Recombinant Antibody B-Lyl

B-Lyl expressing hybridoma cells (*see*, *e.g*., Popperna, S., et al., 1987, Proceedings of the 9th Biotest Symposium, Institute of Education, London, (Sonneborn, H.H. and Tills, D, Eds.); Ling, N.R, et al., 1987, In Leucocyte Typing Conference III: White cell differentiation antigens, 302-355, Oxford University Press, Oxford. (A.J. McMichael, Ed.); Knapp, W. 1990. Leukocyte Typing Conference IV Proceedings, Oxford University Press, Oxford) were grown in RPMI containing 10% FBS and 4 mM L-glutamine. 6 x 10⁶ cells with a viability > 90% were harvested and total RNA was isolated using a Qiagen RNAeasy midi kit. cDNAs encoding the variable light and heavy chains of B-Lyl were amplified by RT-PCR. The RT-PCRreaction was performed using the following conditions: 30 min at 50 °C for the first strand cDNA synthesis; 15 min at 95 °C initial denaturation; 30 cycles of 1 min at 94 °C, 1 min at 45 °C, 1.5 min at 72 °C; and a final elongation step for 10 min at 72 °C. The expected size of the PCR products was confirmed by gel electrophoresis. The PCR products were cloned into suitable *E. coli* vectors and DNA sequencing confirmed that the variable light and heavy chain encoding genes were isolated.

For construction of chimeric B-Lyl expression vectors, synthetic signal sequences and appropriate restriction sites were fused to the variable chains by additional PCR reactions. After a final confirmation of the correct DNA sequence of the variable chains, they were combined with the corresponding human IgG1 constant regions. Once the genes were constructed, they were cloned under control of the MPSV promoter and upstream of a synthetic polyA site, using two separate vectors, one for each chain, resulting in the plasmids pETF1808 (heavy chain expression vector) and pETR1813 (light chain expression vector). Bach vector carried an EBV OriP sequence.

Chimeric B-Lyl was produced by co-transfecting HEK293-EBNA cells with vectors pETR1808 and pETR1813 using a calcium phosphate-transfection approach. Exponentially growing HEK293-EBNA cells were transfected by the calcium phosphate method. Cells were grown as adherent monolayer cultures in T flasks using DMEM culture medium supplemented with 10% FCS, and were transfected when they were between 50 and 80% confluent For the transfection of a T75 flask, 8 million cells were seeded 24 hours before transfection in 14 ml DMEM culture medium supplemented with FCS (at 10% V/V final), 250 µg/ml neomycin, and cells were placed at 37°C in an incubator with a 5% CO₂ atmosphere overnight. For each T75 flask to be transfected, a solution of DNA, CaCl₂ and water was prepared by mixing 47 µg total plasmid vector DNA divided equally between the light and heavy chain expression vectors, 235 µl of a 1M CaCl₂ solution, and adding water to a final volume of 469 µl. To this solution, 469 µl of a 50 mM HEPES, 280 mM NaCl, 1.5 mMNa₂HPO₄ solution at pH 7.05 were added, mixed immediately for 10 sec and left to stand at room temperature for 20 sec. The suspension was diluted with 12 ml of DMEM supplemented with 2% FCS, and added to the T75 in place of the existing medium. The cells were incubated at 37°C, 5% CO₂ for about 17 to 20 hours, then medium was replaced with 12 ml DMEM, 10% FCS. For the production of unmodified antibody "chB-Lyl", the cells were tansfected only with antibody expression vectors pETR1808 and pETR.1813 in a 1:1 ratio. For the production of the glycoengineered antibody "chB-Lyl-ge", the cells were co-transfected with four plasmids, two for antibody expression (pETR1808 and pBTR1813), one for a fusion GnTIII polypeptide expression (pETR1519), and one for mannosidase II expression (pCLF9) at a ratio of 4:4:1:1, respectively. At day 5 post-transfection, supernatant was harvested, centrifuged for 5 min at 1200 rpm, followed by a second centrifugation for 10 min at 4000 rpm and kept at 4°C.

chB-Lyl and chB-Lyl-ge were purified from culture supernatant using three sequential chromatographic steps, Protein A chromatography, cation, exchange chromatography, and a size exclusion chromatography step on a Superdex 200 column (Amersham Pharmacia) exchanging the buffer to phosphate buffer saline and collecting the monomeric antibody peak from this last step. Antibody concentration was estimated using a spectrophotometer from the absorbance at 280 nm.

### Oligosaccharide Analysis

Oligosaccharides were enzymatically released from the antibodies by PNGaseF digestion, with the antibodies being either immobilized on a PVDF membrane or in solution.

The resulting digest solution containing the released oligosaccharides was either prepared directly for MALDI/TOF-MS analysis or was further digested with EndoH glycosidase prior to sample preparation for MALDI/TOF-MS analysis.

### Oligosaccharide release method for PVDF membrane-immobilized antibodies

The wells of a 96-well plate made with a PVDF (Immobilon P, Millipore, Bedford, Massachusetts) membrane were wetted with 100 µl methanol and the liquid was drawn through the PVDF membrane using vacuum applied to the Multiscreen vacuum manifold (Millipore, Bedford, Massachusetts). The PVDF membranes were washed three times with 300 µl of water. The wells were then washed with 50 µl RCM buffer (8M Urea, 360 mM Tris, 3.2 mM EDTA, pH 8.6). Between 30-40 µg antibody was loaded in a well containing 10 µl RCM buffer. The liquid in the well was drawn through the membrane by applying vacuum, and the membrane was subsequently washed twice with 50 µl RCM buffer. The reduction of disulfide bridges was performed by addition of 50 µl of 0.1 M dithiothreitol in RCM and incubation at 37°C for 1 h.

Following reduction, a vacuum was applied to remove the dithiothreitol solution from the well. The wells were washed three times with 300 µl water before performing the carboxymethylation of the cysteine residues by addition of 50 µl 0.1 M iodoacetic acid in RCM buffer and incubation at room temperature in the dark for 30 min.

After carboxymethylation, the wells were drawn with vacuum and subsequently washed three times with 300 µl water. The PVDF membrane was than blocked, to prevent adsorption of the endoglycosidase, by incubating 100 µl of a 1% aqueous solution of polyvinylpyrrolidone 360 at room temperature for 1 hour. The blocking reagent was then removed by gentle vacuum followed by three washes with 300 µl water.

N-linked oligosaccharides were released by addition of 2.5 mU peptide-N-glycosydase F (recombinat N-Glycanase, GLYKO, Novato, CA) and 0.1 mU Sialidase (GLYKO, Novato, CA), to remove any potential charged monosaccharide residues, in a final volume of 25 µl in 20mM NaHCO₃, pH7.0). Digestion was performed for 3 hours at 37°C.

### Oligosaccharide release method for antibodies in solution

Between 40 and 50 µg of antibody were mixed with 2.5 mU of PNGaseF (Glyko, U.S.A.) in 2 mM Tris, pH 7.0 in a final volume of 25 µl, and the mix was incubated for 3 hours at 37°C.

### Use of Endoglycosidase H digestion of PNGaseF-released oligosaccharides for the assignment of hybrid bisected oligosaccharide structures to MALDI/TOF-MS neutral oligosaccharide peaks

The PNGaseF released oligosaccharides were subsequently digested with Endoglycosidase H (EC 3.2.1.96). For the EndoH digestion, 15 mU of EndoH (Roche, Switzerland) were added to the PNGaseF digest (antibody in solution method above) to give a final volume of 30 µl, and the mix was incubated for 3 hours at 37°C. EndoH cleaves between the N-acetylglucosamine residues of the chitobiose core of N-linked oligosaccharides. The enzyme can only digest oligomannose and most hybrid type glycans, where as complex type oligosaccharides are not hydrolyzed.

### Sample preparation for MALDI/TOF-MS

The enzymatic digests containing the released oligosaccharides were incubated for a further 3 h at room after the addition of acetic acid to a final concentration of 150 mM, and were subsequently passed through 0.6 ml of cation exchange resin (AG50W-X8 resin, hydrogen form, 100-200 mesh, BioRad, Switzerland) packed into a micro-bio-spin chromatography column (BioRad, Switzerland) to remove cations and proteins. One microliter of the resulting sample was applied to a stainless steel target plate, and mixed on the plate with 1 µl of sDHB matrix. sDHB matrix was prepared by dissolving 2 mg of 2,5-dihydroxybenzoic acid plus 0.1 mg of 5-methoxysalicylic acid in 1 ml of ethanol/10 mM aqueous sodium chloride 1:1 (v/v). The samples were air dried, 0.2 µl ethanol was applied, and the samples were finally allowed to re-crystallize under air.

### MALDI/TOF-MS

The MALDI-TOF mass spectrometer used to acquire the mass spectra was a Voyager Elite (Perspective Biosystems). The instrument was operated in the linear configuration, with an acceleration of 20 kV and 80 ns delay. External calibration using oligosaccharide standards was used for mass assignment of the ions. The spectra from 200 laser shots were summed to obtain the final spectrum.

### Whole blood B Cell Depletion

495 µl heparinized blood from a healthy donor was aliquoted into 5 ml polystyrene tubes, 5 µl 100-fold concentrated antibody samples (1-1000 ng/ml final concentration) or PBS only were added and the tubes were incubated at 37°. After 24h 50 µl blood was transferred to a fresh tube and stained with anti-CD3-FITC, anti-CD19-PE and anti-CD45-CyChrome (Becton-Dickinson) for 15 min at room temperature in the dark. Before analysis, 500 µl FACS buffer (PBS containing 2% FCS and 5mM EDTA) was added to the tubes. The CD3-FITC and CD19-PE fluorescence of the blood samples were flowcytometrically analyzed by setting a threshold on CD45-CyChrome. B cell-depletion was determined by plotting the ratio of CD19⁺ B cells to CD3⁺ T cells.

### Binding of anti-CD20 Antibodies to Raji Cells

200,000 cells in 180 µl FACS buffer (PBS containing 2% FCS and 5mM EDTA) were transferred to 5 ml polystyrene tubes. Next, 20 µl of 10-fold concentrated anti-CD20 antibody samples (1-5000 ng/ml final concentration) or PBS only were added, and the tubes were incubated at 4°C for 30min. Subsequently, samples were washed twice with FACS buffer and pelleted at 300 x g for 3min. Supernatant was aspirated off and cells were taken up in 100 µl FACS buffer. Next, 1 µl anti-Fc-specific F(ab')2-FITC fragments (Jackson Immune Research Laboratories, USA) was added and the tubes were incubated at 4°C for 30min. Samples were washed twice with FACS buffer and taken up in 500 µl of FACS buffer containing 0.5 µg/ml PI for analysis by Flow Cytometry. Binding was determined by plotting the geometric mean fluorescence against the antibody concentrations.

### EXAMPLE 2

### High Homology Acceptor Approach

The high homology antibody acceptor framework search was performed by aligning the mouse B-ly1 protein sequence to a collection of human germ-line sequences and picking that human sequence that showed the highest sequence identity. Here, the sequence VH1_10 (locus 1-e, Acc. No. DP-88) from the VBase database was chosen as the heavy chain framework acceptor sequence, and the IGKV2-40 (Acc. No X59314) sequence from the IMGT database was chosen to be the framework acceptor for the light chain. Onto these two acceptor frameworks, the three complementary determining regions (CDRs) of the mouse heavy and light variable domains were grafted. Since the framework 4 region is not part of the variable region of the germ line V gene, the alignment for that position was done individually. The JH4 region was chosen for the heavy chain, and the JK4 region was chosen for the light chain. Molecular modelling of the designed immunoglobulin domain revealed one spot potentially requiring the murine amino acid residues instead of the human ones outside of the CDR. Reintroducing murine amino acid residues into the human framework would generate the so-called back mutations. For example, the human acceptor amino acid residue at Kabat position 27 was back mutated to a tyrosine residue. Humanized antibody variants were designed feat either included or omitted the back mutations. The humanized antibody light chain did not require any back mutations. After designing the protein sequences, DNA sequences encoding these proteins were synthesized as detailed below.

### Mixed Framework Approach

In order to avoid introducing back mutations at critical amino acid residue positions (critical to retain good antigen binding affinity or antibody functions) of the human acceptor framework, it was investigated whether either the whole framework region 1 (FR1), or framework regions 1 (FR1) and 2 (FR2) together, could be replaced by human antibody sequences already having donor residues, or functionally equivalent ones, at those important positions in the natural human germline sequence. For this purpose, the VH frameworks 1 and 2 of the mouse B-ly1 sequence were aligned individually to human germ-line sequences. Here, highest sequence identity was not important, and was not used, for choosing acceptor frameworks, but instead matching of several critical residues was assumed to be more important. Those critical residues comprise residues 24, 71, and 94 (Kabat numbering), and also those residues at position 27, 28, and 30 (Kabat numbering), which lie outside of the CDR1 definition by Kabat, but often are involved in antigen binding. The IMGT sequence IGHV3-15 (Acc No X92216) was chosen as a suitable one. After having designed the protein sequences, DNA sequences encoding these proteins were synthesized as detailed below. Using this approach no back mutations were required either for the light or heavy chain, in order to retain good levels of antigen binding.

### Synthesis of the antibody genes

After having designed the amino acid sequence of the humanized antibody V region, the DNA sequence had to be generated. The DNA sequence data of the individual framework regions was found in the databases for human germ line sequences. The DNA sequence of the CDR regions was taken from the corresponding murine cDNA data. With these sequences, the whole DNA sequence was virtually assembled. Having this DNA sequence data, diagnostic restriction sites were introduced in the virtual sequence, by introducing silent mutations, creating recognition sites for restriction endonucleases. To obtain the physical DNA chain, gene synthesis was performed (e.g., Wheeler et al. 1995). In this method, oligonucleotides are designed from the genes of interest, such, that a series of oligonucleotides is derived from the coding strand, and one other series is from the non-coding strand. The 3' and 5' ends of each oligonucleotide (except the very first and last in the row) always show complementary sequences to two primers derived from the opposite strand. When putting these oligonucleotides into a reaction buffer suitable for any heat stable polymerase, and adding Mg²⁺, dNTPs and a DNA polymerase, each oligonucleotide is extended from its 3' end. The newly formed 3' end of one primer then anneals with the next primer of the opposite strand, and extending its sequence further under conditions suitable for template dependant DNA chain elongation. The final product was cloned into a conventional vector for propagation in *E*. *coli.*

### Antibody production

Human heavy and light chain leader sequences (for secretion) were added upstream of the above variable region sequences and these were then joined upstream of human IgG1 kappa constant heavy and light chain sequences, respectively, using standard molecular biology techniques. The resulting full antibody heavy and light chain DNA sequences were subcloned into mammalian expression vectors (one for the light chain and one for the heavy chain) under the control of the MPSV promoter and upstream of a synthetic polyA site, each vector carrying an EBV OriP sequence, as described in Example 1 above. Antibodies were produced as described in Example 1 above, namely by co-transfecting HEK293-EBNA with the mammalian antibody heavy and light chain expression vectors, harvesting the conditioned culture medium 5 to 7 days post-transfection, and purifying the secreted antibodies by Protein A affinity chromatography, followed by cation exchange chromatography and a final size exclusion chromatographic step to isolate pure monomeric IgG1 antibodies. The antibodies were formulated in a 25 mM potassium phosphate, 125 mM sodium chloride, 100 mM glycine solution of pH 6.7. Glycoengineered variants of the humanized antibody variants were produced by co-transfection of the antibody expression vectors together with a GnT-III glycosyltransferase expression vectors, or together with a GnT-III expression vector plus a Golgi mannosidase II expression vector, as described for the chimeric antibody in Example 1 above. Glycoengineered antibodies were purified and formulated as described above for the non-glycoengineered antibodies. The oligosaccharides attached to the Fc region of the antibodies was analysed by MALDI/TOF-MS as described below.

### Oligossacharide analysis

Oligosaccharide release method for antibodies in solution Between 40 and 50 µg of antibody were mixed with 2.5 mU of PNGaseF (Glyko, U.S.A.) in 2 mM Tris, pH7.0 in a final volume of 25 microliters, and the mix was incubated for 3 hours at 37°C.

### Sample preparation for MALDI/TOF-MS

The enzymatic digests containing the released oligosaccharides were incubated for a further 3 h at room temperature after the addition of acetic acid to a final concentration of 150 mM, and were subsequently passed through 0.6 ml of cation exchange resin (AG50W-X8 resin, hydrogen form, 100-200 mesh, BioRad, Switzerland) packed into a micro-bio-spin chromatography column (BioRad, Switzerland) to remove cations and proteins. One microliter of the resulting sample was applied to a stainless steel target plate, and mixed on the plate with 1 µl of sDHB matrix. sDHB matrix was prepared by dissolving 2 mg of 2,5-dihydroxybenzoic acid plus 0.1 mg of 5-methoxysalicylio acid in 1 ml of ethanol/10 mM aqueous sodium chloride 1:1 (vlv). The samples were air dried, 0.2 µl ethanol was applied, and the samples were finally allowed to re-crystallize under air.

### MALDI/TOF-MS

The MALDI-TOF mass spectrometer used to acquire the mass spectra was a Voyager Elite (Perspective Biosystems). The instrument was operated in the linear configuration, with an acceleration of 20kV and 80 ns delay. External calibration using oligosaccharide standards was used for mass assignment of the ions. The spectra from 200 laser shots were summed to obtain the final spectrum.

### Antigen binding assay

The purified, monomeric humanized antibody variants were tested for binding to human CD20 on Raji B-cell lymphoma target cells using a flow cytometry-based assay, as described for the chimeric B-ly1 antibody in Example 1 above.

### Binding of monomeric IgG1 glycovariants to NK cells and FcγRIIIA-expressing CHO cell line

Human NK cells were isolated from freshly isolated peripheral blood mononuclear cells (PBMC) applying a negative selection enriching for CD16- and CD56-positive cells (MACS system, Miltenyi Biotec GmbH, Bergisch Gladbach/Germany). The purity determined by CD56 expression was between 88-95 %. Freshly isolated NK cells were incubated in PBS without calcium and magnesium ions (3 x 10⁵ cells/ml) for 20 minutes at 37°C to remove NK cell-associated IgG. Cells were incubated at 10⁶ cells/ml at different concentrations of anti-CD20 antibody (0, 0.1, 0.3, 1, 3, 10 µg/ml) in PBS, 0.1% BSA. After several washes antibody binding was detected by incubating with 1:200 FITC-conjugated F(ab')₂ goat anti-human, F(ab')2 specific IgG (Jackson ImmunoReasearch, West Grove, PA/USA) and anti-human CD56-PE (BD Biosciences, Allschwil/Switzerland). The anti-FcgammaRIIIA 3G8 F(ab')2 fragments (Ancell, Bayport, MN/USA) were added at a concentration of 10 µg/ml to compete binding of antibody glycovariants (3 µg/ml). The fluorescence intensity referring to the bound antibody variants was determined for CD56-positive cells on a FACSCalibur (BD Biosciences, Allschwil /Switzerland). CHO cells were transfected by electroporation (280 V, 950 µF, 0.4 cm) with an expression vector coding for the FcgammaRIIIA-Val158 α-chain and the γ-chain. Transfectants were selected by addition of 6 µg/ml puromycin and stable clones were analyzed by FACS using 10 µl FITC-conjugated-anti-FcgammaRIII 3G8 monoclonal antibody (BD Biosciences, Allschwil/Switzerland) for 10⁶ cells. Binding of IgG1 to FcgammaRIIIA-Val158-expressing CHO cells was performed analogously to the NK cell binding described above.

### ADCC assay

Human peripheral blood mononuclear cells (PBMC) were used as effector cells and were prepared using Histopaque-1077 (Sigma Diagnostics Inc., St Louis, MO63178 USA) and following essentially the manufacturer's instructions. In brief, venous blood was taken with heparinized syringes from volunteers. The blood was diluted 1:0.75-1.3 with PBS (not containing Ca++ or Mg++) and layered on Histopaque-1077. The gradient was centrifuged at 400 x g for 30 min at room temperature (RT) without breaks. The interphase containing the PBMC was oollected and washed with PBS (50 ml per cells from two gradients) and harvested by centrifugation at 300 x g for 10 minutes at RT. After resuspension of the pellet with PBS, the PBMC were counted and washed a second time by centrifugation at 200 x g for 10 minutes at RT. The cells were then resuspended in the appropriate medium for the subsequent procedures.

The effector to target ratio used for the ADCC assays was 25:1 and 10:1 for PBMC and NK cells, respectively. The effector cells were prepared in AIM-V medium at the appropriate concentration in order to add 50 µl per well of round bottom 96 well plates. Target cells were human B lymphoma cells (e.g., Raji cells) grown in DMEM containing 10% FCS. Target cells were washed in PBS, counted and resuspended in AIM-V at 0.3 million per ml in order to add 30,000 cells in 100 µl per microwell. Antibodies were diluted in AIM-V, added in 50 µl to the pre-plated target cells and allowed to bind to the targets for 10 minutes at RT. Then the effector cells were added and the plate was incubated for 4 hours at 37°C in a humidified atmosphere containing 5% CO₂. Killing of target cells was assessed by measurement of lactate dehydrogenase (LDH) release from damaged cells using the Cytotoxicity Detection kit (Roche Diagnostics, Rotkreuz, Switzerland). After the 4-hour incubation the plates were centrifuged at 800 x g. 100 µl supernatant from each well was transferred to a new transparent flat bottom 96 well plate. 100 µl color substrate buffer from the kit were added per well. The Vmax values of the color reaction were determined in an ELISA reader at 490 mn for at least 10 min using SOFTmax PRO software (Molecular Devices, Sunnyvale, CA94089, USA). Spontaneous LDH release was measured from wells containing only target and effector cells but no antibodies. Maximal release was determined from wells containing only target cells and 1% Triton X-100. Percentage of specific antibody-mediated killing was calculated as follows: ((x-SR)/(MR - SR)*100, where x is the mean of Vmax at a specific antibody concentration, SR is the mean of Vmax of the spontaneous release and MR is the mean of Vmax of the maximal release.

### Complement dependent cytotoxicity assay

Target cells were counted, washed with PBS, resuspended in AIM-V (Invitrogen) at 1 million cells per ml. 50 µl cells were plated per well in a flat bottom 96 well plate. Antibody dilutions were prepared in AIM-V and added in 50 µl to the cells. Antibodies were allowed to bind to the cells for 10 minutes at room temperature. Human serum complement (Quidel) was freshly thawed, diluted 3-fold with AIM-V and added in 50 µl to the wells. Rabbit complement (Cedarlane Laboratories) was prepared as described by the manufacturer, diluted 3-fold with AJM-V and added in 50 µl to the wells. As a control, complement sources were heated for 30 min at 56°C before addition to the assay.
The assay plates were incubated for 2h at 37°C. Killing of cells was determined by measuring LDH release. Briefly, the plates were centrifuged at 300 x g for 3 min. 50 µl supernatant per well were transferred to a new 96 well plate and 50 µl of the assay reagent from the Cytotoxicity Kit (Roche) were added. A kinetic measurement with the ELISA reader determined the Vmax corresponding with LDH concentration in the supernatant, Maximal release was determined by incubating the cells in presence of 1% Trition X-100.

### Whole blood B-cell depletion assay

Normal B-cell depletion in whole blood by the anti-CD20 antibodies was carried out as described in Example 1 above.

### Apoptosis Assay

The apoptotic potency of the antibodies was assayed by incubating the antibody at 10 µg/ml (saturating conditions in respect to antigen binding) with the target cells (at a target cell concentration of 5 x 10⁵ cells/ml) overnight (16-24 h). Samples were stained with AnnV-FITC and analyzed by FACS. Assay was done in triplicates.

Detection is performed by flow cytometry by following the appearance of apoptotic markers like annexin V and phosphatidy serine. Negative control (no apoptosis induced) does not contain any antibody, but only phosphate buffered saline. Positive control (maximal apoptosis) contains 5 micromolar of the strong apoptosis inducer Camptothecin (CPT).

### Results and Discussion

Comparison of the binding to human CD20 antigen of antibody variants B-HH1, B-HH2, B-HH3, complexed with the humanized B-ly1 light chain (BKV1), and the parental, chimeric antibody chB-ly1 (described in Example 1 above) shows that all antibodies have a similar BC50 value, but the B-HH1 construct binds with a lower intensity/stoichiometry than the variants B-HH2 and B-HH3 (Figure 11). B-HH1 can be distinguished from B-HH2 and B-HH3 by its partially human CDR1 and CDR2 regions (Kabat definition), as well as the Ala/Thr polymorphism at position 28 (Kabat numbering). This indicates that either position 28, the complete CDR1, and/or the complete CDR2 are important for antibody/antigen interaction.

The comparison of the B-HL1, B-HH1, and the chimeric chB-ly1 parental antibody showed absence of any binding activity in the B-HL1 construct, and about half of the binding intensity/stoichiometry of the B-HH1 compared to B-ly1 (Figure 12). Both the B-HL1 as well as the B-HH1 are designed based on acceptor frameworks derived from the human VH1 class. Among other differences, position 71 (Kabat numbering) of the B-HL1 construct is a striking difference, indicating its putative importance for antigen binding; The amino acid at position 71 is one of the residues that determine the canonical loop structure of CDR2 of the heavy chain. Here, alanine or a functional equivalent thereof (*e.g*., leucine, valine, or threonine (*see*, *e*.*g*., Morea et al., Methods 20: 267-279 (2000)) seems to be important for antigen binding, whereas arginine seems to be detrimental to antigen binding.

When comparing the antigen binding data of Figures 2, and 9 to 13, the BHH2-BKV1, BHL8-BKV1, and BHL11-BKV1 variants show good binding affinity, among the different humanized antibody variants tested, to human CD20 on the surface of human cells. Despite similar EC50 values for antigen binding, these variants differ strongly in their behavior to induce apoptosis in CD20 positive target cells (see Figures 4 - 6, 14, 15). Since the original B-ly1 antibody showed low induction of apoptosis, the present inventors determined the differences between the parental B-ly1 antibody contstruct and the B-HL8 and B-HH2 constructs. Seven residues were identified in the B-HH2 heavy chain that were not present in the B-HL8 or parental B-ly1 heavy chains: Gln1, Ala9, Val11, Lys12, Ser16, Val20, and Met48. All seven of these residues are located in the framework regions of the VH domain. The likelihood of direct antigen contact was improbable, except for Gln1. To determine if a single one of these residues alone was responsible for the newly generated apoptosis-inducing property, seven variants of the B-HL8 heavy chain were generated, that potentially could restore the apoptotic behavior of the B-HH2 construct B-HL11(having the mutation E1Q), B-HL12(G9A, V48M), B-HL13(L11V, V48M), B-HL14(V12K, V48M), B-HL15(G16S, V48M), B-HL16(L20V, V48M), and B-HL17(V48M). *See* SEQ ID NOs: 32, 34, 36, 38, 40, 42, and 44 (which, as presented in the Sequence listing are not numbered according to Kabat, but whose Kabat numbering can be readily determined by one of ordinary skill). The antigen binding properties of these variants do not differ drastically with respect to EC50 values and stoichiometry (see Figure 2). However, a pronounced difference can be found in their ability to induce apotosis (Figures 4 - 6, 14, 15, and 24). The construct with the L11V, V48M modifications, B-HL13, significantly increased the ability to induce apoptosis (*see* Figure 24). The V48M modification, alone, however, did not have a visible effect (*see* Figure 5). Hence, the residues at Kabat positions 11 and 12 influenced the apoptotic behavior to the largest extent. These residues do not affect the antigen binding directly, but rather influence the interface between the VH and CH1 domains and thus act via a modification of the elbow angle.

The B-HL4 construct is derived from the B-HH2 antibody by replacing the FR1 of the B-HH2 with that of the human germ line sequence IGHV1-45 (Acc No X92209). This construct shows greatly diminished antigen binding capacity, despite having different amino acids at only four positions within FR1. These residues are located at positions 2, 14, 28 and 30 (Kabat numbering). Of these, position 28 and 30 could be an influential position, since it is part of the Chothia definition of CDR1. In variants B-HH8 and 9 (both with the BKV1 light chain), positions 28 and 30 are modified compared to the parental B-ly1 sequence. As seen in Figure 22 the binding property does not suffer significantly if threonine 28 or threonine 30 are present (Figure 22). No back mutations were introduced in the humanized light chain, which had the full Kabat CDR1, CDR2 and CDR3 grafted. In induction of apoptosis (*see* Figures 14, 15 and 21), the most potent variant was humanized B-ly1 variant BHH2-BKV1 (even more potent than the original chB-ly1 and much more potent than an antibody with a sequence identical to rituximab, C2B8). Other humanized variants that can recover the increased apoptosis are: B-HL13 and B-HL14 (derivatives of BHL8), BHH8 ("mixed framework"), BHH9 ("mixed framework" with one back mutation to examine the effect of S30T), and B-HH6 (M34I derivative of B-HH2). Variant BBH4 is another humanized B-ly1 variant that does not introduce additional non-human sequences. Variants B-HH5, B-HH6 and B-HH7 are derivatives of the B-HH2 construct with a partially humanized Kabat CDR1 region.

Important properties of the humanized B-ly1 antibody are that it is a type II anti-CD20 antibody as defined in Cragg, M.S. and Glermie, MJ., Blood 103(7):2738-2743 (April 2004). It therefore did not induce, upon binding to CD20, any significant resistance to non-ionic detergent extraction of CD20 from the surface of CD20+ human cells, using the assay described for this purpose in Polyak, MJ. and Deans, J.P., Blood 99(9):3256-3262 (2002). It induced significantly less resistance to non-ionic detergent extraction of CD20 than the C2B8 antibody (another anti-CD20 antibody with a sequence identical to rituximab (*See* U.S. Pat. Pub. No. 2003 0003097 to Reff)). As expected of a type II anti-CD20 antibody, the humanized B-ly1 did not have any significant complement mediated lysis activity and displayed much lower complement mediated lysis activity than the anti-CD20 antibody C2B8 (chimeric IgG1 with identical sequence to rituximab). Another important property of the humanized B-lyl antibody (variant B-HH2 B-KV1) was that it was very potent in the homotypic aggregation assay. In this assay CD20-positive human cells, Daudi cells, were incubated in cell culture medium for up to 24 hours at 37°C in a 5% CO₂ atmosphere in a mammalian cell incubator as described in detail in Deans *et al*., with the antibody at a concentration of 1 microgram per ml and in parallel at a concentration of 5 micrograms per ml. As a comparison, parallel control incubation of the cells was performed under identical conditions using the anti-CD20 antibody, C2B8. At different time points, including 8 hours and 24 hours of incubation, the cells were inspected visually using a microscope. It was found that the humanized B-lyl antibody led to strong homotypic aggregation, with aggregates being significantly larger that those induced by addition of the C2B8 control antibody. In addition, and consistent with the antibody being anti-CD20 type II, it induced higher levels of apoptosis when CD20-positive human cells were incubated with the humanized B-lyl antibody, relative to a control under identical conditions using the C2B8 chimeric IgG1 antibody with identical sequence to rituximab.

Glycoengineered variants of the humanized antibodies were produced by co-expression of GnTIII glycosyltransferase, together with the antibody genes, in mammalian cells. This led to an increase in the fraction of non-fucosylated oligosaccharides attached to the Fc region of the antibodies, including bisected non-fucosylated oligosaccharides, as has been described in WO 2004/065540 (Figures 17-19). The glycoengineered antibodies had significantly higher levels of binding to human FcgammaRIII receptors (Figure 20) and higher ADCC activity as well (Figure 16), relative to the non-glycoengineered antibody and relative to the C2B8 antibody. The humanized B-lyl antibody was also more potent at inducing human B-cell depletion in a whole blood assay (Figure 16) than the control C2B8 antibody. This was true both for the non-glycoengineered B-lyl antibody and for the glycoengineered version of it. The glycoengineered antibody was approximately 1000-fold more potent than the C2B8 control anti-CD20 antibody in depleting B-cells in the whole blood assay. This comparison is important both for the non-glycoengineered and for the glycoengineered humanized forms of B-lyl antibody, because it showed that in assays that combined Fc receptor-dependent activities, such as ADCC, plus complement mediated lysis, plus induction of apoptosis, that both forms of B-lyl were significantly more potent than C2B8, although both forms of B-lyl have dramatically lower complement mediated lysis activity. The ADCC, Fc receptor-dependent cell killing activities and apoptosis induction were present in this superior activity of the humanized B-lyl antibody variants. Furthermore, in the apoptosis assay, both the glycoengineered and non-glycoengineered forms of this type II anti-CD20 antibody were potent, with the Fc-engineered variants with increased binding affinity to Fcgamma receptors being even more potent in apoptosis induction than the non-Fc-engineered variant, and with all variants being significantly more potent than the control antibody C2B8. The exact mechanism for enhanced homotypic aggregation and induction of apoptopsis mediated by type II anti-CD20 antibodies is not known and concomitant binding to other molecules on the surface of CD20-positive cells, such as Fc gamma receptors, can influence this important property. It was therefore important to demonstrate that anti-CD20 antibodies of type II that have been engineered in their Fc region for increased binding affinity to Fc gamma receptors, including FcgammaRIII and with an associated increase in ADCC activity, were still able to induce strong apoptosis, even higher than the non-Fc-engineered, and homotypic aggregation. Apoptopsis induction is important since, in vivo, there are locations in the body where the target CD20-positive cells can be found, but where access to FcgammaRIII-positive cells is more difficult than in blood (for example, lymph nodes). In those locations, the induction of apoptosis by the anti-CD20 antibody, itself, can be crucial for good efficacy of the anti-CD20 antibody therapy in humans, both for the treatment of haematological malignancies such as non-Hodgkins lymphomas and B-cell chronic lymphocytic leukaemia, and for the treatment of autoimmune diseases such as rheumatoid arthritis and lupus via a B-cell depletion approach. The increased binding affinity to FcgammaRIII and higher ADCC of the humanized, Fc-engineered type II anti-CD20 antibody can also be a very important attribute for such therapies. Finally, the reduced or negligible complement mediated lysis activity of this type II anti-CD20 antibody, including humanized and Fc-engineered variants, can also be important, since higher complement activation by anti-CD20 antibodies has been correlated with increased, undesirable side-effects.

### EXAMPLE 3

To further examine residues influencing apoptotic activity, six variants of the BHH2 heavy chain were generated: BHH2-A (V11L) (SEQ ID NO: 124), BHH2-B (K12V) (SEQ ID NO: 125), BHH2-C (A9G) (SEQ ID NO: 126), BHH2-D (E10G) (SEQ ID NO: 127), BHH2-E (T110I) (SEQ ID NO: 128), and BHH2-F (S112I) (SEQ ID NO: 129). These variant constructs were tested for binding to target antigen (CD20) by methods described herein above. All six variant constructs retained binding activity.

These heavy chain variant constructs were also tested for apoptotic effect by methods described herein above. Five of the constructs, BHH2-B, BHH2-C, BHH2-D, and BHH-2F, had the same apoptic potential as the BHH2 parent construct. However, the ability of BHH2-A (V11L) to induce apoptosis was significantly decreased in comparison to BHH2 (*see* Figure 23).

The apoptotic effect of single amino acid subsitutions in the humanized B-lyl light chain (BKV1) was also examined through the generation of five variants: BKV-10 (P40A) (SEQ ID NO:130), BKV-11 (A80P) (SEQ ID NO:131), BKV-12 (V83F) (SEQ ID NO:132), BKV-13 (E105A) (SEQ ID NO: 133), and BKV-14 (I106A) (SEQ ID NO:134). Constructs BKV-11, BKV-12, BKV-13, and BKV-14 were tested for binding to target antigen (CD20) by methods described herein above, and it was determined that all four retained binding activity. These four light chain variant constructs were also tested for apoptotic effect by methods described herein above. The apoptotic potential of BKV-11, BKV-12, and BKV-13 was unchanged by their respective substiutions. However, BKV-14 demonstrated a reduced ability to induce apoptosis in comparison to BKV1 (*see, e.g*., Figure 25).

## Claims

1. A modified antigen binding molecule specifically binding to human CD20 comprising
(a) a heavy chain variable region of SEQ ID NO: 125, a heavy chain variable region comprising the replacement of the amino acid residue at Kabat position 11 with a leucine and at Kabat position 12 with lysine in SEQ ID NO: 125, a heavy chain variable region comprising the replacement of the amino acid residue at Kabat position 9 with a glycine in SEQ ID NO: 125, or a heavy chain variable region comprising the replacement of the amino acid residue at Kabat position 10 with a glycine in SEQ ID NO: 125; and
(b) a light chain variable region selected from the group consisting of SEQ ID NO: 48, 130, 131, 132, 133 and 134.

2. The modified antigen binding molecule of claim 1, wherein the antigen binding molecule comprises the heavy chain variable region selected from the group consisting of SEQ ID NO: 126, 127 and 124.

3. The modified antigen binding molecule of claim 1 or claim 2, further comprising a human Fc region.

4. The modified antigen binding molecule of claim 3, wherein said Fc region has been modified to have a decreased proportion of fucose residues compared to a non-modified Fc region, or to have an increased ratio of GlcNAc residues to fucose residues compared to a non-modified Fc region.

5. A polynucleotide encoding a heavy chain variable region of claim 1 or claim 2.

6. A polynucleotide encoding a light chain variable region of claim 1.

7. A vector comprising the polynucleotide of claim 5 or claim 6.

8. The vector of claim 7, which is polycistronic.

9. A host cell comprising the polynucleotide of claim 5 or claim 6 or the vector of claim 8.

10. The host cell of claim 9, wherein said host is engineered to express at least one nucleic acid encoding a polypeptide having P(1,4)-N-acetylglucosaminyltransferase III activity.

11. The host cell of claim 10, wherein said polypeptide having β(1,4)-N-acetylglucosaminyltransferase III activity is a fusion polypeptide further comprising the Golgi localization domain of a heterologous Golgi resident polypeptide.

12. The host cell of claim 11, wherein said Golgi localization domain is selected from the localization domain of mannosidase II, the localization domain of β(1,2)-N-acetylglucosaminyltransferase I, the localization domain of β(1,2)-N-acetylglucosaminyltransferase II, the localization domain of mannosidase I, and the localization domain of α1-6 core fucosyltransferase.

13. A method for producing a modified antigen binding molecule of any one of claims 1 to 4 said method comprising: (i) culturing the host cell of any one of claims 10 to 12 under conditions permitting the expression of said polynucleotide; and (ii) recovering said modified antigen binding molecule from the culture medium.

14. A modified antigen binding molecule having altered cell signaling activity of a target antigen and/or altered ability to mediate cross-linking of one or more target antigens, wherein said modified antigen binding molecule is obtainable by the method of claim 13.

15. A pharmaceutical composition comprising the modified antigen binding molecule of any one of claims 1 to 4 or 14, or obtainable by the method of claim 13 and a pharmaceutically acceptable carrier.

16. The antigen binding molecule of any one of claims 1 to 4 or 14, or obtainable by the method of claim 13 for use in the treatment or prophylaxis of cancer or a precancerous condition or lesion.

17. The antigen binding molecule for the use of claim 16, wherein said cancer is selected from the group consisting of B-cell lymphoma, breast cancer, bladder cancer, head and neck cancer, skin cancer, pancreatic cancer, lung cancer, ovarian cancer, colon cancer, prostate cancer, kidney cancer, and brain cancer.

18. The antigen binding "molecule for the use of claim 16, wherein said precancerous condition or lesion is selected from the group consisting of oral leukoplakia, actinic keratosis (solar keratosis), precancerous polyps of the colon or rectum, gastric epithelial dysplasia, adenomatous dysplasia, hereditary nonpolyposis colon cancer syndrome (HNPCC), Barrett's esophagus, bladder dysplasia, and precancerous cervical conditions.

## Patentansprüche

1. Modifiziertes Antigen-bindendes Molekül, das spezifisch an humanes CD20 bindet, umfassend:
(a) eine variable Region der schweren Kette der SEQ ID NO:125, eine variable Region der schweren Kette, die den Austausch des Aminosäurerests an Kabat-Position 11 mit einem Leucin und an Kabat-Position 12 mit Lysin in SEQ ID NO:125 umfasst, eine variable Region der schweren Kette, die den Austausch des Aminosäurerests an Kabat-Position 9 mit einem Glycin in SEQ ID NO:125 umfasst, oder eine variable Region der schweren Kette, die den Austausch des Aminosäurerests an Kabat-Position 10 mit einem Glycin in SEQ ID NO:125 umfasst; und
(b) eine variable Region der leichten Kette ausgewählt aus der Gruppe bestehend aus SEQ ID NO:48, 130, 131, 132, 133 und 134.

2. Modifiziertes Antigen-bindendes Molekül nach Anspruch 1, wobei das Antigen-bindende Molekül die variable Region der schweren Kette ausgewählt aus der Gruppe bestehend aus SEQ ID NO:126, 127 und 124 umfasst.

3. Modifiziertes Antigen-bindendes Molekül nach Anspruch 1 oder Anspruch 2, das des Weiteren eine humane Fc-Region umfasst.

4. Modifiziertes Antigen-bindendes Molekül nach Anspruch 3, wobei die Fc-Region modifiziert wurde, um einen verringerten Anteil an Fucoseresten im Vergleich zu einer nicht-modifizierten Fc-Region aufzuweisen, oder um ein erhöhtes Verhältnis von GlcNAc-Resten zu Fucoseresten im Vergleich zu einer nicht-modifizierten Fc-Region aufzuweisen.

5. Polynucleotid, das eine variable Region einer schweren Kette nach Anspruch 1 oder Anspruch 2 codiert.

6. Polynucleotid, das eine variable Region einer leichten Kette nach Anspruch 1 codiert.

7. Vektor, der das Polynucleotid nach Anspruch 5 oder Anspruch 6 umfasst.

8. Vektor nach Anspruch 7, der polycistronisch ist.

9. Wirtszelle, die das Polynucleotid nach Anspruch 5 oder Anspruch 6 oder den Vektor nach Anspruch 8 umfasst.

10. Wirtszelle nach Anspruch 9, wobei der Wirt verändert ist, um mindestens eine Nucleinsäure zu exprimieren, die ein Polypeptid, das β(1,4)-N-Acetylglucosaminyltransferase III-Aktivität hat, codiert.

11. Wirtszelle nach Anspruch 10, wobei das Polypeptid, das β(1,4)-N-Acetylglucosaminyltransferase III-Aktivität hat, ein Fusionspolypeptid ist, das des Weiteren die Golgi-Lokalisierungsdomäne eines heterologen Golgiresidenten Polypeptids umfasst.

12. Wirtszelle nach Anspruch 11, wobei die Golgi-Lokalisierungsdomäne ausgewählt ist aus der Lokalisierungsdomäne von Mannosidase II, der Lokalisierungsdomäne von β(1,2)-N-Acetylglucosaminyltransferase I, der Lokalisierungsdomäne von β(1,2)-N-Acetylglucosaminyltransferase II, der Lokalisierungsdomäne von Mannosidase I und der Lokalisierungsdomäne von α1-6-Kern-Fucosyltransferase.

13. Verfahren zum Herstellen eines modifizierten Antigen-bindenden Moleküls nach einem der Ansprüche 1 bis 4, wobei das Verfahren umfasst: (i) Züchten der Wirtszelle nach einem der Ansprüche 10 bis 12 unter Bedingungen, die die Expression des Polynucleotids ermöglichen; und (ii) Gewinnen des modifizierten Antigen-bindenden Moleküls aus dem Kulturmedium.

14. Modifiziertes Antigen-bindendes Molekül mit veränderter Zellsignal-Aktivität eines Zielantigens und/oder veränderter Fähigkeit, Cross-Linking eines oder mehrerer Zielantigene zu vermitteln, wobei das modifizierte Antigen-bindende Molekül mit dem Verfahren nach Anspruch 13 erhältlich ist.

15. Arzneimittel, das das modifizierte Antigen-bindende Molekül nach einem der Ansprüche 1 bis 4 oder 14 oder erhältlich mit dem Verfahren nach Anspruch 13 und einen pharmazeutisch verträglichen Träger umfasst.

16. Antigen-bindendes Molekül nach einem der Ansprüche 1 bis 4 oder 14 oder erhältlich mit dem Verfahren nach Anspruch 13 zur Verwendung bei der Behandlung oder Vorbeugung von Krebs oder einem/r präkanzerösen Zustand oder Läsion.

17. Antigen-bindendes Molekül zur Verwendung nach Anspruch 16, wobei die Krebserkrankung ausgewählt ist aus der Gruppe bestehend aus B-Zell-Lymphom, Brustkrebs, Blasenkrebs, Kopf- und Halskrebs, Hautkrebs, Bauchspeicheldrüsenkrebs, Lungenkrebs, Eierstockkrebs, Darmkrebs, Prostatakrebs, Nierenkrebs und Gehirntumor.

18. Antigen-bindendes Molekül zur Verwendung nach Anspruch 16, wobei der/die präkanzeröse Zustand oder Läsion ausgewählt ist aus der Gruppe bestehend aus oraler Leukoplakie, aktinischer Keratose (solarer Keratose), präkanzeröser Kolon- oder Rektumpolypen, Dysplasie des Magenepithels, adenomatöse Dysplasie, hereditärem nicht-Polyposis-assoziiertem kolorektalem Karzinom (hereditary nonpolyposis colon cancer syndrome; HNPCC), Barrett-Ösophagus, Blasendysplasie und präkanzerösen Zuständen des Gebärmutterhalses.

## Revendications

1. Molécule liant des antigènes modifiée qui se lie spécifiquement à CD20 humain comprenant
(a) une région variable de chaîne lourde de SEQ ID NO: 125, une région variable de chaîne lourde comprenant le remplacement du résidu d'acide aminé à la position 11 de Kabat par une leucine et à la position 12 de Kabat par la lysine dans SEQ ID NO: 125, une région variable de chaîne lourde comprenant le remplacement du résidu d'acide aminé à la position 9 de Kabat par une glycine dans SEQ ID NO: 125, ou une région variable de chaîne lourde comprenant le remplacement du résidu d'acide aminé à la position 10 de Kabat par une glycine dans SEQ ID NO: 125; et
(b) une région variable de chaîne légère choisie dans le groupe consistant en SEQ ID NO: 48, 130, 131, 132, 133 et 134.

2. Molécule liant des antigènes modifiée selon la revendication 1, où la molécule liant des antigènes comprend la région variable de chaîne lourde choisie dans le groupe consistant en SEQ ID NO: 126, 127 et 124.

3. Molécule liant des antigènes modifiée selon la revendication 1 ou la revendication 2, comprenant en outre une région Fc humaine.

4. Molécule liant des antigènes modifiée selon la revendication 3, où ladite région Fc a été modifiée pour avoir une proportion diminuée de résidus de fucose comparée à une région Fc non modifiée, ou pour avoir un rapport augmenté de résidus de GlcNAc aux résidus de fucose comparée à une région Fc non modifiée.

5. Polynucléotide codant une région variable de chaîne lourde selon la revendication 1 ou la revendication 2.

6. Polynucléotide codant une région variable de chaîne légère selon la revendication 1.

7. Vecteur comprenant le polynucléotide selon la revendication 5 ou la revendication 6.

8. Vecteur selon la revendication 7, qui est polycistronique.

9. Cellule hôte comprenant le polynucléotide selon la revendication 5 ou la revendication 6 ou le vecteur selon la revendication 8.

10. Cellule hôte selon la revendication 9, où ledit hôte est modifié pour exprimer au moins un acide nucléique codant un polypeptide ayant une activité de β(1,4)-N-acétylglucosaminyltransférase III.

11. Cellule hôte selon la revendication 10, où ledit polypeptide ayant une activité de β(1,4)-N-acétylglucosaminyltransférase III est un polypeptide de fusion comprenant en outre le domaine de localisation de Golgi d'un polypeptide résident de Golgi hétérologue.

12. Cellule hôte selon la revendication 11, où ledit domaine de localisation de Golgi est choisi parmi le domaine de localisation de la mannosidase II, le domaine de localisation de la β(1,2)-N-acétylglucosaminyltransférase I, le domaine de localisation de la β(1,2)-N-acétylglucosaminyltransférase II, le domaine de localisation de la mannosidase I et le domaine de localisation de la core α1-6 fucosyltransférase.

13. Procédé pour produire une molécule liant des antigènes modifiée selon l'une quelconque des revendications 1 à 4 ledit procédé comprenant: (i) la culture de la cellule hôte selon l'une quelconque des revendications 10 à 12 dans des conditions permettant l'expression dudit polynucléotide; et (ii) la récupération de ladite molécule liant des antigènes modifiée à partir du milieu de culture.

14. Molécule liant des antigènes modifiée ayant une activité de signalisation cellulaire altérée d'un antigène cible et/ou une aptitude altérée à assurer la médiation d'une liaison croisée d'un ou plusieurs antigènes cibles, où ladite molécule liant des antigènes modifiée peut être obtenue par le procédé selon la revendication 13.

15. Composition pharmaceutique comprenant la molécule liant des antigènes modifiée selon l'une quelconque des revendications 1 à 4 ou 14, ou qui peut être obtenue par le procédé selon la revendication 13 et un véhicule pharmaceutiquement acceptable.

16. Molécule liant des antigènes selon l'une quelconque des revendications 1 à 4 ou 14, ou qui peut être obtenue par le procédé selon la revendication 13 destinée à être utilisée dans le traitement ou la prophylaxie d'un cancer ou d'une condition ou lésion précancéreuse.

17. Molécule liant des antigènes destinée à être utilisée selon la revendication 16, où ledit cancer est choisi dans le groupe consistant en le lymphome à cellules B, le cancer du sein, le cancer de la vessie, le cancer de la tête et du cou, le cancer de la peau, le cancer du pancréas, le cancer du poumon, le cancer ovarien, le cancer du côlon, le cancer de la prostate, le cancer du rein et le cancer du cerveau.

18. Molécule liant des antigènes destinée à être utilisée selon la revendication 16, où ladite affection ou lésion précancéreuse est choisie dans le groupe consistant en la leucoplasie buccale, la kératose actinique (kératose solaire), les polypes précancéreux du côlon ou du rectum, la dysplasie épithéliale gastrique, la dysplasie adénomateuse, le syndrome de cancer colorectal héréditaire sans polypose (HNPCC), l'oesophage de Barrett, la dysplasie de la vessie et les condition cervicales précancéreuses.
